# EUROPEAN PATENT APPLICATION

(11) **EP 4 365 912 A2**
(43) Date of publication of application: **08.05.2024**
(21) Application number: 24151950.3
(22) Date of filing: 18.01.2018
(51) Int. Cl.: G16H 40/67

(54) **FLEXIBLE ANALYTE SENSORS**

(30) Priority: 19.01.2017 US 201762448295 P
(62) Divisional of application: 18741952.8
(71) Applicant: DexCom, Inc., San Diego, CA 92121 (US)
(72) Inventor: Wang, Shanger, San Diego, CA, 92121 (US); Headen, Devon, M., San Diego, CA, 92121 (US); Böhm, Sebastian, San Diego, CA, 92121 (US); Lee, Ted Tang, San Diego, CA, 92121 (US); Hughes, Jonathan, San Diego, CA, 92121 (US); Simpson, Peter C., San Diego, CA, 92121 (US); Zou, Jiong, San Diego, CA, 92121 (US)
(74) Representative: Hoffmann Eitle

(57) **Abstract**

Flexible analyte sensors are provided. Flexible analyte sensors may be flexible continuous analyte sensors that facilitate continuous monitoring of an analyte such as blood glucose. The flexible analyte sensor may have a relatively flexible conductive or non-conductive core, may be formed from a plurality of substantially planar layers, or may be configured to transform from a freestanding sensor ex vivo to a non-freestanding sensor in vivo.

## Description

### INCORPORATION BY REFERENCE TO RELATED APPLICATIONS

Any and all priority claims identified in the Application Data Sheet, or any correction thereto, are hereby incorporated by reference under 37 CFR 1.57. This application claims the benefit of U.S. Provisional Application No. 62/448,295 filed January 19, 2017. The aforementioned application is incorporated by reference herein in its entirety, and is hereby expressly made a part of this specification.

### TECHNICAL FIELD

The present disclosure generally relates to sensors and, more particularly, to continuous analyte sensors.

### BACKGROUND

Diabetes mellitus is a disorder in which the pancreas cannot create sufficient insulin (Type I or insulin dependent) and/or in which insulin is not effective (Type 2 or non-insulin dependent). In the diabetic state, the victim suffers from high blood sugar, which can cause an array of physiological derangements associated with the deterioration of small blood vessels, for example, kidney failure, skin ulcers, or bleeding into the vitreous of the eye. A hypoglycemic reaction (low blood sugar) can be induced by an inadvertent overdose of insulin, or after a normal dose of insulin or glucose-lowering agent accompanied by extraordinary exercise or insufficient food intake.

Conventionally, a person with diabetes carries a self-monitoring blood glucose (SMBG) monitor, which typically requires uncomfortable finger pricking methods. Due to the lack of comfort and convenience, a person with diabetes normally only measures his or her glucose levels two to four times per day. Unfortunately, such time intervals are spread so far apart that the person with diabetes likely finds out too late of a hyperglycemic or hypoglycemic condition, sometimes incurring dangerous side effects. Glucose levels may be alternatively monitored continuously by a sensor system including an on-skin sensor assembly. The sensor system may have a wireless transmitter which transmits measurement data to a receiver which can process and display information based on the measurements.

Implantable glucose sensors and transdermal glucose sensors have been developed for continuously measuring glucose values. However, many implantable glucose sensors suffer from complications within the body and provide only short-term and less-than-accurate sensing of blood glucose.

Skin is a complex, viscoelastic tissue. The mechanical properties of skin are dynamic and vary with age and skin region from person to person. It can therefore be challenging to provide biomechanically compatible transdermal sensors.

This Background is provided to introduce a brief context for the Summary and Detailed Description that follow. This Background is not intended to be an aid in determining the scope of the claimed subject matter nor be viewed as limiting the claimed subject matter to implementations that solve any or all of the disadvantages or problems presented above.

### SUMMARY

In accordance with various embodiments, flexible analyte sensors such as flexible continuous analyte sensors are disclosed. Flexible analyte sensors may be provided as a portion of a continuous analyte sensor system having a housing that encloses sensor electronics and from which the flexible analyte sensor extends. The flexible analyte sensor may be configured for in vivo implantation such that the flexible analyte sensor extends from the sensor electronics housing into the patient's skin for transcutaneous or subcutaneous analyte measurements.

A flexible analyte sensor may include a low modulus flexible core such as a low modulus flexible metal core, in accordance with some embodiments. In other embodiments, a flexible analyte sensor may include a low modulus flexible non-metal core or may be formed from a plurality of substantially planar flexible layers.

In comparison with conventional in vivo sensors, a flexible analyte sensor may reduce or eliminate post-insertion stress on the local tissue around the sensor and/or associated tissue reaction caused by, for example, micro motion and pressure of the sensor during in vivo use, skin deformation caused by various motions that can induce interfacial stresses, tissue abrasion by displacement of the transdermal sensor relative to local tissue, local inflammation and metabolism, cytokines and production of other cellular products, microbleeding, lymphatic disruption, interstitial fluid mixing, and development of foreign body response. By reducing the tissue reaction to the sensor (e.g., inflammation of surrounding tissue and/or encapsulation of the sensor), the signal quality and reliability can be further improved relative to conventional transdermal sensors. For example, first day effects following implantation of the sensor, in which sensor signal quality can be compromised by inflammation resulting from implantation of the sensor, may be reduced or eliminated. More particularly, flexible analyte sensors may reduce frequent micromotions that can lead to repetitive tissue damage and chronical inflammation. The reduction of these acute and chronic effects of inflammatory response can reduce the occurrence of faulty sensor data and/or negative effects on sensor accuracy. In addition, a reduction in the sliding movement of the sensor relative to local sensing area can reduce disturbances in the delicate tissue-sensor interface and help prevent reestablishment of the local interstitial fluid composition and glucose concentration gradient which can lead to sensor signal fluctuations.

Moreover, a flexible analyte sensor may facilitate tissue integration and reduce or eliminate any painful sensation induced by micro motion of the sensor relative to the local tissue and may thus improve the user experience, especially as the housing and electronic components affixed to the sensor are further miniaturized. Moreover, by reducing motion of the sensor relative to the local tissue, forces on the sensor may be reduced and the functional lifetime of the sensor itself may be increased, thereby reducing the patient cost and frequency at which a patient is pricked with a needle for insertion of a new sensor.

A flexible analyte sensor may have a Young's modulus that is less than the Young's modulus of, for example, Tantalum (e.g., approximately 186 GigaPascal (GPa)) in some embodiments. As another example, a flexible analyte sensor may have a Young's modulus that is less than the Young's modulus of Platinum (e.g., approximately 147 GPa), in some embodiments. In some embodiments, a flexible analyte sensor may have a Young's modulus that is substantially the same as the Young's modulus of the surrounding tissue. A flexible analyte sensor may have a flexural modulus that is less than 150 GPa (e.g., for metallic core flexible analyte sensors) or less than five GPA, less than 2 GPa, or less than one and a half GPa (e.g., for polymeric core or fiber reinforced core flexible analyte sensors).

In some embodiments, a flexible analyte sensor may have a first stiffness prior to implantation and a second stiffness in vivo. For example, the stiffness of the sensor may be reduced responsive to interaction between the sensor and the in vivo environment. In various embodiments, a sensor may transform from a substantially freestanding sensor to a substantially non-freestanding sensor responsive to a change in temperature, responsive to absorption of a fluid, responsive to a chemical reaction, or responsive to an applied or removed electromagnetic field.

In accordance with an embodiment, a continuous analyte sensor configured for in vivo use is provided, the continuous analyte sensor including: an elongated core; a working electrode disposed on the elongated core; and a membrane covering at least a portion of the working electrode, where the membrane includes an enzyme layer, and where a portion of the continuous analyte sensor is configured to extend from a sensor electronics housing and to have a buckling force of less than 0.25 Newtons (N).

In accordance with another embodiment, a continuous analyte sensor configured for in vivo use is provided, the continuous analyte sensor including: an elongated conductive body having a working electrode, where the elongated conductive body has a plurality of substantially planar layers, and where a portion of the plurality of substantially planar layers that is configured to extend from a housing of a continuous analyte sensor system has, in aggregate, a buckling force of less than 0.25 Newtons (N); and a membrane covering at least a portion of the working electrode, where the membrane includes an enzyme layer.

In accordance with another embodiment, a continuous analyte sensor configured for in vivo use is provided, the continuous analyte sensor including: an elongated conductive body comprising a working electrode, wherein the elongated conductive body is configured to be a freestanding elongated conductive body ex vivo and a non-freestanding elongated conductive body in vivo; and a membrane covering at least a portion of the working electrode, wherein the membrane comprises an enzyme layer.

It is understood that various configurations of the subject technology will become readily apparent to those skilled in the art from the disclosure, wherein various configurations of the subject technology are shown and described by way of illustration. As will be realized, the subject technology is capable of other and different configurations and its several details are capable of modification in various other respects, all without departing from the scope of the subject technology. Accordingly, the summary, drawings and detailed description are to be regarded as illustrative in nature and not as restrictive.

### BRIEF DESCRIPTION OF THE DRAWINGS

The present embodiments now will be discussed in detail with an emphasis on highlighting the advantageous features. These embodiments depict the novel and nonobvious sensor signal processing and calibration systems and methods shown in the accompanying drawings, which are for illustrative purposes only and are not to scale, instead emphasizing the principles of the disclosure. These drawings include the following figures, in which like numerals indicate like parts.
Fig. 1 is a schematic view of a continuous analyte sensor system attached to a host and communicating with a plurality of example devices.
Fig. 2 is a block diagram that illustrates electronics associated with the sensor system of Fig. 1.
Fig. 3 is a bottom perspective-view schematic illustrating a continuous analyte sensor system having a freestanding continuous analyte sensor extending from a housing of the continuous analyte sensor system.
Fig. 4 is a bottom perspective-view schematic illustrating a continuous analyte sensor system having a non-freestanding continuous analyte sensor extending from a housing of the continuous analyte sensor system.
Fig. 5A is a cross-sectional side-view schematic illustrating a portion of an analyte sensor.
Fig. 5B is a perspective-view schematic illustrating a portion of an analyte sensor.
Fig. 5C is a cross-sectional side-view schematic illustrating a portion of an analyte sensor.
Fig. 6A is a cross-sectional end-view schematic of the analyte sensor of Fig. 5A, taken on line 6-6.
Fig. 6B is a cross-sectional view schematic of a membrane for an analyte sensor.
Fig. 7 is a perspective-view schematic illustrating a portion of an analyte sensor.
Fig. 8 is a schematic diagram illustrating a response of a freestanding analyte sensor to an external force.
Fig. 9 is a schematic diagram illustrating a response of a non-freestanding analyte sensor to an external force.
Fig. 10 is a perspective-view schematic illustrating a portion of an elongated polymer core with conductive traces for an analyte sensor.
Fig. 11 is a perspective-view schematic of a co-extrusion manufacturing system for the elongated polymer core with conductive traces of Fig. 10.
Fig. 12 is a perspective-view schematic illustrating a portion of another elongated polymer core with conductive traces for an analyte sensor.
Fig. 13 is a perspective-view schematic illustrating a portion of an elongated polymer core for an analyte sensor.
Fig. 14 is a perspective-view schematic of a squeegee manufacturing system for the elongated polymer core of Fig. 13.
Fig. 15 is a perspective-view schematic illustrating a portion of another elongated polymer core with conductive wire traces for an analyte sensor.
Fig. 16 is a perspective-view schematic of a wire trace-lamination manufacturing system for the elongated polymer core of Fig. 12.
Fig. 17 is a perspective-view schematic of an induction heating manufacturing system for trace lamination onto the elongated polymer core of Fig. 12.
Fig. 18 is a perspective-view schematic illustrating a portion of an elongated polymer core with conductive traces and a deposited electrode pad for an analyte sensor.
Fig. 19 is a perspective-view schematic illustrating a portion of an elongated fiber reinforced core within an elongated insulating body for an analyte sensor.
Fig. 20 is a perspective-view schematic illustrating a portion of an elongated fiber reinforced core within an elongated insulating body with conductive traces for an analyte sensor.
Fig. 21 is a perspective-view schematic illustrating a portion of an elongated fiber reinforced core within an elongated insulating body, conductive traces, and an insulated sensor for an analyte sensor.
Fig. 22 is a perspective-view schematic illustrating a back end portion of an elongated fiber reinforced core within an elongated insulating body for an analyte sensor.
Fig. 23 is a perspective-view schematic illustrating a portion of an elongated fiber reinforced core within an elongated insulating body, conductive traces, an insulated sensor, and a reference electrode for an analyte sensor.
Fig. 24 is a perspective-view schematic illustrating a back end portion of the sensor of Fig. 23.
Fig. 25A is a perspective-view schematic illustrating a portion of an elongated metalized Kevlar core within an elongated insulating body for an analyte sensor.
Fig. 25B is a perspective-view schematic illustrating a portion of an elongated metalized single-fiber Kevlar core within an elongated insulating body for an analyte sensor.
Fig. 26 is a perspective-view schematic illustrating a back end portion of the sensor of Fig. 25A.
Fig. 27 is a perspective-view schematic illustrating a portion of another elongated polymer core for an analyte sensor at various stages during stenciling of a working electrode and conductive trace thereon.
Fig. 28 is a perspective-view schematic illustrating a portion of an analyte sensor having a plurality of substantially planar layers.
Fig. 29 is a perspective-view schematic illustrating a back end portion of the sensor of Fig. 28.
Fig. 30 is a perspective-view schematic illustrating a sheet having a plurality of planar layers.
Fig. 31 is a side-view schematic illustrating singulating equipment for generating analyte sensor reels from the sheet of Fig. 30.
Fig. 32 is a perspective-view schematic illustrating a process for generating analyte sensors with rounded edges from the sheet of Fig. 30.
Fig. 33 is a perspective-view schematic illustrating a singulated sensor structure with a counter-electrode.
Fig. 34A is a perspective-view schematic illustrating an analyte sensor with a counter-electrode and rounded edges formed from the singulated sensor structure of Fig. 33.
Fig. 34B is a perspective-view schematic illustrating another singulated analyte sensor.
Fig. 35 shows a pair of graphs illustrating exemplary background noise reduction characteristics of a flexible analyte sensor using sputter coated platinum as an electrode material.
Fig. 36 shows a graph illustrating exemplary bending characteristics of a flexible analyte sensor.
Fig. 37 illustrates a cross-sectional end view schematic of a portion of an analyte sensor having an elongated hydrophilic structure.
Fig. 38 illustrates cross-sectional end view schematics of a portion of an analyte sensor undergoing a chemical/biological softening process.
Fig. 39 illustrates a perspective-view schematic of a portion of an analyte sensor having chemical/biological softening structures.
Fig. 40 illustrates a schematic diagram of a portion of an analyte sensor undergoing dip coating operations in a freestanding configuration.
Fig. 41 illustrates a schematic diagram of a portion of an analyte sensor undergoing unsuccessful dip coating operations in a non-freestanding configuration.
Fig. 42 illustrates a side-view schematic of a portion of an analyte sensor extending from a housing in a freestanding configuration generated by an applied electromagnetic field.
Fig. 43 illustrates a side-view schematic of a portion of an analyte sensor extending from a housing in a non-freestanding configuration.
Fig. 44 illustrates perspective-view schematic of a magnetic field control structure for a continuous analyte sensor system.
Fig. 45 illustrates perspective-view schematic of an electromagnetic field control structure for a continuous analyte sensor system.
Fig. 46 shows a linear-scale graph illustrating exemplary fatigue characteristics of various flexible analyte sensors.
Fig. 47 shows a log-scale graph illustrating exemplary fatigue characteristics of various flexible analyte sensors.
FIG. 48 shows a graph illustrating exemplary buckling force characteristics of flexible analyte sensors.

Like reference numerals refer to like elements throughout. Elements are not to scale unless otherwise noted.

### DETAILED DESCRIPTION

The following description and examples illustrate some exemplary implementations, embodiments, and arrangements of the disclosed invention in detail. Those of skill in the art will recognize that there are numerous variations and modifications of this invention that are encompassed by its scope. Accordingly, the description of a certain example embodiment should not be deemed to limit the scope of the present invention.

### Definitions

In order to facilitate an understanding of the various embodiments described herein, a number of terms are defined below.

The term "analyte" as used herein is a broad term and is to be given its ordinary and customary meaning to a person of ordinary skill in the art (and is not to be limited to a special or customized meaning), and furthermore refers without limitation to a substance or chemical constituent in a biological fluid (for example, blood, interstitial fluid, cerebral spinal fluid, lymph fluid or urine) that can be analyzed. Analytes can include naturally occurring substances, artificial substances, metabolites, and/or reaction products. In some embodiments, the analyte for measurement by the sensor heads, devices, and methods is analyte. However, other analytes are contemplated as well, including but not limited to acarboxyprothrombin; acylcarnitine; adenine phosphoribosyl transferase; adenosine deaminase; albumin; alpha-fetoprotein; amino acid profiles (arginine (Krebs cycle), histidine/urocanic acid, homocysteine, phenylalanine/tyrosine, tryptophan); andrenostenedione; antipyrine; arabinitol enantiomers; arginase; benzoylecgonine (cocaine); biotinidase; biopterin; c-reactive protein; carnitine; carnosinase; CD4; ceruloplasmin; chenodeoxycholic acid; chloroquine; cholesterol; cholinesterase; conjugated 1-β hydroxy-cholic acid; cortisol; creatine kinase; creatine kinase MM isoenzyme; cyclosporin A; D-penicillamine; de-ethylchloroquine; dehydroepiandrosterone sulfate; DNA (acetylator polymorphism, alcohol dehydrogenase, alpha 1-antitrypsin, cystic fibrosis, Duchenne/Becker muscular dystrophy, analyte-6-phosphate dehydrogenase, hemoglobin A, hemoglobin S, hemoglobin C, hemoglobin D, hemoglobin E, hemoglobin F, D-Punjab, beta-thalassemia, hepatitis B virus, HCMV, HIV-1, HTLV-1, Leber hereditary optic neuropathy, MCAD, RNA, PKU, Plasmodium vivax, sexual differentiation, 21-deoxycortisol); desbutylhalofantrine; dihydropteridine reductase; diptheria/tetanus antitoxin; erythrocyte arginase; erythrocyte protoporphyrin; esterase D; fatty acids/acylglycines; free β-human chorionic gonadotropin; free erythrocyte porphyrin; free thyroxine (FT4); free tri-iodothyronine (FT3); fumarylacetoacetase; galactose/gal-1-phosphate; galactose-1-phosphate uridyltransferase; gentamicin; analyte-6-phosphate dehydrogenase; glutathione; glutathione perioxidase; glycocholic acid; glycosylated hemoglobin; halofantrine; hemoglobin variants; hexosaminidase A; human erythrocyte carbonic anhydrase I; 17-alpha-hydroxyprogesterone; hypoxanthine phosphoribosyl transferase; immunoreactive trypsin; lactate; lead; lipoproteins ((a), B/A-1, β); lysozyme; mefloquine; netilmicin; phenobarbitone; phenytoin; phytanic/pristanic acid; progesterone; prolactin; prolidase; purine nucleoside phosphorylase; quinine; reverse tri-iodothyronine (rT3); selenium; serum pancreatic lipase; sissomicin; somatomedin C; specific antibodies (adenovirus, anti-nuclear antibody, anti-zeta antibody, arbovirus, Aujeszky's disease virus, dengue virus, Dracunculus medinensis, Echinococcus granulosus, Entamoeba histolytica, enterovirus, Giardia duodenalisa, Helicobacter pylori, hepatitis B virus, herpes virus, HIV-1, IgE (atopic disease), influenza virus, Leishmania donovani, leptospira, measles/mumps/rubella, Mycobacterium leprae, Mycoplasma pneumoniae, Myoglobin, Onchocerca volvulus, parainfluenza virus, Plasmodium falciparum, poliovirus, Pseudomonas aeruginosa, respiratory syncytial virus, rickettsia (scrub typhus), Schistosoma mansoni, Toxoplasma gondii, Trepenoma pallidium, Trypanosoma cruzi/rangeli, vesicular stomatis virus, Wuchereria bancrofti, yellow fever virus); specific antigens (hepatitis B virus, HIV-1); succinylacetone; sulfadoxine; theophylline; thyrotropin (TSH); thyroxine (T4); thyroxine-binding globulin; trace elements; transferrin; UDP-galactose-4-epimerase; urea; uroporphyrinogen I synthase; vitamin A; white blood cells; and zinc protoporphyrin. Salts, sugar, protein, fat, vitamins, and hormones naturally occurring in blood or interstitial fluids can also constitute analytes in certain embodiments. The analyte can be naturally present in the biological fluid, for example, a metabolic product, a hormone, an antigen, an antibody, and the like. Alternatively, the analyte can be introduced into the body, for example, a contrast agent for imaging, a radioisotope, a chemical agent, a fluorocarbon-based synthetic blood, or a drug or pharmaceutical composition, including but not limited to insulin; ethanol; cannabis (marijuana, tetrahydrocannabinol, hashish); inhalants (nitrous oxide, amyl nitrite, butyl nitrite, chlorohydrocarbons, hydrocarbons); cocaine (crack cocaine); stimulants (amphetamines, methamphetamines, Ritalin, Cylert, Preludin, Didrex, PreState, Voranil, Sandrex, Plegine); depressants (barbituates, methaqualone, tranquilizers such as Valium, Librium, Miltown, Serax, Equanil, Tranxene); hallucinogens (phencyclidine, lysergic acid, mescaline, peyote, psilocybin); narcotics (heroin, codeine, morphine, opium, meperidine, Percocet, Percodan, Tussionex, Fentanyl, Darvon, Talwin, Lomotil); designer drugs (analogs of fentanyl, meperidine, amphetamines, methamphetamines, and phencyclidine, for example, Ecstasy); anabolic steroids; and nicotine. The metabolic products of drugs and pharmaceutical compositions are also contemplated analytes. Analytes such as neurochemicals and other chemicals generated within the body can also be analyzed, such as, for example, ascorbic acid, uric acid, dopamine, noradrenaline, 3-methoxytyramine (3MT), 3,4-Dihydroxyphenylacetic acid (DOPAC), Homovanillic acid (HVA), 5-Hydroxytryptamine (SHT), and 5-Hydroxyindoleacetic acid (FHIAA).

The terms "microprocessor" and "processor" as used herein are broad terms and are to be given their ordinary and customary meaning to a person of ordinary skill in the art (and are not to be limited to a special or customized meaning), and furthermore refer without limitation to a computer system, state machine, and the like that performs arithmetic and logic operations using logic circuitry that responds to and processes the basic instructions that drive a computer.

The term "calibration" as used herein is a broad term and is to be given its ordinary and customary meaning to a person of ordinary skill in the art (and is not to be limited to a special or customized meaning), and furthermore refers without limitation to the process of determining the relationship between the sensor data and the corresponding reference data, which can be used to convert sensor data into meaningful values substantially equivalent to the reference data, with or without utilizing reference data in real time. In some embodiments, namely, in continuous analyte sensors, calibration can be updated or recalibrated (at the factory, in real time and/or retrospectively) over time as changes in the relationship between the sensor data and reference data occur, for example, due to changes in sensitivity, baseline, transport, metabolism, and the like.

The terms "calibrated data" and "calibrated data stream" as used herein are broad terms and are to be given their ordinary and customary meaning to a person of ordinary skill in the art (and are not to be limited to a special or customized meaning), and furthermore refer without limitation to data that has been transformed from its raw state to another state using a function, for example a conversion function, including by use of a sensitivity, to provide a meaningful value to a user.

The term "algorithm" as used herein is a broad term and is to be given its ordinary and customary meaning to a person of ordinary skill in the art (and is not to be limited to a special or customized meaning), and furthermore refers without limitation to a computational process (for example, programs) involved in transforming information from one state to another, for example, by using computer processing.

The term "counts" as used herein is a broad term and is to be given its ordinary and customary meaning to a person of ordinary skill in the art (and is not to be limited to a special or customized meaning), and furthermore refers without limitation to a unit of measurement of a digital signal. In one example, a raw data stream measured in counts is directly related to a voltage (e.g., converted by an A/D converter), which is directly related to current from the working electrode.

The term "sensor" as used herein is a broad term and is to be given its ordinary and customary meaning to a person of ordinary skill in the art (and is not to be limited to a special or customized meaning), and furthermore refers without limitation to the component or region of a device by which an analyte can be quantified. A "lot" of sensors generally refers to a group of sensors that are manufactured on or around the same day and using the same processes and tools/materials.

The terms "glucose sensor" and "member for determining the amount of glucose in a biological sample" as used herein are broad terms and are to be given their ordinary and customary meaning to a person of ordinary skill in the art (and are not to be limited to a special or customized meaning), and furthermore refer without limitation to any mechanism (e.g., enzymatic or non-enzymatic) by which glucose can be quantified. For example, some embodiments utilize a membrane that contains glucose oxidase that catalyzes the conversion of oxygen and glucose to hydrogen peroxide and gluconate, as illustrated by the following chemical reaction:

Glucose + O₂→Gluconate + H₂O₂

Because for each glucose molecule metabolized, there is a proportional change in the co-reactant O₂ and the product H₂O₂, one can use an electrode to monitor the current change in either the co-reactant or the product to determine glucose concentration.

The terms "operably connected" and "operably linked" as used herein are broad terms and are to be given their ordinary and customary meaning to a person of ordinary skill in the art (and are not to be limited to a special or customized meaning), and furthermore refer without limitation to one or more components being linked to another component(s) in a manner that allows transmission of signals between the components. For example, one or more electrodes can be used to detect the amount of glucose in a sample and convert that information into a signal, e.g., an electrical or electromagnetic signal; the signal can then be transmitted to an electronic circuit. In this case, the electrode is "operably linked" to the electronic circuitry. These terms are broad enough to include wireless connectivity.

The term "determining" encompasses a wide variety of actions. For example, "determining" may include calculating, computing, processing, deriving, investigating, looking up (e.g., looking up in a table, a database or another data structure), ascertaining and the like. Also, "determining" may include receiving (e.g., receiving information), accessing (e.g., accessing data in a memory) and the like. Also, "determining" may include resolving, selecting, choosing, calculating, deriving, establishing and/or the like. Determining may also include ascertaining that a parameter matches a predetermined criterion, including that a threshold has been met, passed, exceeded, and so on.

The term "substantially" as used herein is a broad term and is to be given its ordinary and customary meaning to a person of ordinary skill in the art (and is not to be limited to a special or customized meaning), and furthermore refers without limitation to being largely but not necessarily wholly that which is specified.

The term "host" as used herein is a broad term and is to be given its ordinary and customary meaning to a person of ordinary skill in the art (and is not to be limited to a special or customized meaning), and furthermore refers without limitation to mammals, particularly humans.

The term "continuous analyte (or glucose) sensor" as used herein is a broad term and is to be given its ordinary and customary meaning to a person of ordinary skill in the art (and is not to be limited to a special or customized meaning), and furthermore refers without limitation to a device that continuously or continually measures a concentration of an analyte, for example, at time intervals ranging from fractions of a second up to, for example, 1, 2, or 5 minutes, or longer. In one exemplary embodiment, the continuous analyte sensor is a glucose sensor such as described in U.S. Patent 6,001,067, which is incorporated herein by reference in its entirety.

The term "continuous analyte (or glucose) sensing" as used herein is a broad term and is to be given its ordinary and customary meaning to a person of ordinary skill in the art (and is not to be limited to a special or customized meaning), and furthermore refers without limitation to the period in which monitoring of an analyte is continuously or continually performed, for example, at time intervals ranging from fractions of a second up to, for example, 1, 2, or 5 minutes, or longer.

The term "sensing membrane" as used herein is a broad term and is to be given its ordinary and customary meaning to a person of ordinary skill in the art (and is not to be limited to a special or customized meaning), and furthermore refers without limitation to a permeable or semi-permeable membrane that can be comprised of two or more domains and is typically constructed of materials of a few microns thickness or more, which are permeable to oxygen and may or may not be permeable to glucose. In one example, the sensing membrane comprises an immobilized glucose oxidase enzyme, which enables an electrochemical reaction to occur to measure a concentration of glucose.

The term "sensor data," as used herein is a broad term and is to be given its ordinary and customary meaning to a person of ordinary skill in the art (and are not to be limited to a special or customized meaning), and furthermore refers without limitation to any data associated with a sensor, such as a continuous analyte sensor. Sensor data includes a raw data stream, or simply data stream, of analog or digital signals directly related to a measured analyte from an analyte sensor (or other signal received from another sensor), as well as calibrated and/or filtered raw data. In one example, the sensor data comprises digital data in "counts" converted by an A/D converter from an analog signal (e.g., voltage or amps) and includes one or more data points representative of a glucose concentration. Thus, the terms "sensor data point" and "data point" refer generally to a digital representation of sensor data at a particular time. The terms broadly encompass a plurality of time spaced data points from a sensor, such as from a substantially continuous glucose sensor, which comprises individual measurements taken at time intervals ranging from fractions of a second up to, e.g., 1, 2, or 5 minutes or longer. In another example, the sensor data includes an integrated digital value representative of one or more data points averaged over a time period. Sensor data may include calibrated data, smoothed data, filtered data, transformed data, and/or any other data associated with a sensor.

The term "sensor electronics," as used herein, is a broad term, and is to be given its ordinary and customary meaning to a person of ordinary skill in the art (and is not to be limited to a special or customized meaning), and refers without limitation to the components (for example, hardware and/or software) of a device configured to process data.

The terms "sensitivity" or "sensor sensitivity," as used herein, are broad terms, and are to be given their ordinary and customary meaning to a person of ordinary skill in the art (and is not to be limited to a special or customized meaning), and refer without limitation to an amount of signal produced by a certain concentration of a measured analyte, or a measured species (e.g., H2O2) associated with the measured analyte (e.g., glucose). For example, in one embodiment, a sensor has a sensitivity of from about 1 to about 300 picoAmps of current for every 1 mg/dL of glucose analyte.

The term "sample," as used herein, is a broad term, and is to be given its ordinary and customary meaning to a person of ordinary skill in the art (and it is not to be limited to a special or customized meaning), and refers without limitation to a sample of a host body, for example, body fluids, including, blood, serum, plasma, interstitial fluid, cerebral spinal fluid, lymph fluid, ocular fluid, saliva, oral fluid, urine, excretions, or exudates.

The term "calibration," as used herein, is a broad term, and is to be given its ordinary and customary meaning to a person of ordinary skill in the art (and is not to be limited to a special or customized meaning), and refers without limitation to the relationship and/or process of determining the relationship between the sensor data and the corresponding reference data. In some embodiments, namely, in continuous analyte sensors, calibration can be updated or recalibrated over time if changes in the relationship between the sensor data and reference data occur, for example, due to changes in sensitivity, baseline, transport, metabolism, and the like.

The terms "continuous" and "continuously," as used herein in reference to analyte sensing, are broad terms, and are to be given their ordinary and customary meaning to a person of ordinary skill in the art (and is not to be limited to a special or customized meaning), and refer without limitation to the continuous, continual, or intermittent (e.g., regular) monitoring of analyte concentration, such as, for example, performing a measurement about every 1 to 10 minutes. It should be understood that continuous analyte sensors generally continually measure the analyte concentration without required user initiation and/or interaction for each measurement, such as described with reference to continuous glucose sensors in U.S. Pat. No. 6,001,067, for example. These terms include situations wherein data gaps can exist (e.g., when a continuous glucose sensor is temporarily not providing data).

The term "count," as used herein, is a broad term, and is to be given its ordinary and customary meaning to a person of ordinary skill in the art (and is not to be limited to a special or customized meaning), and refers without limitation to a unit of measurement of a digital signal. For example, a raw data stream or raw data signal measured in counts is directly related to a voltage (for example, converted by an A/D converter), which is directly related to current from the working electrode. In some embodiments, the terms can refer to data that has been integrated or averaged over a time period (e.g., 5 minutes).

The term "crosslink," as used herein, is a broad term, and is to be given its ordinary and customary meaning to a person of ordinary skill in the art (and is not to be limited to a special or customized meaning), and refers without limitation to the formation of bonds (e.g., covalent bonds, ionic bonds, hydrogen bonds, etc.) that link one polymer (or oligomer) chain to another, or to a process that increases the cohesiveness of one polymer (or oligomer) chain to another. Crosslinks can be formed, e.g., through various reactions or processes, e.g., chemical processes initiated by heat, pressure, catalysts, radiation, and the like.

The term "distal to," as used herein, is a broad term, and is to be given its ordinary and customary meaning to a person of ordinary skill in the art (and is not to be limited to a special or customized meaning), and refers without limitation to the spatial relationship between various elements in comparison to a particular point of reference. In general, the term indicates an element is located relatively far from the reference point than another element.

The term "proximal to," as used herein, is a broad term, and is to be given its ordinary and customary meaning to a person of ordinary skill in the art (and is not to be limited to a special or customized meaning), and refers without limitation to the spatial relationship between various elements in comparison to a particular point of reference. In general, the term indicates an element is located relatively near to the reference point than another element.

The terms "electrochemically reactive surface" and "electroactive surface," as used herein, are broad terms, and are to be given their ordinary and customary meaning to a person of ordinary skill in the art (and are not to be limited to a special or customized meaning), and refer without limitation to a surface where an electrochemical reaction takes place. As a nonlimiting example, in an electrochemical glucose sensor, a working electrode measures hydrogen peroxide, H2O2, at its electroactive surface. The hydrogen peroxide is produced by the enzyme-catalyzed reaction of the analyte detected, which reacts with the electroactive surface to create a detectable electric current. For example, glucose can be detected utilizing glucose oxidase (GOX), which produces hydrogen peroxide as a byproduct. Hydrogen peroxide reacts with the surface of the working electrode (e.g., the electroactive surface), producing two protons (2H+), two electrons (2e-) and one molecule of oxygen (O2), which produces the electronic current being detected.

The terms "electrical connection" and "electrical contact," as used herein, are broad terms, and are to be given their ordinary and customary meaning to a person of ordinary skill in the art (and are not to be limited to a special or customized meaning), and refer without limitation to any connection between two electrical conductors known to those in the art. In one embodiment, electrodes are in electrical connection with (e.g., electrically connected to) the electronic circuitry of a device. In another embodiment, two materials, such as but not limited to two metals, can be in electrical contact with each other, such that an electrical current can pass from one of the two materials to the other material.

The terms "electronics," "sensor electronics," and "system electronics," as used herein, are broad terms, and are to be given their ordinary and customary meaning to a person of ordinary skill in the art (and are not to be limited to a special or customized meaning), and refer without limitation to electronics operatively coupled to the sensor and configured to measure, process, receive, and/or transmit data associated with a sensor, and/or electronics configured to communicate with a flow control device and to control and/or monitor fluid metering by a flow control device.

The term "elongated conductive body," as used herein, is a broad term, and is to be given its ordinary and customary meaning to a person of ordinary skill in the art (and is not to be limited to a special or customized meaning), and refers without limitation to an elongated body formed at least in part of a conductive material and includes any number of coatings that may be formed thereon. By way of example, an "elongated conductive body" may mean a bare elongated conductive core (e.g., a metal wire), an elongated conductive core coated with one, two, three, four, five, or more layers of material, each of which may or may not be conductive, or an elongated non-conductive core with conductive coatings, traces, and/or electrodes thereon and coated with one, two, three, four, five, or more layers of material, each of which may or may not be conductive.

The term "host," as used herein, is a broad term, and is to be given its ordinary and customary meaning to a person of ordinary skill in the art (and is not to be limited to a special or customized meaning), and refers without limitation to plants or animals, for example humans.

The term "ex vivo portion," as used herein, is a broad term, and is to be given its ordinary and customary meaning to a person of ordinary skill in the art (and is not to be limited to a special or customized meaning), and refers without limitation to a portion of a device (for example, a sensor) adapted to remain and/or exist outside of a living body of a host.

The term "in vivo portion," as used herein, is a broad term, and is to be given its ordinary and customary meaning to a person of ordinary skill in the art (and is not to be limited to a special or customized meaning), and refers without limitation to a portion of a device (for example, a sensor) adapted for insertion into and/or existence within a living body of a host.

The term "multi-axis bending," as used herein, is a broad term, and is to be given its ordinary and customary meaning to a person of ordinary skill in the art (and is not to be limited to a special or customized meaning), and refers without limitation to a preference for bending in more than one plane or about more than one axis.

The terms "operatively connected," "operatively linked," "operably connected," and "operably linked" as used herein, are broad terms, and are to be given their ordinary and customary meaning to a person of ordinary skill in the art (and are not to be limited to a special or customized meaning), and refer without limitation to one or more components linked to one or more other components. The terms can refer to a mechanical connection, an electrical connection, or any connection that allows transmission of signals between the components. For example, one or more electrodes can be used to detect the amount of analyte in a sample and to convert that information into a signal; the signal can then be transmitted to a circuit. In such an example, the electrode is "operably linked" to the electronic circuitry. The terms include wired and wireless connections.

The term "potentiostat," as used herein, is a broad term, and is to be given its ordinary and customary meaning to a person of ordinary skill in the art (and is not to be limited to a special or customized meaning), and refers without limitation to an electronic instrument that controls the electrical potential between the working and reference electrodes at one or more preset values.

The term "processor module," as used herein, is a broad term, and is to be given its ordinary and customary meaning to a person of ordinary skill in the art (and are not to be limited to a special or customized meaning), and refers without limitation to a computer system, state machine, processor, components thereof, and the like designed to perform arithmetic or logic operations using logic circuitry that responds to and processes the basic instructions that drive a computer.

The terms "raw data," "raw data stream," "raw data signal," "data signal," and "data stream," as used herein, are broad terms, and are to be given their ordinary and customary meaning to a person of ordinary skill in the art (and are not to be limited to a special or customized meaning), and refer without limitation to an analog or digital signal from the analyte sensor directly related to the measured analyte. For example, the raw data stream is digital data in "counts" converted by an A/D converter from an analog signal (for example, voltage or amps) representative of an analyte concentration. The terms can include a plurality of time spaced data points from a substantially continuous analyte sensor, each of which comprises individual measurements taken at time intervals ranging from fractions of a second up to, for example, 1, 2, or 5 minutes or longer. In some embodiments, the terms can refer to data that has been integrated or averaged over a time period (e.g., 5 minutes).

The terms "sensor" and "sensor system," as used herein, are broad terms, and are to be given their ordinary and customary meaning to a person of ordinary skill in the art (and are not to be limited to a special or customized meaning), and refer without limitation to a device, component, or region of a device by which an analyte can be quantified.

The term "sensor session," as used herein, is a broad term and is to be given its ordinary and customary meaning to a person of ordinary skill in the art (and is not to be limited to a special or customized meaning), and refers without limitation to a period of time a sensor is in use, such as but not limited to a period of time starting at the time the sensor is implanted (e.g., by the host) to removal of the sensor (e.g., removal of the sensor from the host's body and/or removal of (e.g., disconnection from) system electronics).

The terms "membrane system" and "membrane," as used herein, are broad terms, and are to be given their ordinary and customary meaning to a person of ordinary skill in the art (and are not to be limited to a special or customized meaning), and refer without limitation to a permeable or semi-permeable membrane that can comprise one or more layers and constructed of materials, which are permeable to oxygen and may or may not be permeable to an analyte of interest. In one example, the membrane system comprises an immobilized glucose oxidase enzyme, which enables an electrochemical reaction to occur to measure a concentration of glucose.

The terms "substantial" and "substantially," as used herein, are broad terms, and are to be given their ordinary and customary meaning to a person of ordinary skill in the art (and are not to be limited to a special or customized meaning), and refer without limitation to a sufficient amount that provides a desired function.

Other definitions will be provided within the description below, and in some cases from the context of the term's usage.

As employed herein, the following abbreviations apply: Eq and Eqs (equivalents); mEq (milliequivalents); M (molar); mM (millimolar) µM (micromolar); N (Normal); mol (moles); mmol (millimoles); µmol (micromoles); nmol (nanomoles); g (grams); mg (milligrams); µg (micrograms); Kg (kilograms); L (liters); mL (milliliters); dL (deciliters); µL (microliters); cm (centimeters); mm (millimeters); µm (micrometers); nm (nanometers); h and hr (hours); min. (minutes); s and sec. (seconds); °C (degrees Centigrade) °F (degrees Fahrenheit), Pa (Pascals), kPa (kiloPascals), MPa (megaPascals), GPa (gigaPascals), Psi (pounds per square inch), kPsi (kilopounds per square inch).

### Overview / General Description of System

In vivo continuous analyte sensing technology may rely on in vivo sensors that have a stiffness (e.g., a stiffness defined by a Young's modulus, a flexural modulus, and/or a buckling force) that is determined based on a minimum stiffness for successful coating of the sensor in a membrane for facilitating glucose concentration measurements.

The Young's modulus, which is also known as the elastic modulus, is a mechanical property of linear elastic solid materials, which defines the relationship between stress (force per unit area) and strain (proportional deformation) in a material. The flexural modulus is an intensive property of an object that can be computed as the ratio of stress to strain in flexural deformation, or the tendency for a material to bend. The flexural modulus of an object can be determined from the slope of a stress-strain curve produced by a flexural test (such as the ASTM D 790 flexural text), and can be expressed units of force per area. For isotropic materials such as metals in some arrangements, the Young's modulus and the flexural modules can be the same. However, for some objects, such as elongated polymer objects, the flexural modulus can be different from the Young's modulus. The buckling force may be defined as the maximum compressive load that can be applied before an object buckles.

For example, a metal-clad, tantalum wire is sometimes used as a core bare sensing element for a continuous analyte sensor. This sensing element is coated in membranes to yield the final sensor. The stiffness of the sensor as a whole in these configurations can be much greater than the surrounding skin and adipose tissue. It has been discovered that a stiffness mismatch of this type can cause a fundamental compliance mismatch that can exacerbate a foreign body giant cell response to the sensor in vivo. Essentially, due to the differences in rigidities of the sensor and the surrounding tissue, with increased motion, there can be increased stress at the interface between the tissue and sensor. Since the sensor is more rigid, this can cause additional trauma to the surrounding tissue after initial injury, thus reactivating an inflammatory cascade.

A wide range of elastic modulus has been reported for the various skin layers: 5-1000 MPa with decreasing hydration for stratum corneum, 56-260 kPa for dermis, and 0.12-23 kPa for SubQ tissue. The Young's modulus of a tantalum core sensor can be as high as 27 x 10^6 psi or 186 GPa, which is more than 6000 times the Young's modules of viable skin tissue (e.g., a Young's modulus of between 0.1-260 kPa, for tissue excluding the stratum corneum). This poses a great challenge for biomechanical compatibility.

Described herein are flexible analyte sensors for continuous analyte monitoring with enhanced biocompatibility. The systems and methods described herein provide a reduced modulus-mismatch sensor that includes a flexible and tissue compliant core to improve the device-tissue interaction. The flexible analyte sensors discussed herein provide improved biocompatibility while maintaining mechanical characteristics that facilitate manufacturing operations such as coating processes for the sensors. The flexible analyte sensors described herein may reduce post-insertion injury from micro-motion and minimize sensor noise caused by tissue trauma.

The following description and examples described the present embodiments with reference to the drawings. In the drawings, reference numbers label elements of the present embodiments. These reference numbers are reproduced below in connection with the discussion of the corresponding drawing features.

### Sensor System

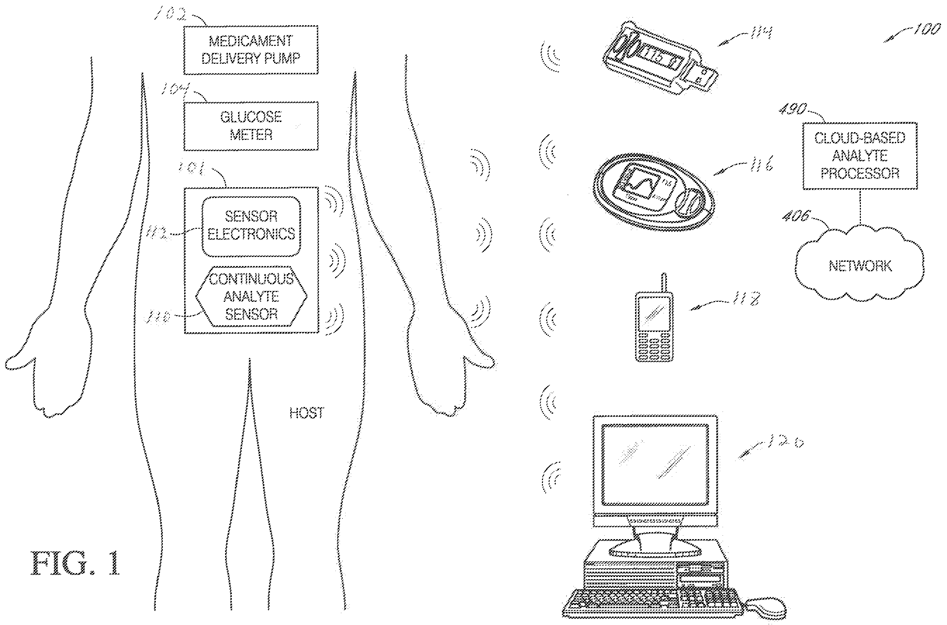

Fig. 1 depicts an example system 100, in accordance with some example implementations. The system 100 includes a continuous analyte sensor system 101 including sensor electronics 112 and a continuous analyte sensor 110. The system 100 may include other devices and/or sensors, such as medicament delivery pump 102 and glucose meter 104. The continuous analyte sensor 110 may be physically connected to sensor electronics 112 and may be integral with (e.g., non-releasably attached to) or releasably attachable to the continuous analyte sensor 110. The sensor electronics 112, medicament delivery pump 102, and/or glucose meter 104 may couple with one or more devices, such as display devices 114, 116, 118, and/or 120.

In some example implementations, the system 100 may include a cloud-based analyte processor 490 configured to analyze analyte data (and/or other patient-related data) provided via network 406 (e.g., via wired, wireless, or a combination thereof) from sensor system 101 and other devices, such as display devices 114, 116, 118, and/or 120 and the like, associated with the host (also referred to as a patient) and generate reports providing high-level information, such as statistics, regarding the measured analyte over a certain time frame. A full discussion of using a cloud-based analyte processing system may be found in U.S. Patent Application No. 13/788,375, entitled "Cloud-Based Processing of Analyte Data" and filed on March 7, 2013, herein incorporated by reference in its entirety. In some implementations, one or more steps of the factory calibration algorithm can be performed in the cloud.

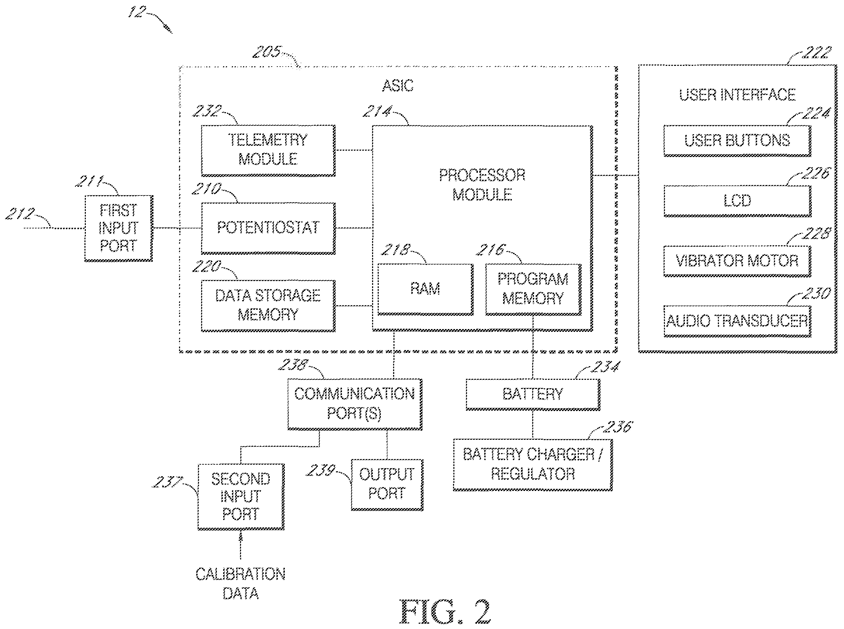

In some example implementations, the sensor electronics 112 may include electronic circuitry associated with measuring and processing data generated by the continuous analyte sensor 110. This generated continuous analyte sensor data may also include algorithms, which can be used to process and calibrate the continuous analyte sensor data, although these algorithms may be provided in other ways as well. The sensor electronics 112 may include hardware, firmware, software, or a combination thereof, to provide measurement of levels of the analyte via a continuous analyte sensor, such as a continuous glucose sensor. An example implementation of the sensor electronics 112 is described further below with respect to Fig. 2.

In one implementation, the factory calibration algorithms described herein may be performed by the sensor electronics.

The sensor electronics 112 may, as noted, couple (e.g., wirelessly and the like) with one or more devices, such as display devices 114, 116, 118, and/or 120. The display devices 114, 116, 118, and/or 120 may be configured for presenting information (and/or alarming), such as sensor information transmitted by the sensor electronics 112 for display at the display devices 114, 116,118, and/or 120.

The display devices may include a relatively small, key fob-like display device 114, a relatively large, hand-held display device 116, a cellular phone 118 (e.g., a smart phone, a tablet, and the like), a computer 120, and/or any other user equipment configured to at least present information (e.g., medicament delivery information, discrete self-monitoring glucose readings, heart rate monitor, caloric intake monitor, and the like).

In one implementation, the factory calibration algorithms described herein may be performed at least in part by the display devices.

In some example implementations, the relatively small, key fob-like display device 114 may comprise a wrist watch, a belt, a necklace, a pendent, a piece of jewelry, an adhesive patch, a pager, a key fob, a plastic card (e.g., credit card), an identification (ID) card, and/or the like. This small display device 114 may include a relatively small display (e.g., smaller than the large display device 116) and may be configured to display certain types of displayable sensor information, such as a numerical value, and an arrow, or a color code.

In some example implementations, the relatively large, hand-held display device 116 may comprise a hand-held receiver device, a palm-top computer, and/or the like. This large display device may include a relatively larger display (e.g., larger than the small display device 114) and may be configured to display information, such as a graphical representation of the continuous sensor data including current and historic sensor data output by sensor system 100.

In some example implementations, the continuous analyte sensor 110 comprises a sensor for detecting and/or measuring analytes, and the continuous analyte sensor 110 may be configured to continuously detect and/or measure analytes as a non-invasive device, a subcutaneous device, a transdermal device, and/or an intravascular device. In some example implementations, the continuous analyte sensor 110 may analyze a plurality of intermittent blood samples, although other analytes may be used as well.

In some example implementations, the continuous analyte sensor 110 may comprise a glucose sensor configured to measure glucose in the blood or interstitial fluid using one or more measurement techniques, such as enzymatic, chemical, physical, electrochemical, spectrophotometric, polarimetric, calorimetric, iontophoretic, radiometric, immunochemical, and the like. In implementations in which the continuous analyte sensor 110 includes a glucose sensor, the glucose sensor may comprise any device capable of measuring the concentration of glucose and may use a variety of techniques to measure glucose including invasive, minimally invasive, and non-invasive sensing techniques (e.g., fluorescence monitoring), to provide data, such as a data stream, indicative of the concentration of glucose in a host. The data stream may be sensor data (raw and/or filtered), which may be converted into a calibrated data stream used to provide a value of glucose to a host, such as a user, a patient, or a caretaker (e.g., a parent, a relative, a guardian, a teacher, a doctor, a nurse, or any other individual that has an interest in the wellbeing of the host). Moreover, the continuous analyte sensor 110 may be implanted as at least one of the following types of flexible analyte sensors: an implantable glucose sensor, a transcutaneous glucose sensor, implanted in a host vessel or extracorporeally, a subcutaneous sensor, a refillable subcutaneous sensor, an intravascular sensor.

Although the disclosure herein refers to some implementations that include a continuous analyte sensor 110 comprising a glucose sensor, the continuous analyte sensor 110 may comprise other types of flexible analyte sensors as well. Moreover, although some implementations refer to the glucose sensor as an implantable glucose sensor, other types of devices capable of detecting a concentration of glucose and providing an output signal representative of glucose concentration may be used as well. Furthermore, although the description herein refers to glucose as the analyte being measured, processed, and the like, other analytes may be used as well including, for example, ketone bodies (e.g., acetone, acetoacetic acid and beta hydroxybutyric acid, lactate, etc.), glucagon, acetyl-CoA, triglycerides, fatty acids, intermediaries in the citric acid cycle, choline, insulin, cortisol, testosterone, and the like.

Fig. 2 depicts an example of sensor electronics 112, in accordance with some example implementations. The sensor electronics 112 may include sensor electronics that are configured to process sensor information, such as sensor data, and generate transformed sensor data and displayable sensor information, e.g., via a processor module. For example, the processor module may transform sensor data into one or more of the following: filtered sensor data (e.g., one or more filtered analyte concentration values), raw sensor data, calibrated sensor data (e.g., one or more calibrated analyte concentration values), rate of change information, trend information, rate of acceleration/deceleration information, sensor diagnostic information, location information, alarm; alert information, calibration information such as may be determined by factory calibration algorithms as disclosed herein, smoothing and/or filtering algorithms of sensor data, and/or the like.

In some embodiments, a processor module 214 is configured to achieve a substantial portion, if not all, of the data processing, including data processing pertaining to factory calibration. Processor module 214 may be integral to sensor electronics 12 and/or may be located remotely, such as in one or more of devices 114, 116, 118, and/or 120 and/or cloud 490. In some embodiments, processor module 214 may comprise a plurality of smaller subcomponents or submodules. For example, processor module 214 may include an alert module (not shown) or prediction module (not shown), or any other suitable module that may be utilized to efficiently process data. When processor module 214 is made up of a plurality of submodules, the submodules may be located within processor module 214, including within the sensor electronics 112 or other associated devices (e.g., 114, 116, 118, 120 and/or 490). For example, in some embodiments, processor module 214 may be located at least partially within a cloud-based analyte processor 490 or elsewhere in network 406.

In some example implementations, the processor module 214 may be configured to calibrate the sensor data, and the data storage memory 220 may store the calibrated sensor data points as transformed sensor data. Moreover, the processor module 214 may be configured, in some example implementations, to wirelessly receive calibration information from a display device, such as devices 114, 116, 118, and/or 120, to enable calibration of the sensor data from sensor 110. Furthermore, the processor module 214 may be configured to perform additional algorithmic processing on the sensor data (e.g., calibrated and/or filtered data and/or other sensor information), and the data storage memory 220 may be configured to store the transformed sensor data and/or sensor diagnostic information associated with the algorithms. The processor module 214 may further be configured to store and use calibration information determined from a factory calibration, as described below.

In some example implementations, the sensor electronics 112 may comprise an application-specific integrated circuit (ASIC) 205 coupled to a user interface 222. The ASIC 205 may further include a potentiostat 210, a telemetry module 232 for transmitting data from the sensor electronics 112 to one or more devices, such as devices 114, 116, 118, and/or 120, and/or other components for signal processing and data storage (e.g., processor module 214 and data storage memory 220). Although Fig. 2 depicts ASIC 205, other types of circuitry may be used as well, including field programmable gate arrays (FPGA), one or more microprocessors configured to provide some (if not all of) the processing performed by the sensor electronics 12, analog circuitry, digital circuitry, or a combination thereof.

In the example depicted in Fig. 2, through a first input port 211 for sensor data the potentiostat 210 is coupled to a continuous analyte sensor 110, such as a glucose sensor to generate sensor data from the analyte. The potentiostat 210 may also provide via data line 212 a voltage to the continuous analyte sensor 110 to bias the sensor for measurement of a value (e.g., a current and the like) indicative of the analyte concentration in a host (also referred to as the analog portion of the sensor). The potentiostat 210 may have one or more channels depending on the number of working electrodes at the continuous analyte sensor 110.

In some example implementations, the potentiostat 210 may include a resistor that translates a current value from the sensor 110 into a voltage value, while in some example implementations, a current-to-frequency converter (not shown) may also be configured to integrate continuously a measured current value from the sensor 110 using, for example, a charge-counting device. In some example implementations, an analog-to-digital converter (not shown) may digitize the analog signal from the sensor 110 into so-called "counts" to allow processing by the processor module 214. The resulting counts may be directly related to the current measured by the potentiostat 210, which may be directly related to an analyte level, such as a glucose level, in the host.

The telemetry module 232 may be operably connected to processor module 214 and may provide the hardware, firmware, and/or software that enable wireless communication between the sensor electronics 112 and one or more other devices, such as display devices, processors, network access devices, and the like. A variety of wireless radio technologies that can be implemented in the telemetry module 232 include Bluetooth, Bluetooth Low-Energy, ANT, ANT+, ZigBee, IEEE 802.11, IEEE 802.16, cellular radio access technologies, radio frequency (RF), infrared (IR), paging network communication, magnetic induction, satellite data communication, spread spectrum communication, frequency hopping communication, near field communications, and/or the like. In some example implementations, the telemetry module 232 comprises a Bluetooth chip, although Bluetooth technology may also be implemented in a combination of the telemetry module 232 and the processor module 214.

The processor module 214 may control the processing performed by the sensor electronics 112. For example, the processor module 214 may be configured to process data (e.g., counts), from the sensor, filter the data, calibrate the data, perform fail-safe checking, and/or the like.

In some example implementations, the processor module 214 may comprise a digital filter, such as for example an infinite impulse response (IIR) or a finite impulse response (FIR) filter. This digital filter may smooth a raw data stream received from sensor 110. Generally, digital filters are programmed to filter data sampled at a predetermined time interval (also referred to as a sample rate). In some example implementations, such as when the potentiostat 210 is configured to measure the analyte (e.g., glucose and/or the like) at discrete time intervals, these time intervals determine the sampling rate of the digital filter. In some example implementations, the potentiostat 210 may be configured to measure continuously the analyte, for example, using a current-to-frequency converter. In these current-to-frequency converter implementations, the processor module 214 may be programmed to request, at predetermined time intervals (acquisition time), digital values from the integrator of the current-to-frequency converter. These digital values obtained by the processor module 214 from the integrator may be averaged over the acquisition time due to the continuity of the current measurement. As such, the acquisition time may be determined by the sampling rate of the digital filter.

The processor module 214 may further include a data generator (not shown) configured to generate data packages for transmission to devices, such as the display devices 114, 116, 118, and/or 120. Furthermore, the processor module 214 may generate data packets for transmission to these outside sources via telemetry module 232. In some example implementations, the data packages may, as noted, be customizable for each display device, and/or may include any available data, such as a time stamp, displayable sensor information, transformed sensor data, an identifier code for the sensor and/or sensor electronics 112, raw data, filtered data, calibrated data, rate of change information, trend information, error detection or correction, and/or the like.

The processor module 214 may also include a program memory 216 and other memory 218. The processor module 214 may be coupled to a communications interface, such as a communication port 238, and a source of power, such as a battery 234. Moreover, the battery 234 may be further coupled to a battery charger and/or regulator 236 to provide power to sensor electronics 12 and/or charge the battery 234.

The program memory 216 may be implemented as a semi-static memory for storing data, such as an identifier for a coupled sensor 110 (e.g., a sensor identifier (ID)) and for storing code (also referred to as program code) to configure the ASIC 205 to perform one or more of the operations/functions described herein. For example, the program code may configure processor module 214 to process data streams or counts, filter, perform the calibration methods described below, perform fail-safe checking, and the like.

The memory 218 may also be used to store information. For example, the processor module 214 including memory 218 may be used as the system's cache memory, where temporary storage is provided for recent sensor data received from the sensor. In some example implementations, the memory may comprise memory storage components, such as read-only memory (ROM), random-access memory (RAM), dynamic-RAM, static-RAM, non-static RAM, easily erasable programmable read only memory (EEPROM), rewritable ROMs, flash memory, and the like.

The data storage memory 220 may be coupled to the processor module 214 and may be configured to store a variety of sensor information. In some example implementations, the data storage memory 220 stores one or more days of continuous analyte sensor data. For example, the data storage memory may store 1, 2, 3, 4, 5, 6, 7, 8, 9, 10, 11, 12, 13, 14, 15, 20, and/or 30 (or more days) of continuous analyte sensor data received from sensor 110. The stored sensor information may include one or more of the following: a time stamp, raw sensor data (one or more raw analyte concentration values), calibrated data, filtered data, transformed sensor data, and/or any other displayable sensor information, calibration information (e.g., reference BG values and/or prior calibration information such as from factory calibration), sensor diagnostic information, and the like.

The user interface 222 may include a variety of interfaces, such as one or more buttons 224, a liquid crystal display (LCD) 226, a vibrator 228, an audio transducer (e.g., speaker) 230, a backlight (not shown), and/or the like. The components that comprise the user interface 222 may provide controls to interact with the user (e.g., the host). One or more buttons 224 may allow, for example, toggle, menu selection, option selection, status selection, yes/no response to on-screen questions, a "turn off" function (e.g., for an alarm), an "acknowledged" function (e.g., for an alarm), a reset, and/or the like. The LCD 226 may provide the user with, for example, visual data output. The audio transducer 230 (e.g., speaker) may provide audible signals in response to triggering of certain alerts, such as present and/or predicted hyperglycemic and hypoglycemic conditions. In some example implementations, audible signals may be differentiated by tone, volume, duty cycle, pattern, duration, and/or the like. In some example implementations, the audible signal may be configured to be silenced (e.g., acknowledged or turned off) by pressing one or more buttons 224 on the sensor electronics 112 and/or by signaling the sensor electronics 112 using a button or selection on a display device (e.g., key fob, cell phone, and/or the like).

Although audio and vibratory alarms are described with respect to Fig. 2, other alarming mechanisms may be used as well. For example, in some example implementations, a tactile alarm is provided including a poking mechanism configured to "poke" or physically contact the patient in response to one or more alarm conditions.

The battery 234 may be operatively connected to the processor module 214 (and possibly other components of the sensor electronics 12) and provide the necessary power for the sensor electronics 112. In some example implementations, the battery is a Lithium Manganese Dioxide battery, however any appropriately sized and powered battery can be used (e.g., AAA, Nickel-cadmium, Zinc-carbon, Alkaline, Lithium, Nickel-metal hydride, Lithium-ion, Zinc-air, Zinc-mercury oxide, Silver-zinc, or hermetically-sealed). In some example implementations, the battery is rechargeable. In some example implementations, a plurality of batteries can be used to power the system. In yet other implementations, the receiver can be transcutaneously powered via an inductive coupling, for example.

A battery charger and/or regulator 236 may be configured to receive energy from an internal and/or external charger. In some example implementations, a battery regulator (or balancer) 236 regulates the recharging process by bleeding off excess charge current to allows all cells or batteries in the sensor electronics 112 to be fully charged without overcharging other cells or batteries. In some example implementations, the battery 234 (or batteries) is configured to be charged via an inductive and/or wireless charging pad, although any other charging and/or power mechanism may be used as well.

One or more communication ports 238, also referred to as external connector(s), may be provided to allow communication with other devices, for example a PC communication (com) port can be provided to enable communication with systems that are separate from, or integral with, the sensor electronics 112. The communication port, for example, may comprise a serial (e.g., universal serial bus or "USB") communication port, and allow for communicating with another computer system (e.g., PC, personal digital assistant or "PDA," server, or the like). In some example implementations, the sensor electronics 112 is able to transmit historical data to a PC or other computing device (e.g., an analyte processor as disclosed herein) for retrospective analysis by a patient and/or physician. As another example of data transmission, factory information may also be sent to the algorithm from the sensor or from a cloud data source.

The one or more communication ports 238 may further include a second input port 237 in which calibration data may be received, and an output port 239 which may be employed to transmit calibrated data, or data to be calibrated, to a receiver or mobile device. Fig. 2 illustrates these aspects schematically. It will be understood that the ports may be separated physically, but in alternative implementations a single communication port may provide the functions of both the second input port and the output port.

In some continuous analyte sensor systems, an on-skin portion of the sensor electronics may be simplified to minimize complexity and/or size of on-skin electronics, for example, providing only raw, calibrated, and/or filtered data to a display device configured to run calibration and other algorithms required for displaying the sensor data. However, the sensor electronics 112 (e.g., via processor module 214) may be implemented to execute prospective algorithms used to generate transformed sensor data and/or displayable sensor information, including, for example, algorithms that: evaluate a clinical acceptability of reference and/or sensor data, evaluate calibration data for best calibration based on inclusion criteria, evaluate a quality of the calibration, compare estimated analyte values with time corresponding measured analyte values, analyze a variation of estimated analyte values, evaluate a stability of the sensor and/or sensor data, detect signal artifacts (noise), replace signal artifacts, determine a rate of change and/or trend of the sensor data, perform dynamic and intelligent analyte value estimation, perform diagnostics on the sensor and/or sensor data, set modes of operation, evaluate the data for aberrancies, and/or the like.

Although separate data storage and program memories are shown in Fig. 2, a variety of configurations may be used as well. For example, one or more memories may be used to provide storage space to support data processing and storage requirements at sensor electronics 112.

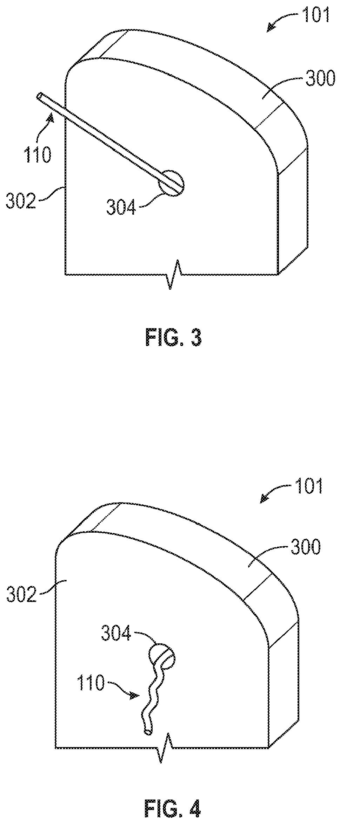

Fig. 3 illustrates an exemplary implementation of continuous analyte sensor system 101 implemented with a housing 300 from which at least a portion of continuous analyte sensor 110 extends (e.g., a portion that is arranged and positioned outside of housing 300 and extending therefrom for transcutaneous insertion). Housing 300 may be a sensor electronics housing that forms an enclosure for sensor electronics 112.

As shown in Fig. 3, at least a portion of continuous analyte sensor 110 may extend from an opening 304 in a bottom surface 302 of housing 300. The portion of continuous analyte sensor 110 that extends from housing 300 may be the portion of sensor 110 that extends beyond a plane defined by bottom surface 302 in some implementations. In various implementations, the portion of continuous analyte sensor 110 that extends from housing 300 may be a portion of a continuous analyte sensor having an additional portion disposed within housing 300 (e.g., in implementations in which an internal or proximal end of sensor 110 is coupled to sensor electronics 112 at a location within housing 300), or the portion of continuous analyte sensor 110 that extends from housing 300 may be substantially the entire continuous analyte sensor (e.g., in implementations in which a proximal end of continuous analyte sensor 110 is coupled to sensor circuitry 112 at or near bottom surface 302 of housing 300). Housing 300 may form a pod configured to be attached to the skin of a host such that bottom surface 302 is in contact with the host's skin and continuous analyte sensor 110 extends into the skin of the host. For example, bottom surface 302 may be provided with a biocompatible adhesive that attaches bottom surface 302 directly to the skin of the host or housing 300 and/or may be attached to the skin of the host by a surrounding adhesive or other attachment mechanism.

In the example of Fig. 3, continuous analyte sensor 110 is a substantially freestanding sensor that holds its shape and position without any supporting forces and regardless of the orientation thereof with respect to gravity. In the configuration of Fig. 3, the portion of continuous analyte sensor 110 that extends from housing 300 may have a Young's modulus of, for example, between 500 MPa and 147 GPa, a flexural modulus of, for example, greater than 2 GPa or 5 GPa, and/or a buckling force of, for example, greater than 0.25 Newtons (N), greater than 0.02 N, greater than 0.01 N, greater than 0.001 N, or that is greater than the weight of the portion of sensor 110 itself under gravity such that the portion of continuous analyte sensor 110 that extends from housing 300 is flexible but freestanding in various implementations.

As shown in Figs. 3 and 4, sensor 110 may be formed from an elongated body such as an elongated conductive body. In some implementations, as described in further detail hereinafter, the elongated conductive body includes an elongated conductive core. In other implementations as described in further detail hereinafter, the elongated conductive body includes an elongated non-conductive core such as a polymeric core or a fiber core. In other implementations as described in further detail hereinafter, the elongated conductive body includes a plurality of substantially planar layers.

Fig. 4 illustrates another exemplary configuration of continuous analyte sensor system 101 implemented with housing 300 from which at least a portion of continuous analyte sensor 110 extends in a non-freestanding configuration. In the configuration of Fig. 4, the shape and position of the extending portion of continuous analyte sensor 110 with respect to housing 300 may be affected by the orientation of continuous analyte sensor 110 and/or housing 300 with respect to gravity and/or other forces such as touching forces from an external object or gas or fluid forces. The configuration of continuous analyte sensor 110 shown in Fig. 4 may be described as a non-freestanding or a "wet noodle" configuration.

The configurations shown in Figs. 3 and 4 may illustrate different implementations of continuous analyte sensor 110 (e.g., a freestanding implementation and a non-freestanding implementation respectively) or the configurations shown in Figs. 3 and 4 may be different states of the same continuous analyte sensor 110. For example, the configuration of Fig. 3 may be an ex vivo configuration in which continuous analyte sensor 110 has not yet been implanted and is a freestanding sensor. Following implantation (e.g., insertion into the host's skin), continuous analyte sensor 110 may transform, in some embodiments, to non-freestanding sensor in vivo.

As described in further detail hereinafter, continuous analyte sensor 110 may include an elongated conductive body configured to be a freestanding elongated conductive body ex vivo and a non-freestanding elongated conductive body in vivo and configured to transform from the freestanding elongated conductive body to the non-freestanding elongated conductive body responsive to (i) contact between at least a portion of the elongated conductive body and tissue of a patient, (ii) a temperature change across a transition temperature (e.g., a transition temperature between seventy-eight and one-hundred degrees Fahrenheit in some implementations), (iii) absorption of a fluid (e.g., water) from patient tissue by at least a portion of the elongated conductive body, (iv) a biological and/or chemical reaction between a fluid from patient tissue and at least a portion of the elongated conductive body, and/or (v) an electromagnetic field (e.g., an electric field, a magnetic field, or a combined electric and magnetic field generated by, for example, sensor electronics for the continuous analyte sensor or external electronics).

In the configuration of Fig. 4, the non-freestanding continuous analyte sensor 110 may have a Young's modulus of, for example, less than 500 MPa, a flexural modulus of, for example, less than 2-5 GPa or less than 1.5 GPa, and/or a buckling force that that is less than 0.25 Newtons (N), less than 0.02 N, less than 0.01 N, less than 0.001 N, or that is substantially zero or is less than the weight of extending portion of sensor 110 itself under gravity (e.g., the gravitational force generated by the Earth in a range of between 0 meters and 500 km above sea level). In one or more embodiments, in the configuration of Fig. 4 (e.g., an in vivo configuration), continuous analyte sensor 110 may have a Young's modulus that is substantially the same as the Young's modulus of the tissue in which the sensor is embedded (e.g., a Young's modulus between 0.1 kPa and 300 kPa in various implementations).

In some embodiments, the sensor is configured and arranged to monitor a single analyte. However, in other embodiments, the sensor is configured and arranged to monitor a plurality of analytes, such as 2, 3, 4, 5, 6, 7, 8, 9, 10, 11, 12, or more different analytes. In yet another embodiment, the sensor is configured to monitor at least one analyte substantially continuously and to monitor at least one analyte intermittently. The analyte that is substantially, continuously monitored and the analyte that is intermittently monitored can be the same analyte or a different one.

In some embodiments, the sensor is configured and arranged for implantation in a host and for generating in vivo a signal associated with an analyte in a sample of the host during a sensor session. In some embodiments, the time length of the sensor session is from about less than 10 minutes, 10 minutes, 20 minutes, 30 minutes, 40 minutes, or 50 minutes to about 1 hour, 2 hours, 3 hours, 4 hours, 5 hours or longer. In some embodiments, the time length of the sensor session is from about 1 hour, 2 hours, 3 hours, 4 hours, 5 hours, 6 hours, 7 hours, 8 hours, 9 hours, or 10 hours to about 11 hours, 12 hours, 13 hours, 14 hours, 15 hours, 16 hours, 17 hours, 18 hours, 19 hours, 20 hours, 21 hours, 22 hours, 23 hours, 24 hours or longer. In some embodiments, the time length of the sensor session is from about less than 0.25 days, 0.25 days, 0.5 days, 0.75 days, or 1 day to about 2 days, 3 days, 4 days, 5 days, 6 days, 7 days, 8 days, 9 days, 10 days or longer.

The analyte sensor can be configured for any type of implantation, such as transcutaneous implantation, subcutaneous implantation, or implantation into the host's circulatory system (e.g., into a vessel, such as a vein or artery). In addition, the sensor may be configured to be wholly implantable or extracorporeally implantable (e.g., into an extracorporeal blood circulatory device, such as a heart-bypass machine or a blood dialysis machine). U.S. Patent Application Publication No. US-2006-0020187-A1, incorporated by reference herein to the extent the disclosure thereof does not contradict the disclosure contained in the specification, describes an exemplary continuous analyte sensor that can be used for transcutaneous implantation by insertion into the abdominal tissue of a host. U.S. Patent Application Publication No. US-2008-0119703-A1, incorporated by reference herein to the extent the disclosure thereof does not contradict the disclosure contained in the specification, describes an exemplary embodiment of a continuous analyte sensor that can be used for insertion into a host's vein (e.g., via a catheter). In some embodiments, the sensor is configured and arranged for in vitro use.

By way of example and not of limitation, a wide variety of suitable detection methods including, but not limited to, enzymatic, chemical, physical, electrochemical, immunochemical, optical, radiometric, calorimetric, protein binding, and microscale methods of detection, can be employed in certain embodiments, although any other techniques can also be used. Additional description of analyte sensor configurations and detection methods that can be used can be found in U.S. Patent Application Publication No. US-2007-0213611-A1, U.S. Patent Application Publication No. US-2007-0027385-A1, U.S. Patent Application Publication No. US-2005-0143635-A1, U.S. Patent Application Publication No. US-2007-0020641-A1, U.S. Patent Application Publication No. US-2007-002064-A11, U.S. Patent Application Publication No. US-2005-0196820-A1, U.S. Pat. No. 5,517,313, U.S. Pat. No. 5,512,246, U.S. Pat. No. 6,400,974, U.S. Pat. No. 6,711,423, U.S. Pat. No. 7,308,292, U.S. Pat. No. 7,303,875, U.S. Pat. No. 7,289,836, U.S. Pat. No. 7,289,204, U.S. Pat. No. 5,156,972, U.S. Pat. No. 6,528,318, U.S. Pat. No. 5,738,992, U.S. Pat. No. 5,631,170, U.S. Pat. No. 5,114,859, U.S. Pat. No. 7,273,633, U.S. Pat. No. 7,247,443, U.S. Pat. No. 6,007,775, U.S. Pat. No. 7,074,610, U.S. Pat. No. 6,846,654, U.S. Pat. No. 7,288,368, U.S. Pat. No. 7,291,496, U.S. Pat. No. 5,466,348, U.S. Pat. No. 7,062,385, U.S. Pat. No. 7,244,582, U.S. Pat. No. 7,211,439, U.S. Pat. No. 7,214,190, U.S. Pat. No. 7,171,312, U.S. Pat. No. 7,135,342, U.S. Pat. No. 7,041,209, U.S. Pat. No. 7,061,593, U.S. Pat. No. 6,854,317, U.S. Pat. No. 7,315,752, and U.S. Pat. No. 7,312,040 all of which are incorporated herein by reference to the extent that the disclosure thereof does not contradict the disclosure contained in the specification.

Although certain sensor configurations and methods of manufacture are described herein, it should be understood that any of a variety of known sensor configurations can be employed with the analyte sensor systems and methods of manufacture described herein, such as those described in U.S. Pat. No. 5,711,861 to Ward et al., U.S. Pat. No. 6,642,015 to Vachon et al., U.S. Pat. No. 6,654,625 to Say et al., U.S. Pat. No. 6,565,509 to Say et al., U.S. Pat. No. 6,514,718 to Heller, U.S. Pat. No. 6,465,066 to Essenpreis et al., U.S. Pat. No. 6,214,185 to Offenbacher et al., U.S. Pat. No. 5,310,469 to Cunningham et al., and U.S. Pat. No. 5,683,562 to Shaffer et al., U.S. Pat. No. 6,579,690 to Bonnecaze et al., U.S. Pat. No. 6,484,046 to Say et al., U.S. Pat. No. 6,103,033 to Say et al., U.S. Pat. No. 6,512,939 to Colvin et al., U.S. Pat. No. 6,424,847 to Mastrototaro et al., U.S. Pat. No. 6,424,847 to Mastrototaro et al., for example, all of which are incorporated herein by reference to the extent that the disclosure thereof does not contradict the disclosure contained in the specification. The sensors described in the above-identified patents documents are not inclusive of all applicable analyte sensors. It should be understood that the disclosed embodiments are applicable to a variety of analyte sensor configurations. It is noted that much of the description of the embodiments, for example the membrane system described below, can be implemented not only with in vivo sensors, but also with in vitro sensors, such as blood glucose meters (SMBG).

The sensors of certain embodiments are configured and arranged for implantation into body structures. In use, the in vivo portion of the sensor can be bent about one or more axes. This bending can occur intermittently, which can be frequently or infrequently, depending upon factors such as the nature of the implantation site (e.g., the type(s) of surrounding tissue, the thicknesses of the tissue, etc.), the type or amount of host activity, and/or the sensor's configuration.

In one embodiment, the sensor is configured and arranged for transcutaneous implantation. One exemplary transcutaneous implantation site is the abdomen, which includes an abdominal wall with a plurality of layers (e.g., skin, fascia, fat, muscles) that can move and/or slide transversely with respect of one another (e.g., in response to movement by the host). The fascia can sometimes slide, stretch or move small distances across underlying fat or muscle tissue.

In some embodiments, when a sensor is transcutaneously implanted, the in vivo portion passes through the skin and into an underlying tissue layer. Depending upon the nature of the implantation site, the sensor may pass through two or more tissue layers. Consequently, voluntary or involuntary movements by the host can move the tissue layers, which in turn can apply various forces to the implanted sensor. Similarly, when the sensor is implanted into the host's circulatory system, such as into a vein or artery, and the host moves (e.g., moves an arm, wrist and/or hand), forces may be applied to the implanted sensor.

When certain forces (e.g., forces that cause the sensor to bend in a non-preferred bending axis) are applied to a conventional sensor, they can cause damage to the sensor and/or the tissue surrounding the sensor. In contrast, the sensors of some of the embodiments are configured and arranged to bend and/or flex (e.g., simultaneously along several bend axes at several corresponding locations or continuously along the length the sensor) in response to forces applied thereto by surrounding tissue and/or body movements. Flexible analyte sensors as disclosed herein may reduce the risk of host tissue damage and sensor damage, while still maintaining sensor accuracy.

In some embodiments, the sensor is configured and arranged for a unique combination of strength and flexibility that enables the sensor to be implanted for at least one, two, three or more days and to measure at least one analyte after implantation, while withstanding intermittent and/or repeated bending and/or flexing about multiple axes often at the same time at various bend locations. In some embodiments, the sensor is configured and arranged to bend and/or flex at one, two, three or more points or continuously along its length (e.g., along a length corresponding to the in vivo portion implanted into the host). Additionally or alternatively, the sensor may be capable of bending about a plurality of axes (e.g., multi-axis bending) and/or within a plurality of planes. As is described in greater detail elsewhere herein, components of the sensor may be treated, formed and/or combined in a way to achieve the requisite combination of strength or flexibility that enables certain sensor embodiments to provide substantially accurate continuous analyte data, while withstanding an implantation environment for at least 1, 2, 3 or more days.

In certain embodiments, the in vivo portion of the sensor may have a Young's modulus that is substantially the same as the Young's modules of the tissue in which the sensor is embedded so that the sensor complies with the tissue and provides substantially no resistance to motions of the tissue.

The Young's modulus, flexural modulus, and/or buckling force of continuous analyte sensor 110 (e.g., the portion of continuous analyte sensor 110 that extends from the sensor electronics housing) may, in some embodiments, be substantially determined by the Young's modulus, flexural modulus, and/or buckling force respectively of an elongated core structure for the sensor (e.g., for that portion of the sensor). In various embodiments, an elongated core structure may be implemented as an elongated conductive core or an elongated non-conductive core such as an elongated polymer core or an elongated fiber core (e.g., a core formed from one or more para-aramid synthetic fibers such as Kevlar^{®} fibers).

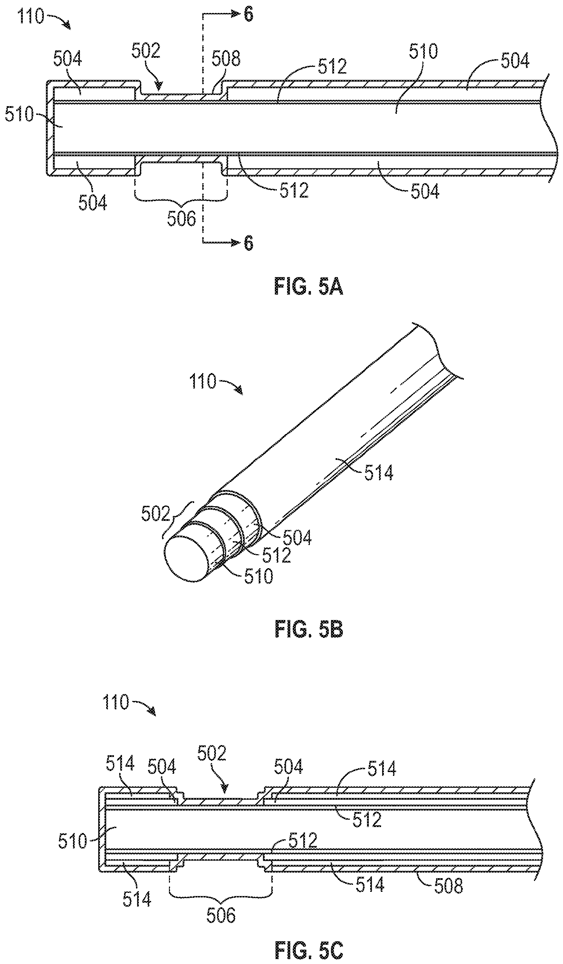

FIGS. 5A, 5B, 5C illustrate one aspect (e.g., the in vivo portion) of a flexible continuous analyte sensor 110 in implementations that include an elongated conductive core. As shown in Fig. 5A, continuous analyte sensor 110 may include an elongated conductive body 502 that includes an elongated core 510 and a first layer 512 at least partially surrounding core 510. First layer 512 may include a working electrode (e.g., located in window 506) and a membrane 508 may be disposed over the working electrode. In the examples of Figs.5A-5C, core 510 may be implemented as a flexible conductive core configured and arranged for multi-axis bending. In some embodiments, core 510 and first layer 512 can be formed from a common flexible metal or metal alloy. In some embodiments, elongated conductive body 502 may be a composite of at least two materials, such as a composite of two conductive materials, or a composite of at least one conductive material and at least one non-conductive material. In some embodiments, the elongated conductive body comprises a plurality of layers. In certain embodiments, there are at least two concentric (e.g., annular) layers, such as a core 510 formed of a first material and a first layer 512 formed of a second material. However, additional layers can be included in some embodiments. In some embodiments, the layers are coaxial.

Elongated conductive body 502 may be long and thin, yet flexible and strong. For example, in some embodiments, the smallest dimension of the elongated conductive body is less than about 0.1 inches, 0.075 inches, 0.05 inches, 0.025 inches, 0.01 inches, 0.004 inches, 0.002 inches, or 12 microns. While elongated conductive body 502 is illustrated in FIGS. 5A through 5C as having a circular cross-section, in other embodiments the cross-section of the elongated conductive body can be ovoid, elliptical, rectangular, triangular, polyhedral, star-shaped, C-shaped, T-shaped, X-shaped, Y-Shaped, irregular, or the like. In some embodiments, core 510 of the elongated conductive body 502 may be implemented as a conductive wire electrode. A conductive wire electrode core may be clad with one or more additional conducting layers (e.g., with intervening insulating layers provided for electrical isolation). The conductive layers can be comprised of any suitable material. In certain embodiments, it can be desirable to employ a conductive layer comprising conductive particles (i.e., particles of a conductive material) in a polymer or other binder.

In certain embodiments a flexible low modulus elongated core of the elongated conductive body may be provided with a surface metal cladding. The elongated core may be formed from a low modulus polymer, metal, a metal alloy or a combination thereof, having a Young's modulus less than 27 x 106 psi (186 GPa). The surface metal may include one or more metals such as platinum or and platinum alloy. The elongated core and the surface metal may be co-axially arranged in the longitudinal direction and may have a total diameter of less than 100 microns. Preferably, the low modulus core polymer, metal, or alloy is also biocompatible and provides fatigue resistance to mechanical bending and twisting.

Examples of low modulus metals and metal alloys that may be used to form a low modulus elongated core 510 include: copper, gold, Magnesium, Silver, Tin, Titanium, various Titanium alloys (e.g., a β-type titanium alloy, Ti-13Nb-13Zr, Ti-12Mo-6Zr-2Fe, Ti-15Mo, Ti-5Mo-5Zr-3Al, Ti-35Nb-7Zr-5Ta, Ti-9Nb-13Ta-4.6Zr), and/or Zinc.

In certain embodiments, the elongated core 510 (e.g., a non-conductive implementation of core 510) comprises a non-conductive material (e.g., a flexible polymer/polymeric material including polyurethane and/or polyimide and/or a shape-memory or other state-change polymer material as described herein). In some embodiments, the elongated core 510 is formed of a softenable, shape-memory thermoplastic material may include a copolymer (e.g., a block or segmented copolymer) and another polymer. The other polymer may be blended with the copolymer to act as an antiplasticizer. Suitable polymers may include polyetherurethanes, polyester-urethanes, polyether-polyesters, polyetherpolyamide, polynorbornene, high molecular weight (e.g., more and 100 kDa) poly(methyl methacrylate), poly(alkyl methacrylate) copolymers, polystyrene copolymers, filler-modified epoxy networks, chemically cross-linked amorphous polyurethanes, poly((methyl methacrylate)-co-(N-vinyl-2-pyrrolidone))-PEG semi-IPNs, HDI-HPED-TEA network, biodegradable copolyester-urethane networks, and copolymers thereof.

In addition to providing structural support, resiliency and flexibility, in some embodiments, the core 510 (or a component thereof) provides electrical conduction for an electrical signal from the working electrode to sensor electronics (not shown), which are described elsewhere herein. One skilled in the art will appreciate that additional configurations are possible.

Referring again to FIGS. 5A-5C, in some embodiments, first layer 512 is formed of a conductive material. The working electrode may be formed by an exposed portion of the surface of first layer 512. Accordingly, first layer 512 may be formed of a material configured to provide a suitable electroactive surface for the working electrode, a material such as but not limited to platinum, platinum-iridium, gold, palladium, iridium, graphite, carbon, a conductive polymer, an alloy and/or the like.

As shown in Fig. 5A-5C, a second layer 504 may surround a least a portion of first layer 512 in some embodiments, thereby defining the boundaries of the working electrode. In some embodiments, second layer 504 serves as an insulator and is formed of an insulating material, such as polyimide, polyurethane, parylene, or any other known insulating materials. For example, in one embodiment second layer 504 is disposed on the first layer and configured such that the working electrode is exposed via window 506. In another embodiment, an elongated conductive body, including the core 510, the first layer 512 and the second layer 504, is provided, and the working electrode is exposed by removing a portion of the second layer 504, thereby forming the window 506 through which the electroactive surface of the working electrode (e.g., the exposed surface of the first layer) is exposed. In some embodiments, the working electrode is exposed by (e.g., window 506 is formed by) removing a portion of the second and (optionally) third or other layers. Removal of coating materials from one or more layers of elongated conductive body (e.g., to expose the electroactive surface of the working electrode) can be performed by hand, excimer lasing, chemical etching, laser ablation, grit-blasting, or the like.

In some embodiments, the sensor further comprises a third layer 514 comprising a conductive material. In further embodiments, third layer 514 may comprise a reference electrode, which may be formed of a silver-containing material that is applied onto the second layer (e.g., an insulator). The silver-containing material may include any of a variety of materials and be in various forms, such as, Ag/AgCl-polymer pastes, paints, polymer-based conducting mixture, and/or inks that are commercially available, for example. Third layer 514 can be processed using a pasting/dipping/coating step, for example, using a die-metered dip coating process. In one exemplary embodiment, an Ag/AgCl polymer paste is applied to an elongated body by dip-coating the body (e.g., using a meniscus coating technique) and then drawing the body through a die to meter the coating to a precise thickness. In some embodiments, multiple coating steps are used to build up the coating to a predetermined thickness. Such a drawing method can be utilized for forming one or more of the electrodes in the device depicted in Fig. 5B.

In some embodiments, the silver grain in the Ag/AgCl solution or paste can have an average particle size associated with a maximum particle dimension that is less than about 100 microns, or less than about 50 microns, or less than about 30 microns, or less than about 20 microns, or less than about 10 microns, or less than about 5 microns. The silver chloride grain in the Ag/AgCl solution or paste can have an average particle size associated with a maximum particle dimension that is less than about 100 microns, or less than about 80 microns, or less than about 60 microns, or less than about 50 microns, or less than about 20 microns, or less than about 10 microns. The silver grain and the silver chloride grain may be incorporated at a ratio of the silver chloride grain:silver grain of from about 0.01:1 to 2:1 by weight, or from about 0.1:1 to 1:1. The silver grains and the silver chloride grains are then mixed with a carrier

(e.g., a polyurethane) to form a solution or paste. In certain embodiments, the Ag/AgCl component form from about 10% to about 65% by weight of the total Ag/AgCl solution or paste, or from about 20% to about 50%, or from about 23% to about 37%. In some embodiments, the Ag/AgCl solution or paste has a viscosity (under ambient conditions) that is from about 1 to about 500 centipoise, or from about 10 to about 300 centipoise, of from about 50 to about 150 centipoise.

In some embodiments, Ag/AgCl particles are mixed into a polymer, such as polyurethane, polyimide, or the like, to form the silver-containing material for the reference electrode. In some embodiments, third layer 514 is cured, for example, by using an oven or other curing process. In some embodiments, a covering of fluid-permeable polymer with conductive particles (e.g., carbon particles) therein is applied over the reference electrode and/or third layer 514. A layer of insulating material may be located over a portion of the silver-containing material, in some embodiments.

In some embodiments, elongated conductive body 502 further comprises one or more intermediate layers located between the core and the first layer. For example, in some embodiments, the intermediate layer is an insulator, a conductor, a polymer, and/or an adhesive.

It is contemplated that the ratio between the thickness of the Ag/AgCl layer and the thickness of an insulator (e.g., polyurethane or polyimide) layer can be controlled, so as to allow for a certain error margin (e.g., an error margin associated with the etching process) that would not result in a defective sensor (e.g., due to a defect resulting from an etching process that cuts into a depth more than intended, thereby unintentionally exposing an electroactive surface). This ratio may be different depending on the type of etching process used, whether it is laser ablation, grit blasting, chemical etching, or some other etching method. In one embodiment in which laser ablation is performed to remove a Ag/AgCl layer and a polyurethane layer, the ratio of the thickness of the Ag/AgCl layer and the thickness of the polyurethane layer can be from about 1:5 to about 1:1, or from about 1:3 to about 1:2.

In certain embodiments, the core for a continuous analyte sensor comprises a non-conductive polymer and the first layer comprises a conductive material. Such a sensor configuration can sometimes provide reduced material costs, additional flexibility, and/or state-change properties. For example, in some embodiments, the core is formed of a non-conductive polymer, such as, a nylon or polyester filament, string or cord, which can be coated and/or plated with a conductive material, such as platinum, platinum-iridium, gold, palladium, iridium, graphite, carbon, a conductive polymer, and allows or combinations thereof.

As shown in FIGS. 5A and 5C, sensor 110 may also include a membrane 508 covering at least a portion of the working electrode. In some embodiments, sensor 110 includes a membrane 508 in contact with the electroactive surface (e.g., at least the working electrode). As described elsewhere herein, an implanted sensor can be subjected to repeated bending and/or flexing along a plurality of axes. In order to withstand this harsh treatment and to provide analyte data over 1, 2, 3 or more days, the sensor membrane may be configured to withstand repeated compression, flexing and pulling while substantially maintaining membrane function (e.g., without ripping, buckling or tearing). Accordingly, the sensor membrane may be strong yet elastic. Shore hardness is a measure of a material's elasticity. In some embodiments, the membrane 508 comprises a polymer having a Shore hardness of at least about 65 A, 70 A, 75 A, 80 A, 85 A, 90 A, 95 A, 30 D, 35 D, 40 D, 45 D, 50 D, 55 D, 60 D, or more. In some embodiments, the polymer has a Shore hardness of from about 70 A to about 55 D. Polymers having a Shore hardness within the range of from about 70 A to about 55 D include but are not limited to polyurethanes, polyimides, silicones, and the like, such as described elsewhere herein.

In some embodiments, the entire membrane is formed of one or more polymers having a Shore hardness of from about 70 A to about 55 D. However, in other embodiments, only a portion of the membrane, such as a membrane layer or domain, is formed of one of these polymers. In one exemplary embodiment, an outer layer of the membrane is formed of a polymer having a Shore hardness of from about 70 A to about 55 D. In some embodiments, approximately the outer 5%, 10%, 15%, 20%, 25%, 30%, 35%, 35%, 40%, 45%, or 50% of the membrane is formed of a polymer having a Shore hardness of from about 70 A to about 55 D. In some embodiments, the resistance domain is formed from a polymer having a Shore hardness of from about 70 A to about 55 D. Additional membrane domains are also formed of a polymer having a Shore hardness within this range, in other embodiments. In one embodiment, at least a portion of the membrane is formed of a polymer having a Shore hardness of from about 70 A to about 55 D and an enzyme is disposed in the polymer.

As discussed herein, membranes can be formed by any suitable method. It can be desirable to form membranes on the various exposed electrodes of the sensors of some embodiments by dipping the sensor at different lengths, by masking, controlled UV curing, printing, and/or similar methods.

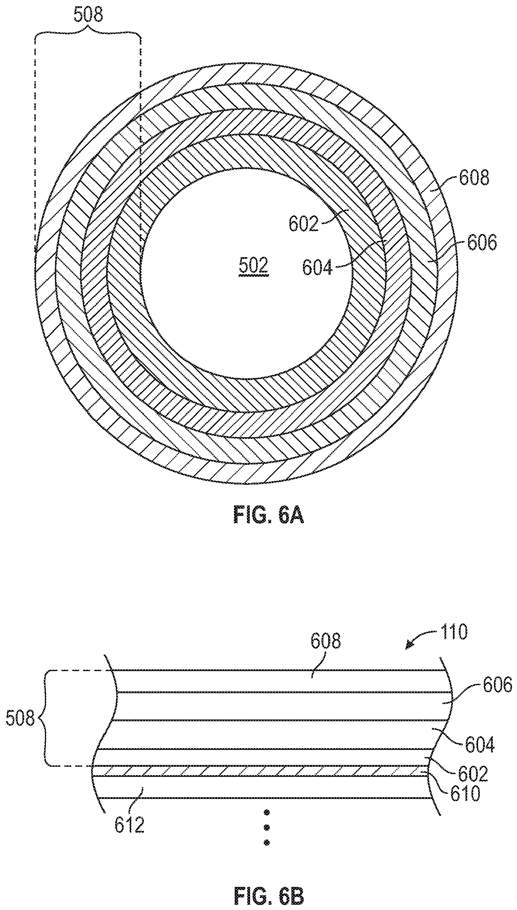

Fig. 6A is a cross section of the sensor shown in Fig. 5A, taken at line 6-6. As shown in Fig. 6A, a membrane 508 may deposited over electroactive surfaces of the sensor and may include a plurality of domains or layers, such as described in more detail below. In some embodiments, the membrane comprises a single layer. In some embodiments, the single layer includes one or more functional domains (e.g., portions or areas). In other embodiments, the membrane comprises two or more layers. In some embodiments, each of the layers performs a different function. In some embodiments, multiple layers can perform the same function. Membrane 508 can be deposited on the exposed electroactive surfaces using known thin film techniques (for example, spraying, electro-depositing, dipping, and the like). In one exemplary embodiment, each domain is deposited by dipping the sensor into a solution and drawing out the sensor at a speed that provides the appropriate domain thickness. In another exemplary embodiment, each domain is deposited by spraying the solution onto the sensor for a period of time that provides the appropriate domain thickness. In general, the membrane system can be disposed over (deposited on) the electroactive surfaces using methods appreciated by one skilled in the art.

In general, the membrane system includes a plurality of domains, for example, an electrode domain 602, an interference domain 604, an enzyme domain 606 (e.g., including glucose oxidase), and/or a resistance domain 608, as shown in Fig. 6A, and can include a high oxygen solubility domain, and/or a bioprotective domain (not shown), such as is described in more detail in U.S. Patent Application Publication No. US-2005-0245799-A1, and such as is described in more detail below. The membrane system can be deposited on the exposed electroactive surfaces using known thin film techniques (for example, vapor deposition, spraying, electro-depositing, dipping, and the like). In alternative embodiments, however, other vapor deposition processes (e.g., physical and/or chemical vapor deposition processes) can be useful for providing one or more of the insulating and/or membrane layers, including ultrasonic vapor deposition, electrostatic deposition, evaporative deposition, deposition by sputtering, pulsed laser deposition, high velocity oxygen fuel deposition, thermal evaporator deposition, electron beam evaporator deposition, deposition by reactive sputtering molecular beam epitaxy, atmospheric pressure chemical vapor deposition (CVD), atomic layer CVD, hot wire CVD, low-pressure CVD, microwave plasma-assisted CVD, plasma-enhanced CVD, rapid thermal CVD, remote plasma-enhanced CVD, and ultra-high vacuum CVD, for example. However, the membrane system can be disposed over (or deposited on) the electroactive surfaces using any known method, as will be appreciated by one skilled in the art. When enzymes are employed, e.g., in certain dual working electrode glucose sensor configurations, for ease of fabrication each of the electrodes can be dipped with an enzyme domain including enzyme. Enzyme over one of the working electrodes (e.g., the second working electrode) can then be denatured/killed, e.g., by exposure to UV or other irradiation, or by exposure to other agents or treatment methods as known in the art for denaturing enzyme. In some embodiments, one or more domains of the membrane systems are formed from materials such as silicone, polytetrafluoroethylene, polyethylene-co-tetrafluoroethylene, polyolefin, polyester, polycarbonate, biostable polytetrafluoroethylene, homopolymers, copolymers, terpolymers of polyurethanes, polypropylene (PP), polyvinylchloride (PVC), polyvinylidene fluoride (PVDF), polybutylene terephthalate (PBT), polymethylmethacrylate (PMMA), polyether ether ketone (PEEK), polyurethanes, cellulosic polymers, polysulfones and block copolymers thereof including, for example, di-block, tri-block, alternating, random and graft copolymers. U.S. Patent Application Publication No. US-2005-0245799-A1 describes biointerface and membrane system configurations and materials that may be applied.

In selected embodiments, the membrane system comprises an electrode domain 602. The electrode domain 602 is provided to ensure that an electrochemical reaction occurs between the electroactive surfaces of the working electrode and the reference electrode, and thus the electrode domain may be situated more proximal to the electroactive surfaces than the interference domain 604 and/or enzyme domain 606. The electrode domain 602 may include a coating that maintains a layer of water at the electrochemically reactive surfaces of the sensor. In other words, the electrode domain may provide an environment between the surfaces of the working electrode and the reference electrode, which facilitates an electrochemical reaction between the electrodes. For example, a humectant in a binder material can be employed as an electrode domain; this allows for the full transport of ions in the aqueous environment. The electrode domain can also assist in stabilizing the operation of the sensor by accelerating electrode start-up and drifting problems caused by inadequate electrolyte. The material that forms the electrode domain can also provide an environment that protects against pH-mediated damage that can result from the formation of a large pH gradient due to the electrochemical activity of the electrodes.

In one embodiment, the electrode domain includes hydrophilic polymer film (e.g., a flexible, water-swellable, hydrogel) having a "dry film" thickness of from about 0.05 microns or less to about 20 microns or more, or from about 0.05, 0.1, 0.15, 0.2, 0.25, 0.3, 0.35, 0.4, 0.45, 0.5, 1, 1.5, 2, 2.5, 3, or 3.5 microns to about 4, 5, 6, 7, 8, 9, 10, 11, 12, 13, 14, 15, 16, 17, 18, 19, or 19.5 microns, or from about 3, 2.5, 2, or 1 microns, or less, to about 3.5, 4, 4.5, or 5 microns or more. "Dry film" thickness refers to the thickness of a cured film cast from a coating formulation by standard coating techniques.

In certain embodiments, electrode domain 602 is formed of a curable mixture of a urethane polymer and a hydrophilic polymer. In some embodiments, the coatings are formed of a polyurethane polymer having carboxylate or hydroxyl functional groups and nonionic hydrophilic polyether segments, wherein the polyurethane polymer is crosslinked with a water-soluble carbodiimide (e.g., 1-ethyl-3-(3-dimethylaminopropyl)carbodiimide (EDC)) in the presence of polyvinylpyrrolidone and cured at a moderate temperature of about 50° C.

In some embodiments, electrode domain 602 is formed from a hydrophilic polymer (e.g., a polyamide, a polylactone, a polyimide, a polylactam, a functionalized polyamide, a functionalized polylactone, a functionalized polyimide, a functionalized polylactam or a combination thereof) that renders the electrode domain substantially more hydrophilic than an overlying domain, (e.g., interference domain, enzyme domain). In some embodiments, electrode domain 602 is formed substantially entirely and/or primarily from a hydrophilic polymer. In some embodiments, the electrode domain is formed substantially entirely from PVP. In some embodiments, electrode domain 602 is formed entirely from a hydrophilic polymer. Useful hydrophilic polymers include but are not limited to poly-N-vinylpyrrolidone (PVP), poly-N-vinyl-2-piperidone, poly-N-vinyl-2-caprolactam, poly-N-vinyl-3-methyl-2-caprolactam, poly-N-vinyl-3-methyl-2-piperidone, poly-N-vinyl-4-methyl-2-piperidone, poly-N-vinyl-4-methyl-2-caprolactam, poly-N-vinyl-3-ethyl-2-pyrrolidone, poly-N-vinyl-4,5-dimethyl-2-pyrrolidone, polyvinylimidazole, poly-N,N-dimethylacrylamide, polyvinyl alcohol, polyacrylic acid, polyethylene oxide, poly-2-ethyl-oxazoline, copolymers thereof and mixtures thereof. A blend of two or more hydrophilic polymers may be used in some embodiments. In some embodiments, the hydrophilic polymer(s) is not crosslinked. In alternative embodiments, crosslinking may be performed, such as by adding a crosslinking agent, such as but not limited to EDC, or by irradiation at a wavelength sufficient to promote crosslinking between the hydrophilic polymer molecules, which is believed to create a more tortuous diffusion path through the domain.

An electrode domain formed from a hydrophilic polymer (e.g., PVP) has been shown to substantially reduce break-in time of analyte sensors; for example, a glucose sensor utilizing a cellulosic-based interference domain such as described in more detail elsewhere herein. In some embodiments, a uni-component electrode domain formed from a single hydrophilic polymer (e.g., PVP) has been shown to substantially reduce break-in time of a glucose sensor to less than about 2 hours, less than about 1 hour, less than about 20 minutes and/or substantially immediately.

Generally, sensor break-in is the amount of time required (after implantation) for the sensor signal to become substantially representative of the analyte concentration. Sensor break-in includes both membrane break-in and electrochemical break-in, which are described in more detail elsewhere herein. In some embodiments, break-in time is less than about 2 hours. In other embodiments, break-in time is less than about 1 hour. In still other embodiments, break-in time is less than about 30 minutes, less than about 20 minutes, less than about 15 minutes, less than about 10 minutes, or less. In one embodiment, sensor break-in occurs substantially immediately. Advantageously, in embodiments wherein the break-in time is about 0 minutes (substantially immediately), the sensor can be inserted and begin providing substantially accurate analyte (e.g., glucose) concentrations almost immediately post-insertion, for example, wherein membrane break-in does not limit start-up time.

In some embodiments, providing a flexible sensor (e.g., a sensor having a flexural modulus of less than 2-5 GPa or a Young's modulus of less than 147 GPa or that substantially matches the modulus of one or more layers of tissue in which the sensor is embedded), may reduce the break-in time and/or may prevent first-day effects that follow the break-in time such as erroneous measurements caused by tissue reaction (e.g., inflammation) to the newly implanted sensor.

Moreover, providing an electrode domain that is substantially more hydrophilic than the next more distal membrane layer or domain (e.g., the overlaying domain, the layer more distal to the electroactive surface than the electrode domain, such as an interference domain or an enzyme domain) reduces the break-in time of an implanted sensor, by increasing the rate at which the membrane system is hydrated by the surrounding host tissue. Increasing the amount of hydrophilicity of the electrode domain relative to the overlaying layer (e.g., the distal layer in contact with electrode domain, such as the interference domain, enzyme domain, etc.), may increase the rate of water absorption, resulting in reduced sensor break-in time. The hydrophilicity of the electrode domain can be substantially increased by the proper selection of hydrophilic polymers, based on their hydrophilicity relative to each other and relative to the overlaying layer (e.g., cellulosic-based interference domain), with certain polymers being substantially more hydrophilic than the overlaying layer. In one exemplary embodiment, PVP forms the electrode domain, the interference domain is formed from a blend of cellulosic derivatives, such as but not limited to cellulose acetate butyrate and cellulose acetate; it is believed that since PVP is substantially more hydrophilic than the cellulosic-based interference domain, the PVP rapidly draws water into the membrane to the electrode domain, and enables the sensor to function with a desired sensitivity and accuracy and starting within a substantially reduced time period after implantation. Reductions in sensor break-in time reduce the amount of time a host has to wait to obtain sensor readings, which is particularly advantageous not only in ambulatory applications, but particularly in hospital settings where time is critical.

When the water absorption of the overlying domain (e.g., the domain overlying the electrode domain) is less than the water absorption of the electrode domain (e.g., during membrane equilibration), then the difference in water absorption between the two domains may drive membrane equilibration and thus membrane break-in. Namely, increasing the difference in hydrophilicity (e.g., between the two domains) may result in an increase in the rate of water absorption, which, in turn, may result in a decrease in membrane break-in time and/or sensor break-in time. As discussed elsewhere herein, the relative hydrophilicity of the electrode domain as compared to the overlying domain can be modulated by a selection of more hydrophilic materials for formation of the electrode domain (and/or more hydrophobic materials for the overlying domain(s)). For example, an electrode domain with hydrophilic polymer capable of absorbing larger amounts of water can be selected instead of a second hydrophilic polymer that is capable of absorbing less water than the first hydrophilic polymer. In some embodiments, the water content difference between the electrode domain and the overlying domain (e.g., during or after membrane equilibration) is from about 1% or less to about 90% or more. In other embodiments, the water content difference between the electrode domain and the overlying domain is from about 10% or less to about 80% or more. In still other embodiments, the water content difference between the electrode domain and the overlying domain is from about 30% or less to about 60% or more. In some embodiments, the electrode domain absorbs 5 wt. % or less to 95 wt. % or more water, or from about 5, 10, 15, 20, 25, 30, 35, 40, 45, or 50 wt. % to about 55, 60, 65, 70, 75, 80, 85, 90 or 95 wt. % water than the adjacent (overlying) domain (e.g., the domain that is more distal to the electroactive surface than the electrode domain).

In another example, the rate of water absorption by a polymer can be affected by other factors, such as but not limited to the polymer's molecular weight. For example, the rate of water absorption by PVP is dependent upon its molecular weight, which is typically from about 40 kDa or less to about 360 kDa or more; with a lower molecular weight PVP (e.g., 40 kDa) absorbing water faster than a higher molecular weight PVP. Accordingly, modulating factors, such as molecular weight, that affect the rate of water absorption by a polymer, can promote the proper selection of materials for electrode domain fabrication. In one embodiment, a lower molecular weight PVP is selected, to reduce break-in time.

Electrode domain 602 may be deposited by known thin film deposition techniques (e.g., spray coating or dip-coating the electroactive surfaces of the sensor). In some embodiments, the electrode domain is formed by dip-coating the electroactive surfaces in an electrode domain solution (e.g., 5, 10, 15, 20, 25 or 30% or more PVP in deionized water) and curing the domain for a time of from about 15 minutes to about 30 minutes at a temperature of from about 40° C. to about 55° C. (and can be accomplished under vacuum (e.g., 20 to 30 mmHg)). In embodiments wherein dip-coating is used to deposit the electrode domain, an insertion rate of from about 1 to about 3 inches per minute into the electrode domain solution may be used, with a dwell time of from about 0.5 to about 2 minutes in the electrode domain solution, and a withdrawal rate of from about 0.25 to about 2 inches per minute from the electrode domain solution provide a functional coating. However, values outside of those set forth above can be acceptable or even desirable in certain embodiments, for example, depending upon solution viscosity and solution surface tension, as is appreciated by one skilled in the art. In one embodiment, the electroactive surfaces of the electrode system are dip-coated one time (one layer) and cured at 50° C. under vacuum for 20 minutes. In another embodiment, the electroactive surfaces of the electrode system is dip-coated and cured at 50° C under vacuum for 20 minutes a first time, followed by dip coating and curing at 50° C under vacuum for 20 minutes a second time (two layers). In still other embodiments, the electroactive surfaces can be dip-coated three or more times (three or more layers). In other embodiments, the 1, 2, 3 or more layers of PVP are applied to the electroactive surfaces by spray coating or vapor deposition. In some embodiments, a crosslinking agent (e.g., EDC) can be added to the electrode domain casting solution to promote crosslinking within the domain (e.g., between electrode domain polymer components, latex, etc.). In some alternative embodiments however, no crosslinking agent is used and the electrode domain is not substantially crosslinked.

In some embodiments, the deposited PVP electrode domain has a "dry film" thickness of from about 0.05 microns or less to about 20 microns, or from about 0.05, 0.1, 0.15, 0.2, 0.25, 0.3, 0.35, 0.4, 0.45, 0.5, 1, 1.5, 2, 2.5, 3, or 3.5 microns to about 4, 5, 6, 7, 8, 9, 10, 11, 12, 13, 14, 15, 16, 17, 18, 19, or 19.5 microns, or from about 2, 2.5 or 3 microns to about 3.5, 4, 4.5, or 5 microns. Although an independent electrode domain is described herein, in some embodiments sufficient hydrophilicity can be provided in the interference domain and/or enzyme domain (the domain adjacent to the electroactive surfaces) so as to provide for the full transport of ions in the aqueous environment (e.g. without a distinct electrode domain). In these embodiments, an electrode domain is not necessary.

An elongated core (e.g., an elongated conductive core or an elongated non-conductive core) may have a Young's or flexural modulus (e.g., at least an ex vivo Young's or flexural modulus) that is sufficiently large (e.g., greater than 500 MPa) that the core holds its shape during dipping operations (e.g., the core is not bent due to a force from the surface of the dipping fluid as the core is dipped into the fluid). In some embodiments, the Young's or flexural modulus may be increased (e.g., by application of an electromagnetic field or by cooling to a threshold temperature) during dipping operations. In other embodiments, the Young's or flexural modulus may decrease in vivo (e.g., due to a rise in temperature, due to wetting or hydration of one or more layers or other structures, due to an applied or removed electromagnetic field, or due to a chemical or biological reaction) as described in further detail hereinafter.

An elongated core (e.g., an elongated conductive core or an elongated non-conductive core for a portion of a continuous analyte sensor that is configured to extend from a sensor electronics housing) may have a buckling force (e.g., at least an ex vivo buckling force) that is sufficiently large (e.g., greater than 0.25 N, greater than 0.02 N, greater than 0.01 N, greater than 0.001 N, or greater than the weight of the core under gravity) that the core holds its shape during dipping operations (e.g., the core is not bent due to a force from the surface of the dipping fluid as the core is dipped into the fluid). In some embodiments, the buckling force may be increased (e.g., by application of an electromagnetic field or by cooling to a threshold temperature) during dipping operations. In other embodiments, the buckling force may decrease in vivo (e.g., due to a rise in temperature, due to wetting or hydration of one or more layers or other structures, due to an applied or removed electromagnetic field, or due to a chemical or biological reaction) as described in further detail hereinafter.

Interferents are molecules or other species that are reduced or oxidized at the electrochemically reactive surfaces of the sensor, either directly or via an electron transfer agent, to produce a false positive analyte signal (e.g., a non-analyte-related signal). This false positive signal causes the host's analyte concentration (e.g., glucose concentration) to appear higher than the true analyte concentration. False-positive signal is a clinically significant problem in some conventional sensors. For example in a case of a dangerously hypoglycemic situation, wherein the host has ingested an interferent (e.g., acetaminophen), the artificially high glucose signal can lead the host to believe that he is euglycemic (or, in some cases, hyperglycemic). As a result, the host can make inappropriate treatment decisions, such as taking no action, when the proper course of action is to begin eating. In another example, in the case of a euglycemic or hyperglycemic situation, wherein a host has consumed acetaminophen, an artificially high glucose signal caused by the acetaminophen can lead the host to believe that his glucose concentration is much higher than it truly is. Again, as a result of the artificially high glucose signal, the host can make inappropriate treatment decisions, such as giving himself too much insulin, which in turn can lead to a dangerous hypoglycemic episode.

In some embodiments, an interference domain 604 is provided that substantially restricts or blocks the flow of one or more interfering species therethrough; thereby substantially preventing artificial signal increases. Some known interfering species for a glucose sensor, as described in more detail herein, include acetaminophen, ascorbic acid, bilirubin, cholesterol, creatinine, dopamine, ephedrine, ibuprofen, L-dopa, methyl dopa, salicylate, tetracycline, tolazamide, tolbutamide, triglycerides, and uric acid. In general, the interference domain of some embodiments is less permeable to one or more of the interfering species than to the measured species, e.g., the product of an enzymatic reaction that is measured at the electroactive surface(s), such as but not limited to H₂O₂.

In one embodiment, the interference domain is formed from one or more cellulosic derivatives. Cellulosic derivatives can include, but are not limited to, cellulose esters and cellulose ethers. In general, cellulosic derivatives include polymers such as cellulose acetate, cellulose acetate butyrate, 2-hydroxyethyl cellulose, cellulose acetate phthalate, cellulose acetate propionate, cellulose acetate trimellitate, and the like, as well as their copolymers and terpolymers with other cellulosic or non-cellulosic monomers. Cellulose is a polysaccharide polymer of β-D-glucose. While cellulosic derivatives may be used in some embodiments, other polymeric polysaccharides having similar properties to cellulosic derivatives can also be employed in other embodiments. Descriptions of cellulosic interference domains can be found in U.S. Patent Application Publication No. US-2006-0229512-A1, U.S. Patent Application Publication No. US-2007-0173709-A1, U.S. Patent Application Publication No. US-2006-0253012-A1, and U.S. Patent Application Publication No. US-2007-0213611-A1, all of which are incorporated herein by reference to the extent that the disclosure thereof does not contradict the disclosure contained in the specification.

In some embodiments, the interferent's equivalent glucose signal response (measured by the sensor) is 50 mg/dL or less. In certain embodiments, the interferent produces an equivalent glucose signal response of 40 mg/dL or less. In some embodiments, the interferent produces an equivalent glucose signal response of less than about 30, 20 or 10 mg/dL. In one exemplary embodiment, the interference domain is configured to substantially block acetaminophen passage therethrough, wherein the equivalent glucose signal response of the acetaminophen is less than about 30 mg/dL.

In alternative embodiments, the interference domain is configured to substantially block a therapeutic dose of acetaminophen. The term "therapeutic dose" as used herein is a broad term, and is to be given its ordinary and customary meaning to a person of ordinary skill in the art (and is not to be limited to a special or customized meaning), and refers without limitation to the quantity of any substance required to effect the cure of a disease, to relieve pain, or that will correct the manifestations of a deficiency of a particular factor in the diet, such as the effective dose used with therapeutically applied compounds, such as drugs. For example, a therapeutic dose of acetaminophen can be an amount of acetaminophen required to relieve headache pain or reduce a fever. As a further example, 1,000 mg of acetaminophen taken orally, such as by swallowing two 500 mg tablets of acetaminophen, is the therapeutic dose frequently taken for headaches. In some embodiments, the interference membrane is configured to block a therapeutic dose of acetaminophen, wherein the equivalent glucose signal response of the acetaminophen is less than about 60 mg/dL. In one embodiment, the interference membrane is configured to block a therapeutic dose of acetaminophen, wherein the equivalent glucose signal response of the acetaminophen is less than about 40 mg/dL. In another embodiment, the interference membrane is configured to block a therapeutic dose of acetaminophen, wherein the equivalent glucose signal response of the acetaminophen is less than about 30 mg/dL.

In some alternative embodiments, additional polymers, such as NATION^{®}, can be used in combination with cellulosic derivatives to provide equivalent and/or enhanced function of the interference domain. As one example, a layer of a 5 wt. % NAFION^{®} casting solution was applied over a previously applied (e.g., and cured) layer of 8 wt. % cellulose acetate, e.g., by dip coating at least one layer of cellulose acetate and subsequently dip coating at least one layer NAFION^{®} onto a needle-type sensor. Any number of coatings or layers formed in any order may be suitable for forming the interference domain.

In some alternative embodiments, other polymer types that can be utilized as a base material for the interference domain include polyurethanes, polymers having pendant ionic groups, and polymers having controlled pore size, for example. In one such alternative embodiment, the interference domain includes a thin, hydrophobic membrane that is non-swellable and restricts diffusion of high molecular weight species. The interference domain is permeable to relatively low molecular weight substances, such as hydrogen peroxide, but restricts the passage of higher molecular weight substances, including glucose and ascorbic acid. Other systems and methods for reducing or eliminating interference species that can be applied to the membrane system are described in U.S. Pat. No. 7,074,307, U.S. Patent Application Publication No. US-2005-0176136-A1, U.S. Pat. No. 7,081,195, and U.S. Patent Application Publication No. US-2005-0143635-A1, all of which are incorporated herein by reference to the extent that the disclosure thereof does not contradict the disclosure contained in the specification. In some alternative embodiments, a distinct interference domain is not included.

In some embodiments, the interference domain is deposited either directly onto the electroactive surfaces of the sensor or onto the distal surface of the electrode domain, for a domain thickness of from about 0.05 microns to about 20 microns, or from about 0.05, 0.1, 0.15, 0.2, 0.25, 0.3, 0.35, 0.4, 0.45, 0.5, 1, 1.5, 2, 2.5, 3, or 3.5 microns to about 4, 5, 6, 7, 8, 9, 10, 11, 12, 13, 14, 15, 16, 17, 18, 19, or 19.5 microns, or from about 1, 1.5 or 2 microns to about 2.5 or 3 microns. Thicker membranes can also be desirable in certain embodiments, but thinner membranes may be used because they have a lower impact on the rate of diffusion of hydrogen peroxide from the enzyme membrane to the electroactive surface(s).

In general, the membrane systems of some embodiments can be formed and/or deposited on the exposed electroactive surfaces (e.g., one or more of the working and reference electrodes) using known thin film techniques (for example, casting, spray coating, drawing down, electro-depositing, dip coating, and the like), however casting, printing, or other known application techniques can also be utilized. Interference domain 604 may be deposited by spray or dip coating (as examples). In one exemplary embodiment of a needle-type (transcutaneous) sensor such as described herein, the interference domain 604 is formed by dip coating the sensor into an interference domain solution using an insertion rate of from about 0.5 inch/min to about 60 inches/min, or about 1 inch/min, a dwell time of from about 0 minute to about 2 minutes, or about 1 minute, and a withdrawal rate of from about 0.5 inch/minute to about 60 inches/minute, or about 1 inch/minute, and curing (drying) the domain from about 1 minute to about 30 minutes, or from about 3 minutes to about 15 minutes (and can be accomplished at room temperature or under vacuum (e.g., 20 to 30 mmHg)). In one exemplary embodiment including cellulose acetate butyrate interference domain, a 3-minute cure (i.e., dry) time is sometimes between each layer applied. In another exemplary embodiment employing a cellulose acetate interference domain, a 15 minute cure (i.e., dry) time is used between each layer applied.

In some embodiments, the dip process can be repeated at least one time and up to 10 times or more. In other embodiments, only one dip is performed. The number of repeated dip processes used depends upon the cellulosic derivative(s) used, their concentration, conditions during deposition (e.g., dipping) and the desired thickness (e.g., sufficient thickness to provide functional blocking of certain interferents), and the like. In some embodiments, 1 to 3 microns may be used for the interference domain thickness; however, values outside of these can be acceptable or even desirable in certain embodiments, for example, depending upon viscosity and surface tension, as is appreciated by one skilled in the art. In one exemplary embodiment, an interference domain is formed from three layers of cellulose acetate butyrate. In another exemplary embodiment, an interference domain is formed from ten layers of cellulose acetate. In another embodiment, an interference domain is formed from one layer of a blend of cellulose acetate and cellulose acetate butyrate. In alternative embodiments, the interference domain can be formed using any known method and combination of cellulose acetate and cellulose acetate butyrate, as will be appreciated by one skilled in the art. In some embodiments, the electroactive surface can be cleaned, smoothed or otherwise treated prior to application of the interference domain. In some embodiments, the interference domain of some embodiments can be useful as a bioprotective or biocompatible domain, namely, a domain that interfaces with host tissue when implanted in an animal (e.g., a human) due to its stability and biocompatibility. In still other embodiments, other portions of the membrane system, such as but not limited to the enzyme domain and/or the resistance domain can be configured for interference blocking. In still other embodiments, an interference domain can be located either more distally or proximally to the electroactive surface than other membrane domains. For example, an interference domain can be located more distal to the electroactive surface than an enzyme domain or a resistance domain, in some embodiments.

In certain embodiments, the membrane system further includes an enzyme domain 606 disposed more distally from the electroactive surfaces than the interference domain, however other configurations can be desirable. In certain embodiments, enzyme domain 606 provides an enzyme to catalyze the reaction of the analyte and its co-reactant, as described in more detail below. In the some embodiments of a glucose sensor, enzyme domain 606 includes glucose oxidase (GOX); however other oxidases, for example, galactose oxidase or uricase oxidase, can also be used. In some embodiments, the enzyme domain is configured and arranged for detection of at least one of albumin, alkaline phosphatase, alanine transaminase, aspartate aminotransferase, bilirubin, blood urea nitrogen, calcium, CO₂, chloride, creatinine, glucose, gamma-glutamyl transpeptidase, hematocrit, lactate, lactate dehydrogenase, magnesium, oxygen, pH, phosphorus, potassium, sodium, total protein, uric acid, a metabolic marker, a drug, various minerals, various metabolites, and/or the like. In a further embodiment, the sensor is configured and arranged to detect two or more of albumin, alkaline phosphatase, alanine transaminase, aspartate aminotransferase, bilirubin, blood urea nitrogen, calcium, CO₂, chloride, creatinine, glucose, gamma-glutamyl transpeptidase, hematocrit, lactate, lactate dehydrogenase, magnesium, oxygen, pH, phosphorus, potassium, sodium, total protein, uric acid, a metabolic marker, a drug, various minerals, various metabolites, and/or the like.

For an enzyme-based electrochemical glucose sensor to perform well, the sensor's response may be limited by neither enzyme activity nor co-reactant concentration. Because enzymes, including glucose oxidase, are subject to deactivation as a function of time even in ambient conditions, this behavior is compensated for in forming the enzyme domain. In some embodiments, the enzyme domain is constructed of aqueous dispersions of colloidal polyurethane polymers including the enzyme. However, in alternative embodiments the enzyme domain is constructed from an oxygen enhancing material, for example, silicone, or fluorocarbon, in order to provide a supply of excess oxygen during transient ischemia. The enzyme may be immobilized within the domain. See, e.g., U.S. Patent Application Publication No. US-2005-0054909-A1 which is incorporated herein by reference to the extent that the disclosure thereof does not contradict the disclosure contained in the specification. In some embodiments, enzyme domain 606 may be deposited onto interference domain 604 for a domain thickness of from about 0.05 micron to about 20 microns, or from about 0.05, 0.1, 0.15, 0.2, 0.25, 0.3, 0.35, 0.4, 0.45, 0.5, 1, 1.5, 2, 2.5, 3, or 3.5 microns to about 4, 5, 6, 7, 8, 9, 10, 11, 12, 13, 14, 15, 16, 17, 18, 19, or 19.5 microns, or from about 2, 2.5 or 3 microns to about 3.5, 4, 4.5, or 5 microns. However in some embodiments, enzyme domain 606 can be deposited directly onto the electroactive surfaces. The enzyme domain may be deposited by spray or dip coating. In one embodiment of needle-type (transcutaneous) sensor such as described herein, the enzyme domain is formed by dip coating the interference domain coated sensor into an enzyme domain solution and curing the domain for from about 15 to about 30 minutes at a temperature of from about 40° C to about 55° C (and can be accomplished under vacuum (e.g., 20 mmHg to 30 mmHg)). In embodiments wherein dip coating is used to deposit the enzyme domain at room temperature to provide a functional coating, the insertion rate used may be from about 0.25 inch per minute to about 3 inches per minute, with a dwell time of from about 0.5 minutes to about 2 minutes, and a withdrawal rate of from about 0.25 inch per minute to about 2 inches per minute. However, values outside of those set forth above can be acceptable or even desirable in certain embodiments, for example, depending upon viscosity and surface tension, as is appreciated by one skilled in the art. In one embodiment, the enzyme domain is formed by dip coating two times (namely, forming two layers) in an enzyme domain solution and curing at 50° C under vacuum for 20 minutes. However, in some embodiments, the enzyme domain can be formed by dip coating and/or spray coating one or more layers at a predetermined concentration of the coating solution, insertion rate, dwell time, withdrawal rate, and/or desired thickness.

Enzymatic analyte sensors are dependent upon the kinetics of the enzymes that they comprise. As is understood by one skilled in the art, in order to calculate the concentration of one reactant/analyte (e.g., using a defined amount of enzyme) all other reactants/co-reactants are present in excess. However, in the body, this is often not the case; sometimes the analyte is in excess. Accordingly, in some embodiments, the sensor membrane is configured and arranged to restrict diffusion of the analyte to the enzyme domain, such that the analyte can be measured accurately. In some embodiments, the membrane system includes a resistance domain 608 disposed more distal from the electroactive surfaces than the enzyme domain. Although the following description is directed to a resistance domain for a glucose sensor, the resistance domain can be modified for other analytes and co-reactants as well.

With respect to glucose sensors, there exists a molar excess of glucose relative to the amount of oxygen in blood; that is, for every free oxygen molecule in extracellular fluid, there are typically more than 100 glucose molecules present (see Updike et al., Diabetes Care 5 :207-21 (1982), incorporated herein by reference to the extent that the disclosure thereof does not contradict the disclosure contained in the specification). However, an immobilized enzyme-based glucose sensor employing oxygen as co-reactant may be supplied with oxygen in non-rate-limiting excess in order for the sensor to respond linearly to changes in glucose concentration, while not responding to changes in oxygen concentration. Specifically, when a glucose-monitoring reaction is oxygen limited, linearity is not achieved above minimal concentrations of glucose. Without a semipermeable membrane situated over the enzyme domain to control the flux of glucose and oxygen, a linear response to glucose levels can be obtained only for glucose concentrations of up to about 40 mg/dL. However, in a clinical setting, a linear response to glucose levels is desirable up to at least about 400 mg/dL.

Resistance domain 608 may include a semipermeable membrane that controls the flux of oxygen and glucose to the underlying enzyme domain, thereby rendering oxygen in a non-rate-limiting excess. As a result, the upper limit of linearity of glucose measurement is extended to a much higher value than that which is achieved without the resistance domain. In one embodiment, resistance domain 608 exhibits an oxygen to glucose permeability ratio of from about 50:1 or less to about 400:1 or more, or about 200:1. As a result, one-dimensional reactant diffusion is adequate to provide excess oxygen at all reasonable glucose and oxygen concentrations found in the subcutaneous matrix (See Rhodes et al., Anal. Chem., 66: 1520-1529 (1994), incorporated herein by reference to the extent that the disclosure thereof does not contradict the disclosure contained in the specification).

In alternative embodiments, a lower ratio of oxygen-to-glucose can be sufficient to provide excess oxygen by using a high oxygen solubility domain (for example, a silicone or fluorocarbon-based material or domain) to enhance the supply/transport of oxygen to the enzyme domain. If more oxygen is supplied to the enzyme, then more glucose can also be supplied to the enzyme without creating an oxygen rate-limiting excess. In alternative embodiments, the resistance domain is formed from a silicone composition, such as is described in U.S. Patent Application Publication No. US-2005-0090607-A1, incorporated herein by reference to the extent that the disclosure thereof does not contradict the disclosure contained in the specification.

In one embodiment, resistance domain 608 includes a polyurethane membrane with both hydrophilic and hydrophobic regions to control the diffusion of glucose and oxygen to an analyte sensor, the membrane being fabricated easily and reproducibly from commercially available materials. A suitable hydrophobic polymer component is a polyurethane, or polyetherurethaneurea. Polyurethane is a polymer produced by the condensation reaction of a diisocyanate and a difunctional hydroxyl-containing material. A polyurethaneurea is a polymer produced by the condensation reaction of a diisocyanate and a difunctional amine-containing material. Diisocyanates that may be used include, but are not limited to, aliphatic diisocyanates containing from about 4 to about 8 methylene units. Diisocyanates containing cycloaliphatic moieties can also be useful in the preparation of the polymer and copolymer components of the membranes of some embodiments. The material that forms the basis of the hydrophobic matrix of the resistance domain can be any of those known in the art as appropriate for use as membranes in sensor devices and as having sufficient permeability to allow relevant compounds to pass through it, for example, to allow an oxygen molecule to pass through the membrane from the sample under examination in order to reach the active enzyme or electrochemical electrodes. Examples of materials which can be used to make non-polyurethane type membranes include vinyl polymers, polyethers, polyesters, polyamides, inorganic polymers such as polysiloxanes and polycarbosiloxanes, natural polymers such as cellulosic and protein based materials, and mixtures or combinations thereof.

In one embodiment, the hydrophilic polymer component is polyethylene oxide. For example, one useful hydrophobic-hydrophilic copolymer component is a polyurethane polymer that includes about 20% hydrophilic polyethylene oxide. The polyethylene oxide portions of the copolymer are thermodynamically driven to separate from the hydrophobic portions of the copolymer and the hydrophobic polymer component. The 20% polyethylene oxide-based soft segment portion of the copolymer used to form the final blend affects the water pick-up and subsequent glucose permeability of the membrane.

In some embodiments, the resistance domain is formed from a silicone polymer modified to allow analyte (e.g., glucose) transport.

In some embodiments, the resistance domain is formed from a silicone polymer/hydrophobic-hydrophilic polymer blend. In one embodiment, the hydrophobic-hydrophilic polymer for use in the blend may be any suitable hydrophobic-hydrophilic polymer, including but not limited to components such as polyvinylpyrrolidone (PVP), polyhydroxyethyl methacrylate, polyvinyl alcohol, polyacrylic acid, polyethers such as polyethylene glycol or polypropylene oxide, and copolymers thereof, including, for example, di-block, tri-block, alternating, random, comb, star, dendritic, and graft copolymers (block copolymers are discussed in U.S. Pat. No. 4,803,243 and U.S. Pat. No. 4,686,044, both of which are incorporated herein by reference to the extent that the disclosure thereof does not contradict the disclosure contained in the specification). In one embodiment, the hydrophobic-hydrophilic polymer is a copolymer of poly(ethylene oxide) (PEO) and poly(propylene oxide) (PPO). Suitable such polymers include, but are not limited to, PEO-PPO diblock copolymers, PPO-PEO-PPO triblock copolymers, PEO-PPO-PEO triblock copolymers, alternating block copolymers of PEO-PPO, random copolymers of ethylene oxide and propylene oxide, and blends thereof. In some embodiments, the copolymers may be optionally substituted with hydroxy substituents. Commercially available examples of PEO and PPO copolymers include the PLURONIC^{®} brand of polymers available from BASF^{®}. In one embodiment, PLURONIC^{®} F-127 is used. Other PLURONIC^{®} polymers include PPO-PEO-PPO triblock copolymers (e.g., PLURONIC^{®} R products). Other suitable commercial polymers include, but are not limited to, SYNPERONICS^{®} products available from UNIQEMA^{®}. U.S. Patent Application Publication No. US-2007-0244379-A1, incorporated herein by reference to the extent that the disclosure thereof does not contradict the disclosure contained in the specification, describes systems and methods suitable for the resistance and/or other domains of the membrane system.

In some embodiments, resistance domain 608 is deposited onto enzyme domain 606 to yield a domain thickness of from about 0.05 microns to about 20 microns, or from about 0.05, 0.1, 0.15, 0.2, 0.25, 0.3, 0.35, 0.4, 0.45, 0.5, 1, 1.5, 2, 2.5, 3, or 3.5 microns to about 4, 5, 6, 7, 8, 9, 10, 11, 12, 13, 14, 15, 16, 17, 18, 19, or 19.5 microns, or from about 2, 2.5 or 3 microns to about 3.5, 4, 4.5, or 5 microns. The resistance domain may be deposited onto the enzyme domain by vapor deposition, spray coating, printing, or dip coating. In one embodiment, spray coating is the preferred deposition technique. The spraying process atomizes and mists the solution, and therefore most or all of the solvent is evaporated prior to the coating material settling on the underlying domain, thereby minimizing contact of the solvent with the enzyme.

In another embodiment, physical vapor deposition (e.g., ultrasonic vapor deposition) is used for coating one or more of the membrane domain(s) onto the electrodes, wherein the vapor deposition apparatus and process include an ultrasonic nozzle that produces a mist of micro-droplets in a vacuum chamber. In these embodiments, the micro-droplets move turbulently within the vacuum chamber, isotropically impacting and adhering to the surface of the substrate. Advantageously, vapor deposition as described above can be implemented to provide high production throughput of membrane deposition processes (e.g., at least about 20 to about 200 or more electrodes per chamber), greater consistency of the membrane on each sensor, and increased uniformity of sensor performance, for example, as described below.

In some embodiments, depositing the resistance domain (for example, using one of the techniques described above) includes formation of a membrane system that substantially blocks or resists ascorbate (a known electrochemical interferent in hydrogen peroxide-measuring glucose sensors). During the process of depositing the resistance domain, a structural morphology may be formed that is characterized in that ascorbate does not substantially permeate therethrough.

In one embodiment, resistance domain 608 is deposited on enzyme domain 606 by spray coating a solution of from about 1 wt. % to about 5 wt. % polymer and from about 95 wt. % to about 99 wt. % solvent. In spraying a solution of resistance domain material, including a solvent, onto the enzyme domain, it is desirable to mitigate or substantially reduce any contact with enzyme of any solvent in the spray solution that can deactivate the underlying enzyme of the enzyme domain. Tetrahydrofuran (THF) is one solvent that minimally or negligibly affects the enzyme of the enzyme domain upon spraying. Other solvents can also be suitable for use, as is appreciated by one skilled in the art.

Although a variety of spraying or deposition techniques can be used, spraying the resistance domain material and rotating the sensor at least one time by 180° can typically provide adequate coverage by the resistance domain. Spraying the resistance domain material and rotating the sensor at least two times by 120° provides even greater coverage (one layer of 360° coverage), thereby ensuring resistivity to glucose, such as is described in more detail above.

In some embodiments, resistance domain 608 is spray coated and subsequently cured for a time of from about 15 minutes to about 90 minutes at a temperature of from about 40° C. to about 60° C. (and can be accomplished under vacuum (e.g., from 20 to 30 mmHg)). A cure time of up to about 90 minutes or more can be advantageous to ensure complete drying of the resistance domain.

In one embodiment, the resistance domain is formed by spray coating at least six layers (namely, rotating the sensor seventeen times by 120° for at least six layers of 360° coverage) and curing at 50° C. under vacuum for 60 minutes. However, the resistance domain can be formed by dip coating or spray coating any layer or plurality of layers, depending upon the concentration of the solution, insertion rate, dwell time, withdrawal rate, and/or the desired thickness of the resulting film. Additionally, curing in a convection oven can also be employed.

In certain embodiments, a variable frequency microwave oven can be used to cure the membrane domains/layers. In general, microwave ovens directly excite the rotational mode of solvents. Consequently, microwave ovens cure coatings from the inside out rather than from the outside in as with conventional convection ovens. This direct rotational mode excitation is responsible for the typically observed "fast" curing within a microwave oven. In contrast to conventional microwave ovens, which rely upon a fixed frequency of emission that can cause arcing of dielectric (metallic) substrates if placed within a conventional microwave oven, Variable Frequency Microwave (VFM) ovens emit thousands of frequencies within 100 milliseconds, which substantially eliminates arcing of dielectric substrates. Consequently, the membrane domains/layers can be cured even after deposition on metallic electrodes as described herein. VFM curing can increase the rate and completeness of solvent evaporation from a liquid membrane solution applied to a sensor, as compared to the rate and completeness of solvent evaporation observed for curing in conventional convection ovens. In certain embodiments, VFM is can be used together with convection oven curing to further accelerate cure time. In some sensor applications wherein the membrane is cured prior to application on the electrode (see, for example, U.S. Patent Application Publication No. US-2005-0245799-A1, incorporated herein by reference to the extent that the disclosure thereof does not contradict the disclosure contained in the specification) conventional microwave ovens (e.g., fixed frequency microwave ovens) can be used to cure the membrane layer.

A variety of therapeutic (bioactive) agents can be used with the analyte sensor system of some embodiments. In some embodiments, the therapeutic agent is an anticoagulant. In some embodiments, an anticoagulant is included in the analyte sensor system to prevent coagulation within or on the sensor (e.g., within or on the catheter or within or on the sensor). In some embodiments, the therapeutic agent is an antimicrobial, such as but not limited to an antibiotic or antifungal compound. In some embodiments, the therapeutic agent is an antiseptic and/or disinfectant. Therapeutic agents can be used alone or in combination of two or more of them. The therapeutic agents can be dispersed throughout the material of the sensor (and/or catheter). In some embodiments, the membrane system of some embodiments includes a therapeutic agent, which is incorporated into at least a portion of the membrane system, or which is incorporated into the device and adapted to diffuse through the membrane. There are a variety of systems and methods by which the therapeutic agent is incorporated into the membrane. In some embodiments, the therapeutic agent is incorporated at the time of manufacture of the membrane system. For example, the therapeutic agent can be blended prior to curing the membrane system, or subsequent to membrane system manufacture, for example, by coating, imbibing, solvent-casting, or sorption of the bioactive agent into the membrane system. Although the therapeutic agent may be incorporated into the membrane system, in some embodiments the therapeutic agent can be administered concurrently with, prior to, or after insertion of the device intravascularly, for example, by oral administration, or locally, for example, by subcutaneous injection near the implantation site. A combination of therapeutic agent incorporated in the membrane system and therapeutic agent administration locally and/or systemically can be used in certain embodiments.

As a non-limiting example, in some embodiments, the analyte sensor 110 is a continuous electrochemical analyte sensor configured to provide at least one working electrode and at least one reference electrode, which are configured to measure a signal associated with a concentration of the analyte in the host. The output signal is typically a raw data stream that is used to provide a useful value of the measured analyte concentration in a host to the patient or doctor, for example. However, the analyte sensors of some embodiments comprise at least one additional working electrode configured to measure at least one additional signal, as discussed elsewhere herein. For example, in some embodiments, the additional signal is associated with the baseline and/or sensitivity of the analyte sensor, thereby enabling monitoring of baseline and/or sensitivity changes that may occur over time.

In general, electrochemical continuous analyte sensors define a relationship between sensor-generated measurements (for example, current in pA, nA, or digital counts after A/D conversion) and a reference measurement (for example, glucose concentration mg/dL or mmol/L) that are meaningful to a user (for example, patient or doctor). For example, in the case of an implantable diffusion-based glucose oxidase electrochemical glucose sensor, the sensing mechanism generally depends on phenomena that are linear with glucose concentration, for example: (1) diffusion of glucose through a membrane system (for example, biointerface membrane and membrane system) situated between implantation site and/or the electrode surface, (2) an enzymatic reaction within the membrane system, and (3) diffusion of the H₂O₂ to the sensor. Because of this linearity, calibration of the sensor can be understood by solving an equation: *y*=*mx*+*b* in which y represents the sensor signal (e.g., counts), x represents the estimated glucose concentration (e.g., mg/dL), m represents the sensor sensitivity to glucose (e.g., counts/mg/dL), and b represents the baseline signal (e.g., counts). When both sensitivity m and baseline (background) b change over time in vivo, calibration can be improved by using at least two independent, matched data pairs (x₁, y₁; x₂, y₂) to solve for m and b and thus allow glucose estimation when only the sensor signal, y is available. Matched data pairs can be created by matching reference data (for example, one or more reference glucose data points from a blood glucose meter, or the like) with substantially time corresponding sensor data (for example, one or more glucose sensor data points) to provide one or more matched data pairs, such as described in co-pending U.S. Patent Application Publication No. US-2005-0027463-A1, incorporated herein by reference to the extent that the disclosure thereof does not contradict the disclosure contained in the specification. In some implantable glucose sensors, such as described in more detail in U.S. Pat. No. 6,329,161 to Heller et al., incorporated herein by reference to the extent that the disclosure thereof does not contradict the disclosure contained in the specification, the sensing layer utilizes immobilized mediators (e.g., redox compounds) to electrically connect the enzyme to the working electrode, rather than using a diffusional mediator. In some implantable glucose sensors, such as described in more detail in U.S. Pat. No. 4,703,756, incorporated herein by reference to the extent that the disclosure thereof does not contradict the disclosure contained in the specification, the system has two oxygen sensors situated in an oxygen-permeable housing, one sensor being unaltered and the other contacting glucose oxidase allowing for differential measurement of oxygen content in body fluids or tissues indicative of glucose levels. A variety of systems and methods of measuring glucose in a host are known, all of which may benefit from some of all of the embodiments to provide a sensor having a signal-to-noise ratio that is not substantially affected by non-constant noise.

Advantageously, continuous analyte monitoring is enabled. For example, when the analyte is glucose, continuous glucose monitoring enables tight glucose control, which can lead to reduced morbidity and mortality among diabetic hosts. In some embodiments, the medical staff is not unduly burdened by additional patient interaction requirements.

Advantageously, there is no net sample (e.g., blood) loss for the host, which is a critical feature in some clinical settings. For example, in a neonatal intensive care unit, the host is extremely small and loss of even a few milliliters of blood can be life threatening. Furthermore, returning the body fluid sample to the host, instead of delivering to a waste container greatly reduces the accumulation of biohazardous waste that requires special disposal procedures. The integrated sensor system components, as well as their use in conjunction with an indwelling analyte sensor, are discussed in greater detail below.

A variety of known sensor configurations and/or features can be employed with the sensor systems described herein, such as U.S. Pat. No. 5,711,861 to Ward et al., U.S. Pat. No. 6,642,015 to Vachon et al., U.S. Pat. No. 6,654,625 to Say et al., U.S. Pat. No. 6,565,509 to Say et al., U.S. Pat. No. 6,514,718 to Heller, U.S. Pat. No. 6,465,066 to Essenpreis et al., U.S. Pat. No. 6,214,185 to Offenbacher et al., U.S. Pat. No. 5,310,469 to Cunningham et al., and U.S. Pat. No. 5,683,562 to Shaffer et al., U.S. Pat. No. 6,579,690 to Bonnecaze et al., U.S. Pat. No. 6,484,046 to Say et al., U.S. Pat. No. 6,512,939 to Colvin et al., U.S. Pat. No. 6,424,847 to Mastrototaro et al., U.S. Pat. No. 6,424,847 to Mastrototaro et al, U.S. Patent Application Publication No. US-2006-0020187-A1 to Brister et al., U.S. Patent Application Publication No. US-2007-0027370-A1 to Brauker et al., U.S. Patent Application Publication No. US-2005-0143635-A1 to Kamath et al., U.S. Patent Application Publication No. US-2007-0027385-A1 to Brister et al., U.S. Patent Application Publication No. US-2007-0213611-A1 to Simpson et al., U.S. Patent Application Publication No. US-2008-0083617-A1 to Simpson et al., U.S. Patent Application Publication No. US-2008-0119703-A1 to Brister et al., U.S. Patent Application Publication No. US-2008-0108942-A1 to Brister et al., and U.S. Patent Application Publication No. US-2009-0018424-A1 Kamath et al., (as examples) all of which are incorporated herein by reference to the extent that the disclosure thereof does not contradict the disclosure contained in the specification. The above-referenced patents and publications are not inclusive of all applicable analyte sensors; in general, it should be understood that the disclosed embodiments are applicable to a variety of analyte sensor configurations.

Additional features and details that may be included, particularly for sensors having elongated conductive or non-conductive cores are described in U.S. Patent 9,131,885, which is incorporated herein by reference in its entirety to the extent that the disclosure thereof does not contradict the disclosure contained in the specification.

Although the embodiments of sensor 110 described above in connection with FIGS 5A, 5B, 5C, and 6A include an elongated conductive core, this is merely illustrative.

In other embodiments, an elongated non-conductive structure such as an elongated polymer structure or an elongated fiber structure may employed as a core. In yet other embodiments, sensor 110 may be formed from a plurality of substantially planar layers. In embodiments that include an elongated non-conductive core, the core may be provided with conductive traces and/or other contacts to form the elongated conductive body as described in further detail hereinafter in connection with various embodiments.

In various embodiments, continuous analyte sensors 110 are described that include metallic, polymer, or fiber cores and/or include a plurality of substantially planar layers. In general, in various embodiments, a metallic core, a polymer core, or a fiber core, or a planar-layered sensor may be provided with a flexibility that is defined by a Young's modulus of, for example, less than 147 GPa, a flexural modulus of, for example, less than 147 GPa (e.g., for a metallic core implementation) or less than between 2-5 GPa (e.g., for a polymer core or fiber reinforced core implementation), and/or a buckling force of, for example, less than 0.25 N, less than 0.02 N, less than 0.01 N, less than 0.001 N, or that is substantially zero or less than the sensor's own weight, in some embodiments. It will be appreciated that, in materials with isotropic properties, the Young's modulus can be reflective of the flexural modulus and hence the stiffness of a sensor. In various embodiments, a core material (e.g., in embodiments with an elongated conductive or non-conductive core) and/or a layer of material (e.g., a layer formed around an elongated core or one of a plurality of substantially planar layers) may be a state-change material having a Young's modulus, a flexural modulus, and/or a buckling force that decreases from an ex vivo value to an in vivo value such that an implanted sensor softens upon implantation. In this way, a continuous analyte sensor (or at least a support structure therein such as a core or a substantially planer or other layer) may be relatively stiff (e.g., freestanding) ex vivo to facilitate manufacturing, packaging, and/or implantation processes and may become relatively flexible (e.g., non-freestanding) in vivo.

Additionally, a measurement of the sensor's ability to withstand the implantation environment is fatigue life. In some embodiments, the fatigue life of sensor 110 having a metallic, polymer, or fiber core or formed from a plurality of substantially planar layers is at least 1,000 cycles of flexing of from about 28° to about 110° at a bend radius of about 0.125-inches.

In the configuration of Fig. 6A, membrane 508 is formed on a substantially circular body 502 and, in cross-section, substantially surrounds the core. This is merely illustrative. In other embodiments, membrane 508 may include a plurality of substantially planar membrane layers disposed on a substantially planar working electrode as in the example of Fig. 6B.

As shown in Fig. 6B, electrode domain 602 may be a substantially planar layer formed on a substantially planar conductive layer 610. Conductive layer 610 may, for example, be a platinum, platinum alloy, or other conductive coating formed on an underlying substantially planar layer 612 such as a silver trace layer or a core (e.g., a substantially cylindrical core of an elongated body). Trace layer 612 or core may be formed on one or more additional substantially planar layers such as insulating layers, working electrode layers, auxiliary electrode layers, reference electrode layers, counter electrode layers, and/or other conductive layers as discussed herein. In the example of Fig. 6, interference domain 604 is implemented as a substantially planar interference layer formed on the substantially planar electrode domain 602, enzyme domain 606 is implemented as a substantially planar enzyme layer formed on the substantially planar interference domain, and resistance domain 608 is implemented as a substantially planar interference layer formed on the substantially planar enzyme domain. In other embodiments, a biointerface or bioprotective domain (not shown) may be incorporated into the membrane system to help control a foreign body response. For example, the biointerface or bioprotective domain may be used to reduce biomaterial-associated inflammation or to reduce the rate of biofouling of the membrane, thereby increasing the longevity of an implanted device. In various embodiments, any of the above-described domains (e.g., disclosed in Figs. 6A or 6B) can be omitted, altered, substituted for, and/or incorporated together without departing from the spirit of the preferred embodiments. For example, in certain embodiments, an electrode domain may not exist. In some of these embodiments, the interference domain may be designed to accomplish the function of the electrode domain.

In embodiments such as the embodiment shown in Fig. 6B in which sensor 110 is formed from a plurality of substantially planar layers, membrane 508 may be formed locally on the working electrode (e.g., using printing or other patterned deposition operations) such that other portions of sensor 110 are substantially free of membrane materials. In this way, the cost of the sensor may be reduced by reducing the amount of unused membrane material formed on the sensor (e.g., membrane material formed away from the working electrode).

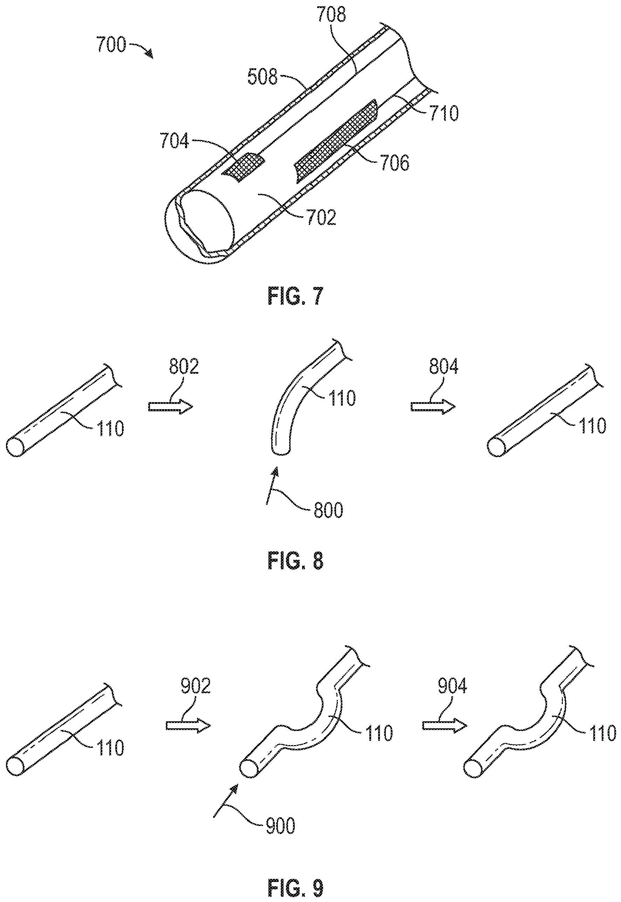

The membrane 508 of Fig. 6A and variations described herein may also be implemented in various sensors having a non-conductive (e.g., polymer or fiber core). For example, Fig. 7 illustrates a continuous analyte sensor having an elongated non-conductive core. In this particular embodiment, the sensor 700 (e.g., an implementation of continuous analyte sensor 110) comprises an elongated core 702 configured and arranged for multi-axis bending. The elongated core 702 (e.g., a non-conductive implementation of core 510) comprises a non-conductive material (e.g., a flexible polymer/polymeric material including polyurethane and/or polyimide and/or a shape-memory or other state-change polymer material as described herein). A working electrode 704 may be located on the elongated core 702. A reference or counter electrode 706 may also be located on the elongated core 702. A membrane 508 may be disposed on the core 702 covering at least the working electrode 704. Conductive pathways 708 and 710 (e.g., conductive traces) may run along a length of elongated non-conductive core 702 to connect the working electrode 704 and the reference or counter electrode 706 (respectively) to the sensor electronics (not shown). In aggregate, core 702 and the conductive features formed thereon (e.g., one or more working electrodes, reference or counter electrodes, conductive traces, etc.) may form an elongated conductive body for a flexible analyte sensor 110.

In various embodiments, the sensor of Fig. 7 may be configured and arranged such that the fatigue life of the sensor is at least 1,000 cycles of flexing of from about 28° to about 110° at a bend radius of about 0.125 inches. Non-conductive core 702 may have a Young's modulus of, for example, less than 147 GPa, less than 1GPa, less than 1MPa, or between 1kPa and IMPa (as examples).

In some embodiments, non-conductive core 702 may be formed from a material configured to soften in vivo responsive to environmental influences from tissue in which sensor 110 is embedded and/or responsive to external influences such as externally applied or removed electromagnetic fields. For example, non-conductive core 702 may, in some embodiments, be formed from a shape-memory material with a temperature-dependent Young's modulus, flexural modulus, and/or buckling force. For example, when sensor 700 is implanted in a host, the rising temperature of the core 702, due to absorption of heat from host tissue (e.g., host tissue having a temperature of between 78° F and 98° F), may soften core 702 such that the Young's modulus, the flexural modulus, and/or the buckling force of the core 702 is reduced (e.g., by a factor of five, ten, 100, or more than 100). As another example, hydration of the core 702 due to absorption of a fluid such as water from the host tissue may cause softening of core 702 such that the Young's modulus, the flexural modulus, and/or the buckling force of the core 702 is reduced (e.g., by a factor of five, ten, 100 or more than 100). In some embodiments, the Young's modulus, the flexural modulus, and/or the buckling force may be reduced by one, two, three, or more than three orders of magnitude response to absorption of heat and liquid from the host tissue.

For example, core 702 may be formed from a softenable, shape-memory thermoplastic material. A softenable, shape-memory thermoplastic material may include a copolymer (e.g., a block or segmented copolymer) and another polymer. The other polymer may be blended with the copolymer to act as an antiplasticizer. Suitable copolymers may include polyetherurethanes, polyester-urethanes, polyether-polyesters, and/or polyetherpolyamides. In one suitable example, core 702 may be formed from a polyester/polyether block copolymer with high molecular weight phenoxy. Other examples of shape memory polymers that may be used to form core 702 include polynorbornene, high molecular weight (e.g., more and 100 kDa) poly(methyl methacrylate), poly(alkyl methacrylate) copolymers, polystyrene copolymers, filler-modified epoxy networks, chemically cross-linked amorphous polyurethanes, poly((methyl methacrylate)-co-(N-vinyl-2-pyrrolidone))-PEG semi-I PNs, HDI-HPED-TEA network, and biodegradable copolyester-urethane networks.

For example, the softenable, shape-memory thermoplastic material may be formed from a blend of polymeric materials suitably chosen to set the stiffness (e.g., a freestanding stiffness) of core 702 ex vivo and to determine a transition temperature (e.g., a temperature of between 78° F and 98° F) at which core 702 substantially softens (e.g., to become non-freestanding in some implementations). Core 702 may be hydrophilic such that absorption of water in vivo may further lower the transition temperature and speed the softening of core 702 upon implantation. As discussed in further detail hereinafter, polymeric materials may be blended and suitably shaped using an extruder in some embodiments.

Figs. 8 and 9 show examples of a freestanding sensor 110 and a non-freestanding sensor 110. For example, the configuration of Fig. 8 may represent an ex vivo configuration of sensor 110 in which sensor 110 is a freestanding sensor (e.g., a sensor having a Young's modulus and/or a flexural modulus of greater than 500 MPa or having a buckling force that is substantially greater than the weight of the sensor under gravity). For example, the configuration of Fig. 9 may represent an in vivo configuration of sensor 110 in which sensor 110 is a non-freestanding sensor (e.g., a sensor having a Young's modulus and/or a flexural modulus of less than 500 MPa or having a buckling force that is less than 0.01 N or that substantially less than the weight of the sensor under gravity). A sensor as shown in Fig. 9 may be non-freestanding following a transition from a freestanding sensor as shown in Fig. 8 or may be a permanently non-freestanding sensor in some implementations.

As shown in Fig. 8, a freestanding sensor 110 may, as indicated by arrow 802 bend or deform when a sufficient force 800 is applied thereto and may return, as indicated by arrow 804, to the same shape and position as prior to application of force 800, when the force is withdrawn. As shown in Fig. 9, a non-freestanding sensor 110 may, as indicated by arrow 902 bend or deform at various locations when a sufficient force (e.g., a force greater than the buckling force of sensor 110) 900 is applied thereto and may remain, as indicated by arrow 904, in the bent or deformed shape, when the force is withdrawn. Force 900 of FIG. 9 may be greater than force 800 of FIG. 8 in some scenarios. However, in some arrangements the buckling force of sensor 1 10 may be reduced (e.g., due to in vivo placement) such that force 800 can cause the buckling shown in FIG. 9.

In various embodiments, an elongated core such as core 510 of Figs. 5A-6A and/or core 702 of Fig. 7 can be formed out of any nonconductive material that can be formed into a thin, elongated structure. In further embodiments, the nonconductive material is a polymer. The polymer may be a nylon or polyester filament, string or cord, etc. In some embodiments, the elongated body is non-planar, such as described herein, and thus has a non-rectangular cross-section. However, in certain embodiments, the elongated body is planar. In some embodiments, the smallest dimension (e.g., the diameter or width) of the elongated body is less than about 0.004 inches. However, in certain embodiments, relatively larger or smaller sensor diameters are acceptable, such as described elsewhere herein.

The sensor 700 illustrated in Fig. 7 can be manufactured using a variety of techniques as described herein. In some embodiments, the working electrode 704 is applied by depositing a conductive material (e.g., at least one of platinum, platinum-iridium, gold, palladium, iridium, graphite, carbon, a conductive polymer and an alloy) on the elongated body. In some embodiments, the conductive material is an ink, paint or paste, and is deposited using thick film and/or thin film deposition techniques known in the art, such as but not limited to screen printing, jet printing, block printing, and/or other techniques as described herein. However, in some embodiments the working electrode material is plated on the elongated body, using plating techniques known in the art, such as but not limited to electroplating. The reference and/or counter electrode can be applied by depositing a silver-containing material on the elongated body. Similar to the working electrode, the silver-containing material of the reference electrode can be deposited using thick film and/or thin film deposition techniques, various printing techniques known in the art, and/or plating. The membrane 508 may be applied to at least the working electrode using one or more processes as described herein. In particular, in some embodiments, the membrane is applied by applying a polymer having a Shore hardness of from about 70 A to about 55 D. As described with reference to Figs. 6A and 6B, the membrane can include a plurality of layers and/or domains.

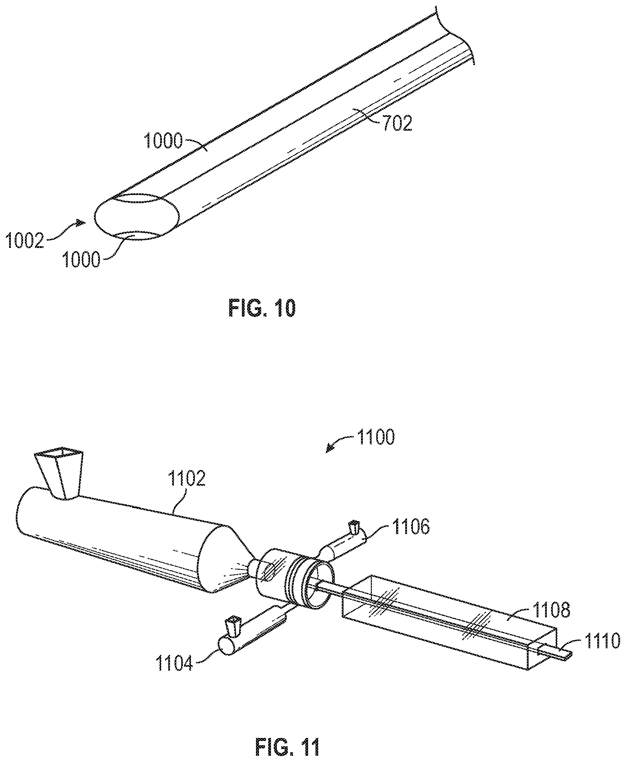

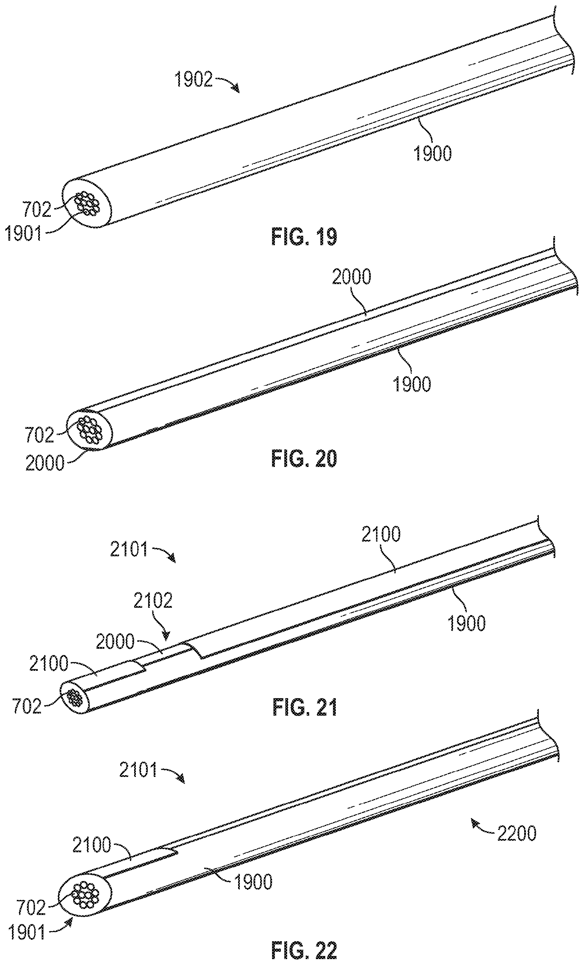

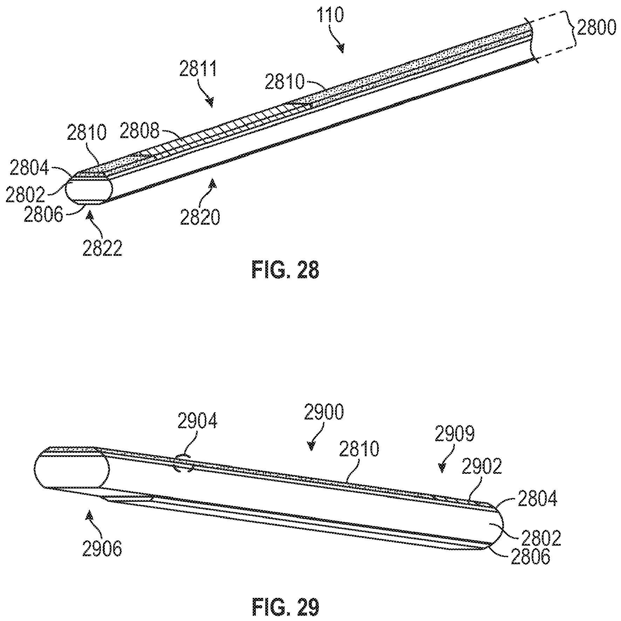

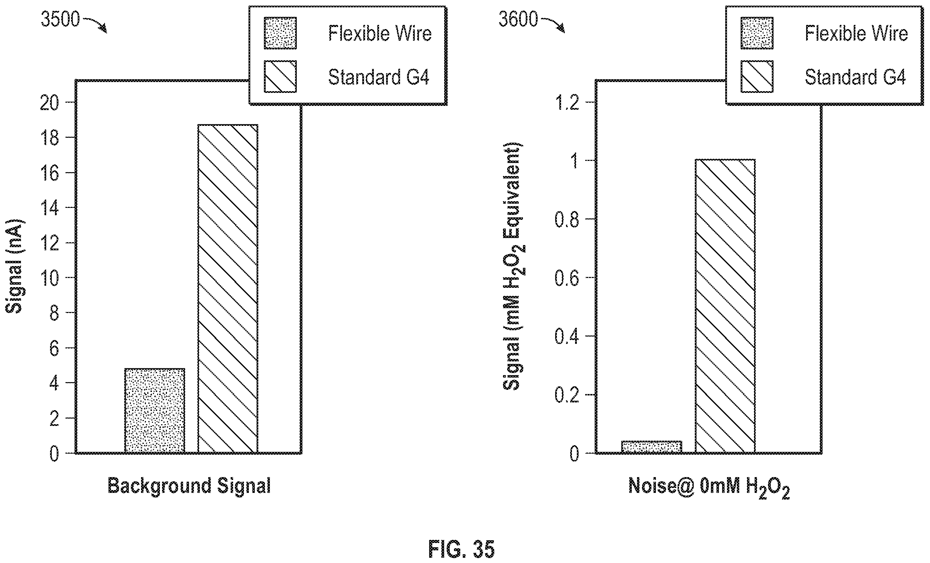

Various exemplary arrangements and manufacturing processes for a continuous analyte sensor 110 having a non-conductive core 702 are described in connection with Figs. 10-27. In particular, Figs. 10-18 and 27 illustrate non-conductive polymer core implementations for sensor 110 and Figs. 19-26 illustrate fiber core implementations for sensor 110. Various implementations in which sensor 110 includes a plurality of substantially planar layers are then described in connection with Figs. 28-34. Various features and properties of flexible analyte sensors 110 relating to implementations with flexible metallic cores, flexible non-conductive cores, and/or pluralities of planar layers are discussed in connection with Figs. 35-45.

In the examples of Figs. 10-18 and 27, a polymer-based or polymeric core 702 is described and various methods and arrangements for forming a flexible analyte sensor having a working (e.g., platinum) electrode with a well-defined surface area, a reference (e.g., silver and/or silver chloride) electrode, one or more conductive traces for interfacing between the electrodes and sensor electronics 112, and suitable insulation/patterns for sensor electronic interfacing are described.

As shown in Fig. 10, a polymer-based core 702 for a flexible analyte sensor 110 may have an elliptical cross-sectional shape 1002 and may be provided with one or more conductive traces 1000 thereon. Conductive traces 1000 may, for example, be formed from carbon black, platinum, platinum-iridium, silver/silver chloride, or other suitable conductive material. The elliptical cross-sectional shape of core 702 may provide for enhanced twist and/or orientation control for sensor 110 and can be beneficial during sensor manufacturing so that no twist in the "line" develops in a reel to reel process. For example, the elliptical cross-sectional shape (or any similar cross-sectional shape having such as a rectangular cross-sectional shape having a major dimension that is larger than an orthogonal minor dimension) may provide a preferred bend axis parallel to the major axis. In this way, potential bend damage to structures such as electrodes and/or traces formed on the core may be reduced. In the example of Fig. 10, conductive traces 1000 are formed in grooves on opposing sides of the major axis of core 702. Providing an elongated sensor body with an elliptical cross-section may also provide the advantages of a large surface area, devoid of sharp edges, for electrode configuration. A relatively large surface area electrode can therefore be provided which can be beneficial for sensing and averaging the glucose measured in interstitial fluid and minimizing the influence of local environment fluctuation. Providing an elliptical cross-section sensor that is devoid of sharp edges can help reduce local stress and tissue damage and provide a more desirable implant design.

Fig. 11 shows a manufacturing system for forming core 702 and/or conductive traces 1000. As shown in Fig. 11, manufacturing system 100 may be an extruding system that includes a filament extruder 1102 for extruding core 702 from, for example, a blend of copolymer and other polymer material(s) as described above. Filament extruder 1102 may, for example, be a twin-screw extruder configured to blend and extrude core 702 (for example). In the example of Fig. 11, conductive traces 1000 are co-extruded with core 702 using trace extruders 1104 and 1106. A cooling medium 1108 may then cool the output of the extruder system to form an elongated core structure 1110 such as a core structure having a polymer core 702 and conductive traces 1000.

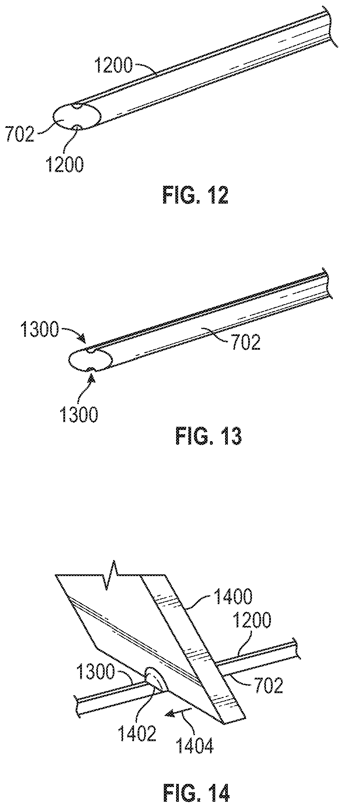

However, this is merely illustrative. In other embodiments, conductive traces such as conductive traces 1200 of Fig. 12 may be deposited into grooves such as grooves 1300 of Fig. 13 after formation (e.g., via extrusion) of a grooved core 702. For example, traces 1200 may be deposited into grooves 1300 using a squeegee 1400 after core 702 has been formed, as illustrated in Fig. 14. In one embodiment, traces 1200 are formed by wiping a liquid conductor 1402 (e.g., a conductive ink such as a polymeric liquid with carbon, platinum, silver, or other conductive particles suspended therein) into grooves 1300 by moving squeegee 1400 along core 702 as indicated by arrow 1404.

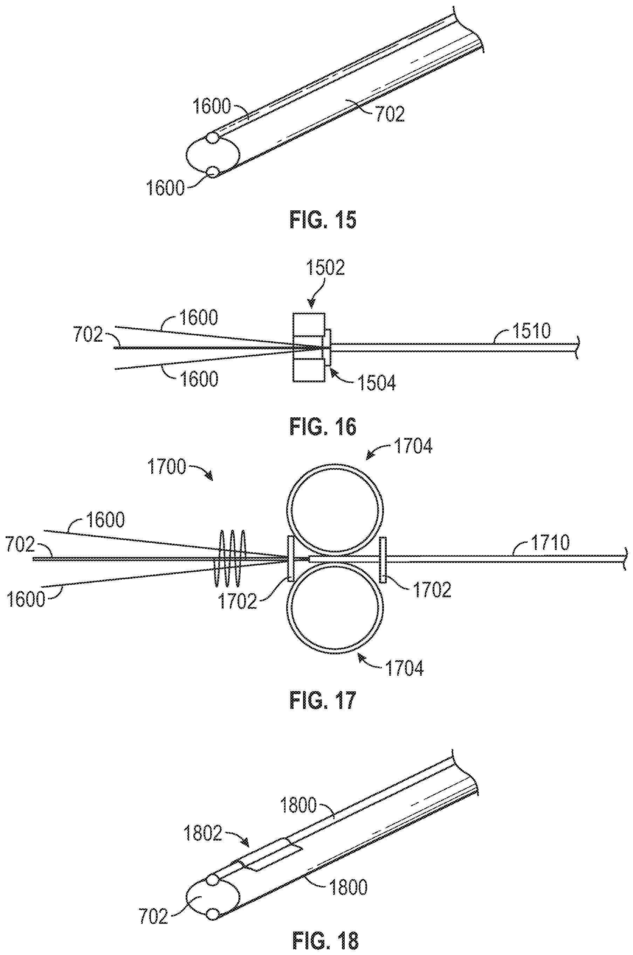

In other embodiments, a conductive ribbon such as a metal ribbon may be laminated (e.g., heat laminated) into one or more grooves 1300 in a non-conductive core 702. Fig. 16 shows an example implementation in which core 702 includes a pair of conductive ribbon traces 1600 (e.g., silver or silver chloride traces) disposed in grooves on opposing sides of core 702.

Fig. 15 shows a manufacturing system that may be used for placing traces 1600 into the grooves in core 702. As shown in Fig. 15, core 702 and traces 1600 may be fed (e.g., between reels, spools, or other feeder equipment) through a fiber heater 1502 and laminating die 1504 such that traces 1600 are heated and laminated onto core 702 to form an elongated conductive core structure 1510 for a flexible analyte sensor 110. However, the system of Fig. 15 is merely illustrative. Fig. 16 shows another exemplary manufacturing system that may be used for laminating conductive traces onto core 702. In the example of Fig. 16, an induction heater 1700 is used to heat traces 1600 prior to contact with core 702. As illustrated in Fig. 17, after heating by induction heater 1700, positioning guides 1702 may be used to guide core 702 and heated traces 1600 between rollers 1704 to generate contact pressure between heated traces 1600 and core 702 to laminate traces 1600 onto core 702 to form an elongated conductive core structure 1710 for a flexible analyte sensor 110. In this way, heat and pressure can be applied primarily to traces 1600 such that heating of core 702, and potential related problems and/or defects (e.g., necking and breaking under heating and tension in a reel-to-reel process) can be avoided.

In various embodiments, one or more portions of traces 1600 may be used to form a portion of a working electrode or a reference electrode for a flexible analyte sensor 110, or traces 1600 may be implementations of traces 708 and 710 of Fig. 7 that conductively couple respective electrodes to, for example, sensor electronics at a back end of core 702 that is proximal to or within the sensor electronics housing. Fig. 18 shows an exemplary elongated conductive core formed from an elongated polymer core 702 with traces 1800 (e.g., implementations of traces 1000, 1200, or 1600) and an electrode 1802 formed over and in conductive contact with a trace 1800.

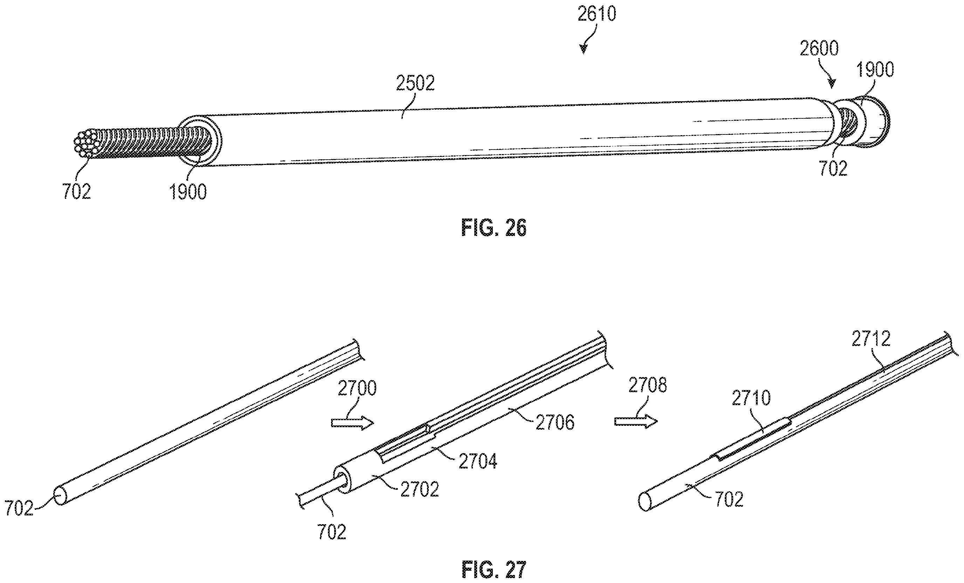

Electrode 1802 may be formed, for example, by pad printing, stenciling, aerosol jet printing or any other suitable deposition or printing processes as would be understood by one skilled in the art. Pad printing may, as one illustrative example, include providing conductive ink from a container into an electrode-shaped recess in a substrate, pressing a transfer pad against the conductive ink in the electrode-shaped recess to lift the conductive ink having the electrode shape on to the transfer pad, and pressing the transfer pad with the electrode-shaped conductive ink onto core 702 and trace 1800 to deposit the conductive ink having the desired shape onto core 702 in conductive communication with trace 1800. Stenciling and aerosol jet printing are described in further detail hereinafter in connection with, for example, Fig. 27.

In the various examples of Figs. 7, 10, 12, 15, and 18, other layers (e.g., insulating layers, additional electrode layers, membrane layers, or the like) may be formed over one or more portions of core 702 and/or traces disposed thereon using one or more methods as descried herein to form a completed flexible analyte sensor for coupling to sensor electronics 112.

In some embodiments, core 702 may include materials and/or components that are resistant to mechanical fatigue and heat deformation. For example, in some embodiments, a non-conductive core 702 may be implemented as a fiber core having one or more fiber elements. Fiber elements may prevent the sensor substrate from exhibiting heat-related stretching and elongation, which may be desirable in, for example, reel-to-reel manufacturing processes.

Fig. 19 shows an exemplary implementation of non-conductive core for a flexible analyte sensor implemented using a bundle of fibers 1901. Fibers 1901 may, for example, be para-aramid synthetic fibers such as Kevlar^{®} fibers that prevent changes in core diameter due to heating under tension, for example during co-extrusion, and provide longitudinal strength that prevents stretching. Fibers 1901 may be encapsulated in an elongated insulating body 1900. Elongated insulating body 1900 may be formed from a thermoplastic adhesive in some implementations. For example, providing an elongated insulating body 1900 formed from a thermoplastic adhesive may enhance adhesion of subsequent metal ribbons for forming conductive traces. Elongated insulating body 1900 and fiber core 702 may be co-extruded to form an elongated non-conductive core structure 1902 for a flexible analyte sensor 110. In addition, a fiber reinforced non-conductive core may provide the advantages of resisting axial deformation, which may be helpful to maintain the surface area of the electrode and electric connectivity for the sensor.

As shown in Fig. 20, one or more conductive traces such as conductive trace 2000 may be formed on elongated insulating body 1900. Conductive trace 2000 may be, for example, a platinum or platinum alloy trace (as examples) and may be attached to elongated insulating body 1900 by applying heat and pressure to trace 2000 and body 1900 using a heater and laminating die as described above in connection with Fig. 15 to at least partially melt trace 2000 and/or body 1900 for lamination of trace 2000 onto body 1900. As shown in Fig. 21, a patterned insulating layer 2100 may be formed over a portion of trace 2000 such that a window 2102 in insulating layer 2100 is formed over a portion of trace 2000 to be used as an electrode (e.g., a working electrode). Although only one side of elongated conductive core structure 2101 is shown in Fig. 21, in some implementations conductive traces 2000 (see, e.g., Fig. 20) and patterned insulating layers 2100 may be formed on opposing sides of core 1902 to form a working electrode on a first side of body 2101 and an additional (e.g., working, reference, or counter) electrode on an opposing second side of body 2101. As shown in Fig. 19, core structure 1902 may be formed by a substantially cylindrical fiber bundle 1901 encapsulated in an elongated insulating body having an elliptical cross-section to provide a measure of twist and/or orientation control for core structure 1902 and a large surface area for electrode configuration. Although some of the embodiments described herein have two grooves, traces, and/or electrodes formed on opposing sides of a core, in alternative embodiments, there may be three, four, five, six, seven, eight, nine, ten, eleven, twelve, or more grooves, traces, and/or electrodes. In some of these alternative embodiments, the grooves, traces, and/or electrodes may be substantially symmetrical along a longitudinal axis of the elongated core. For example, in one embodiment with three grooves, traces, and/or electrodes, the three grooves, traces, and/or electrodes may be substantially equal distant from the longitudinal axis of the elongated core and substantially equally spaced from each other with respect to a cross-sectional perspective view of the elongated body.

Fig. 22 shows an exemplary back or proximal end 2200 (e.g., proximal to the sensor electronics than the distal end shown in Fig. 21) of an elongated conductive body formed from a non-conductive fiber reinforced core, showing how the bundle 1901 of fibers, encapsulated in elongated insulating body 1900 may extend to the back end and showing how insulating layer 2100 may be patterned at the back end

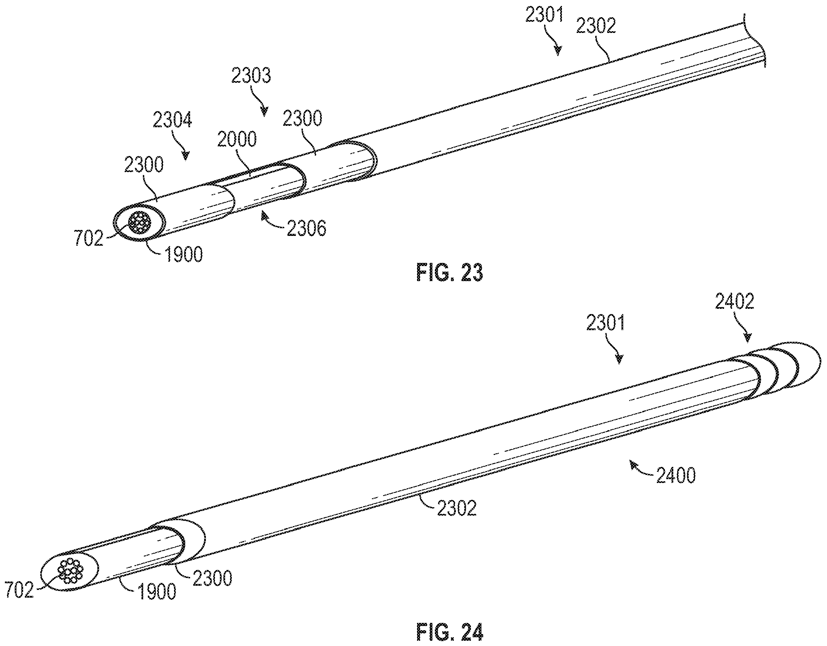

Analyte sensing using a flexible analyte sensor with a fiber reinforced core may be improved by providing additional working, counter, and/or reference electrode sensing areas to provide spatial signal average from multiple electrodes in some embodiments. For example, in some embodiments, a fiber-core flexible analyte sensor may be provided with symmetrically mirror-oriented conductive traces 2000 (e.g., platinum or platinum-based ribbons) and a coaxial coating 2302 (e.g., a AgCl coating) for both spatial averaging of the working signal and for increased reference loading using the coaxial coating 2302, as shown in Fig. 23. As shown, a coaxial insulating layer 2300 may be disposed between coating 2302 and traces 2000 to electrically insulate coating 2302 from traces 200. A window 2303 (e.g., a cutaway portion of insulating layer 2300) may be provided to form symmetrically disposed working electrodes on first side 2304 and second side 2306 of flexible elongated conductive body 2301. Because insulating layer 2300 and electrode layer 2302 extend around the entire circumference of the fiber reinforced core in the example of Fig. 23, forming and/or patterning of these layers may be faster, less complicated, and/or less costly that forming ribbon-type layers as shown in, for example, Fig. 21.

Fig. 24 shows an exemplary back or proximal end 2400 of an elongated conductive body 2301 formed from a non-conductive fiber reinforced core with coaxial coating 2302, showing how the bundle 1901 of fibers, encapsulated in elongated insulating body 1900 may extend to the back end and showing how insulating layer 2300 and coating 2302 may be patterned at the back end to form, for example, a cutaway 2402 at which sensor electronics 112 or electrical contacts may be coupled to the sensor. Various additional layers such as one or more membrane layers as discussed herein may be formed over at least exposed portions of traces 2000 in windows 2102 and 2303 to form the working electrodes, in various embodiments.

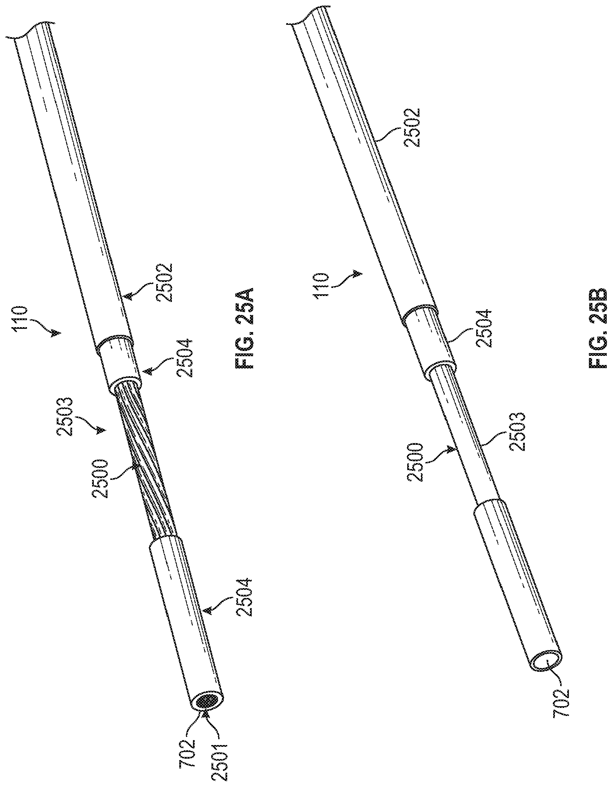

The fiber reinforced core examples described above in connection with Figs. 19-24 are merely illustrative. Fig. 25A shows another implementation of a fiber reinforced core for a flexible analyte sensor. In the example of Fig. 25A, core 702 is formed from a bundle 2501 of fibers (e.g., para-aramid synthetic fibers such as Kevlar^{®} fibers) with a conductive coating 2500 (e.g., a metal cladding such as a platinum or platinum-based cladding, or platinum sputtered coating) formed on the bundle. In this way, a window 2503 in a coaxial insulating layer 2504 that substantially encapsulates the metal clad core may allow a working electrode to be formed from a portion of the metal clad fiber core itself. As shown in Fig. 25A, a coaxial conductive layer 2502 (e.g., a silver or silver chloride electrode layer) may be disposed around at least a portion of insulating layer 2504 to provide a reference electrode.

It should be noted that the example of Fig. 25A in which a bundle of metal-clad non-conductive fibers is used as an elongated conductive body for a flexible analyte sensor is merely illustrative. As shown in the example of Fig. 25B, a non-conductive fiber reinforced core 702 for a flexible analyte sensor may be formed from a single fiber having a conductive coating 2500 and substantially surrounded by insulating layer 2504 (e.g., a polymer material include polyurethane and/or polyimide) and conductive layer 2502. In the example of Fig. 25B, a working electrode formed in window (e.g., cutaway region) 2503 of insulating layer 2504 may be sufficiently flexible that membrane coating procedures other than drip coating (e.g., vapor coating, etc.) may be used to deposit membrane 508 on the exposed portion of conductive coating 2500 in window 2503.

In any of the embodiments described herein, including the embodiments illustrated in Fig. 25A or FIG. 25B, the working electrode's sensitivity can be increased by increasing its surface area. For example, in one embodiment, the surface area of the working electrode is increased by using metal nanoparticles, such as platinum nanoparticles, or nanoparticles formed of other conductive materials. In one embodiment, the conductive nanoparticles may be mixed into a matrix that forms the conductive coating. In an alternative embodiment, the conductive nanoparticles can be bound (e.g., by covalent bonding) to the surface of the non-conductive fiber core. Because conductive nanoparticles can provide a larger surface area, a greater rate of oxidation of the measured species (e.g., hydrogen peroxide) may be achieved, thereby resulting in a sensor with a higher sensor sensitivity.

Fig. 26 shows an exemplary back or proximal end 2610 of an elongated conductive body formed from a non-conductive fiber reinforced core with a metal coating 2500, showing how the bundle 1901 of fibers, encapsulated in elongated insulating body 1900 may extend to the back end and showing how insulating layer 1900 may be patterned at the back end to form a cutout 2600 at which the metallized core is exposed for coupling of the working electrode to sensor electronics 112.

In embodiments in which core 702 is implemented as a fiber core, core 702 may include any suitable number of fibers (e.g., a single fiber of one or more braided or woven fibers) to generate a desired core diameter, stiffness, and/or longitudinal strength. For example, a single-fiber core may have a diameter of less than or equal to 12 microns in some embodiments. In some embodiments, metallic or polymer cores may be formed with relatively small diameters or major axes (e.g., a diameter or major axis of between 10 microns and 50 microns) to reduce the stiffness, flexural modulus, and/or buckling force of the core along with, or in addition to the selection of materials for the core.

In some embodiments, an in vivo portion of a flexible analyte sensor having a metallic, polymer, or fiber core by decreasing the interface area between the sensor and host tissue thereby reducing transduction of mechanical forces from tissue to interface. For example, the length of the in vivo portion may be between 1 mm and 5 mm in some embodiments.

In various embodiments, conductive structures such as electrodes and/or conductive traces (e.g., for coupling the electrodes to sensor electronics 112) may be formed on a non-conductive core using stenciling or jet printing procedures. Fig. 27 shows an example of a core 702 implemented as a polymer core that, as indicated by arrow 2700, may be provided with a patterned mask or stencil 2702 that substantially surrounds at least a portion of core 702 and that includes openings such as openings 2704 and 2706. A conductive material such as a metallic material (e.g., platinum or a platinum alloy) may be applied to the stencil 2702 within openings 2704 and 2706 and wiped (e.g., squeegeed) away such that the conductive material is formed only within openings 2704 and 2706. As indicated by arrow 2708, stencil 2702 may then be removed leaving the deposited conductive material on core 702 to form electrode 2710 (e.g., a working electrode or reference electrode for a flexible analyte sensor) and a conductive trace 2712 corresponding to the shape of openings 2704 and 2706). However, the process of Fig. 27 is merely illustrative. In some embodiments, conductive structures such as electrode 2710 and trace 2712 (e.g., implementations of electrode 704 and/or 706 and traces 708 and/or 710) may be printed on core 702 (e.g., using an aerosol jet printer in which a sheath gas flow is used to focus a stream of conductive ink droplets to provide high resolution conductive structures on core 702).

Although various implementations of flexible continuous analyte sensor 110 having a wire-type (e.g., metallic, polymer, conductive, non-conductive, and/or fiber) core have been described herein as examples, these examples are merely illustrative. In other embodiments, sensor 110 may be formed from a plurality of substantially planar layers.

For example, Fig. 28 illustrates a perspective view of an in vivo portion of a continuous analyte sensor 110 implemented using a plurality of planar layers 2800. As shown in Fig. 28, in some embodiments, sensor 110 may include a substantially planar insulating layer 2802, a substantially planar first electrode layer 2808 disposed on a first side of the insulating layer 2802 and a substantially planar second electrode layer 2806 disposed on an opposing second side of insulating layer 2802. In various embodiments, insulating layer 2802 may be an insulating polymer layer formed from a polyethylene, a polyimide and/or a state-change material (e.g., a temperature-dependent shape-memory polymer, a hydration-dependent shape-memory polymer, a chemical-dependent state-change material, or a field-dependent state-change material as discussed herein).

First electrode layer 2808 may be a formed, for example, from a metallic material such as platinum, platinum-iridium, gold, palladium, iridium, alloys thereof, graphite, carbon, or a conductive polymer. At least a portion of first electrode layer 2808 may form a working electrode for sensor 110 and may be disposed in a window 2811 in an additional insulating layer 2810 (e.g., an additional insulating polymer layer). A trace layer 2804 such as a silver or silver alloy trace layer may be disposed on the first side of insulating layer 2802 may extend along the surface of insulating layer 2802 between insulating layer 2802 and additional insulating layer 2810 and between insulating layer 2802 and first electrode layer 2808.

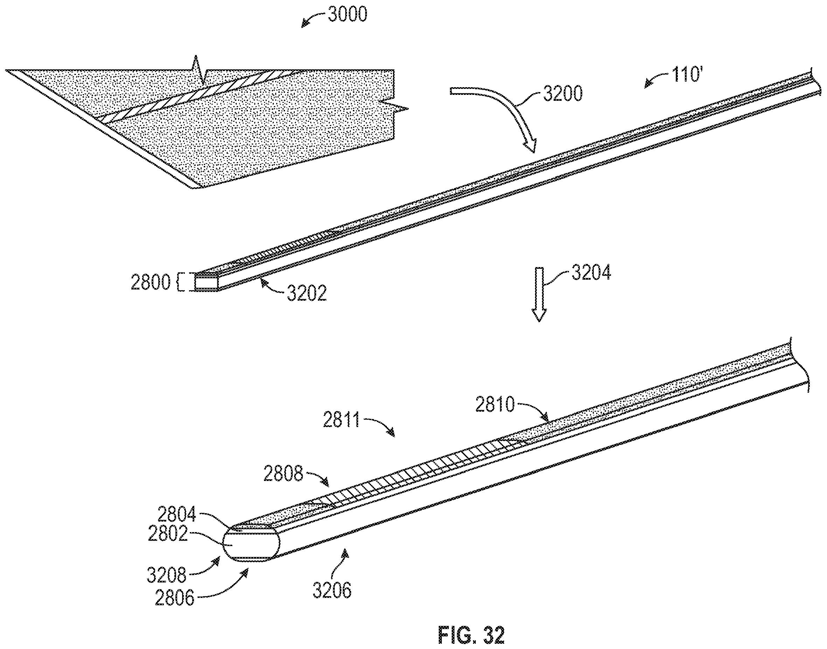

In this way, trace layer 2804 may provide a conductive coupling between first electrode layer 2808 (e.g., the working electrode) and sensor electronics 112 (not shown in Fig. 28) or electrical contacts. Second electrode layer 2806 may be formed, for example, from silver, silver chloride or other suitable electrode materials. Second electrode layer 2806 may form a reference electrode for sensor 110. One or more membrane layers for a membrane 508 may be formed on first electrode layer 2808 at least in window 2811 as described above in connection with, for example, Fig. 6B (e.g., conductive layer 610 of Fig. 6B may be an implementation of layer 2808 of Fig. 28 and/or Fig. 32).

As shown in Fig. 28, although each of layers 2800 is substantially planar (e.g., includes a substantially planar surface in contact with a substantially planar surface of at least one other layer), the outer edge 2820 may be rounded such that the cross-sectional profile 2822 of sensor 110 is substantially elliptical to provide twist and/or bend control as discussed herein.

Fig. 29 shows a back (e.g., proximal) end portion 2900 of sensor 110 in the implementation of Fig. 28. As shown in Fig. 29, second electrode layer 2806 and insulating layer 2810 may include features such a cutout 2906 and additional window 2909 configured for coupling to sensor electronics 112 (not shown). Edge portions 2904 of sensor 110, in implementations in which sensor 110 includes a plurality of substantially planar layer 2800, maybe coated or otherwise treated (e.g., with an insulating coating) to prevent electrical crosstalk across layers in some embodiments.

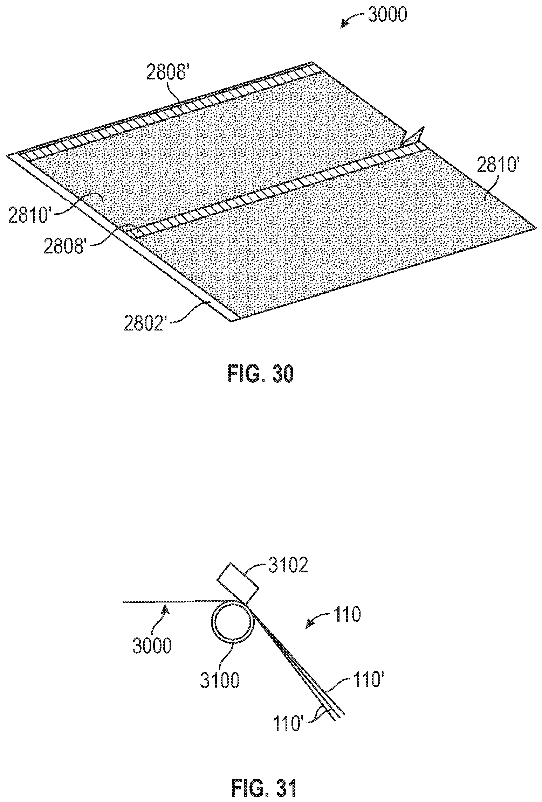

Sensors 110 that are implemented with a plurality of substantially planar layers 2800 may be formed, for example, by patterning all traces and electrodes in a large sheet format and then singulating the large sheet including all the desired layers into individual sensors. Fig. 30 shows an example of a planar sheet having a plurality of planar layers from which singulated sensors 110 can be formed in one embodiment.

As shown in Fig. 30, a planar sheet of insulating material 2802' (e.g., an insulating polymer sheet formed from a polyethylene, a polyimide, other polymers, and/or a state-change material such as a temperature-dependent shape-memory polymer, a hydration-dependent shape-memory polymer, a chemical/biological-dependent state-change material, or a field-dependent state-change material as discussed herein) may be provided. Sheet 2802' may be provided, and may be coated with a planar sheet of trace material (e.g., silver) 2808'that substantially covers a first side of sheet 28C>2'. Trace material 2808' may be deposited using any of various metal deposition techniques such as sputtering, screen printing, or other deposition techniques as discussed herein.

A sheet 2810' may be deposited in a pattern that covers the contiguous layer of conductive (e.g., trace) material 2808', while leaving a pre-defined surface area (e.g., strips as shown in FIG. 30) of the conductive material 2808' uncovered for deposition electrochemical membrane materials and/or electrode material deposition. Sheet 2810' may be deposited using, for example, radio frequency (RF) sputtering of insulating material (e.g., silicon dioxide (SiO2) or a silicon nitride (SiNx)) onto predefined areas of the planar sheet of electrode material or screen printing of insulating material (e.g., polyurethane dispersions in an organic solvent, a thermoplastic silicone polycarbonate urethane, or polydimethylsiloxane (PDMS)) onto the planar trace layer. Sheet 2810' may be deposited with gaps that form the conductive strips of material shown in FIG. 30 that leave the desired portions of trace layer 2808' uncovered. In this way, laser ablation or laser skiving of insulating material to define windows for electrode strips can be avoided, thereby reducing the time and cost of production of flexible analyte sensors. However, this is merely illustrative. In some embodiments, full planar sheets of insulating material may be deposited on the planar sheet of trace material and patterned using laser skiving or other suitable techniques.

Sheet 2802' may be provided with a planar sheet of reference electrode material (e.g., a planar sheet of silver or silver chloride) on a second side (e.g., opposite to the side on which the trace layer is formed, not shown in Fig. 30) by providing a sheet of the reference electrode material on to sheet 2802' or by providing sheet 2802' onto a previously-formed sheet of reference electrode material. Various additional materials such as insulating materials, electrode materials, or membrane materials as described herein may be deposited, laminated, printed, wiped, or otherwise provided to form a sheet structure 3000 from which a plurality of sensors can be singulated.

In one suitable example, sheet 3000 may be formed, in part, by providing a sheet of silver trace material 2808', and screen printing (or otherwise depositing) insulating sheets 2810' to cover portions of the sheet of silver trace material while leaving other portions uncovered. In another suitable example, sheet 3000 may be formed, in part, by providing a sheet of silver trace material, attaching one or more strips 2808' of platinum or platinum alloy foil to the sheet of silver trace material while the sheet of silver trace material is wet, and screen printing (or otherwise depositing) insulating sheets 2810' to cover remaining portions of the sheet of silver trace material. In another suitable example, sheet 3000 may be formed, in part, by providing a sheet of titanium or titanium alloy trace material, sputtering a platinum or platinum alloy material in strips 2808' on the sheet of titanium or titanium alloy trace material, and screen printing (or otherwise depositing) insulating sheets 2810' to cover remaining portions of the sheet of silver trace material.

Strips of membrane materials as described in connection with Fig. 6B may be printed or otherwise deposited along strips 2808' so that membrane material is formed only on the working electrode of each sensor and remaining portions of the singulated sensors are substantially free of membrane materials. In this way, the cost of membrane materials for each sensor, in comparison with, for example, dip coating of wire-core type sensors may be substantially reduced.

Fig. 31 shows an example of singulating equipment that may be used to singulate individual sensors 110. As shown in Fig. 31, sheet 3000 may be fed over a roller 3100 into the path of cutting apparatus 3102 (e.g., a block of evenly spaced blades such as metallic blades, a linear array of laser cutters, or a linear array of water-jet cutters) that cut sheet 3000 into individual sensor strips 110', each forming a reel containing a plurality of non-singulated sensors 110. Each sensor strip 110' may then be cut and/or further processed (e.g., rounded and/or edge coated) to singulate the reel to form individual sensors 110. Alternatively, cutting apparatus 3102 may be configured to slice sheet 3000 in two orthogonal directions to singulate individual sensors from sheet 3000 at cutting apparatus 3102.

Fig. 32 shows a schematic illustration of a process for forming a flexible analyte sensor 110 from a sheet 3000. As indicated by arrow 3200, sheet 3000 may be cut (e.g., using a apparatus 3102) to form a plurality of singulated sensor structures such as singulated sensor structure 110'. Singulated sensor structure 110' may have a plurality of substantially planar layers 2800 that have a substantially rectangular cross-sectional profile and substantially flat edges 3202. However, as indicated by arrow 3204, each singulated sensor structure may be further processed (e.g., in an additional thermal shaping process such as hot pressing) to round the edges of singulated sensor structure 110' to provide a singulated sensor 110 having a rounded sidewall 3206 and a substantially elliptical cross-sectional profile 3208. This hot pressing can be more economically performed prior to the cutting of singulated string of configured wire. However, this is merely illustrative. In some implementations, cutting apparatus 3102 may be configured to singulate and round the edges of sensors 110 in a common cutting operation or substantially rectangular sensors may be provided.

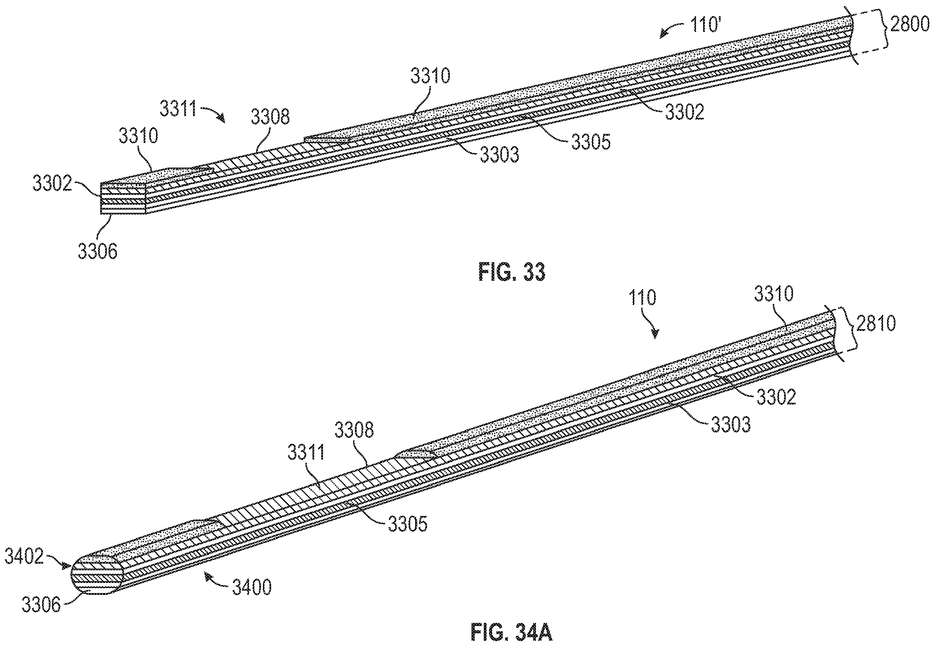

The plurality of substantially planar layers 2800 shown in Fig. 32 are the same as those described above in connection with Fig. 28. However, this arrangement of layers is merely illustrative and other arrangements of layers are contemplated. For example, various configurations for sensors 110 having a plurality of planar layers arranged to provide multi-analyte sensing (e.g., using multiple working electrodes or specialized arrangements of membrane layers) and/or to provide a counter electrode are contemplated. For example, as shown in Fig. 33, a singulated sensor structure 110' that has been cut from a sheet of planar sensor layers may include a counter electrode 3305.

In the example implementation of Fig. 33, singulated sensor structure 110' includes a counter electrode 3305 disposed between a first substantially planar insulating layer 3302 on a first side of counter electrode 3305 and a second substantially planar insulating layer 3303 on a second side of counter electrode 3305. A substantially planar first electrode layer 3308 (e.g., a platinum or platinum alloy electrode layer) may be formed on first substantially planar insulating layer 3302. A substantially planar second electrode layer 3306 (e.g., a silver or silver chloride electrode layer) may be formed on second substantially planar insulating layer 3303. An additional insulating layer 3310 may be formed on the substantially planar first electrode layer 3308. A window 3311 may be formed in substantially planar insulating layer 3310 to expose a portion of electrode layer 3308 to form a working electrode for sensor 110. Membrane 508 (not explicitly shown in Fig. 33) may be formed over at least the exposed portion of layer 3308 in window 3311. For example, the exposed portion of layer 3308 may be an implementation of conductive layer 610 of Fig. 6B.

Window 3311 may be formed by depositing sheets of insulating material separated by spaces corresponding to the width of window 3311 on a sheet of electrode material or by substantially covering the sheet of electrode material with a continuous sheet of insulating material and removing strips of insulating material, each strip having a width corresponding to the width of window 3311 from the sheet of electrode material as described above in connection with, for example, Figs 30-32.

As shown in Fig. 33, singulated sensor structure 110' may have a plurality of substantially planar layers 2800 that have a substantially rectangular cross-sectional profile. However, as shown in Fig. 34A, each singulated sensor structure may be further processed (e.g., in an additional cutting and/or grinding process) to round the edges singulated sensor structure 110' to provide a singulated sensor 110 having a rounded sidewall 3400 and a substantially elliptical cross-sectional profile 3402.

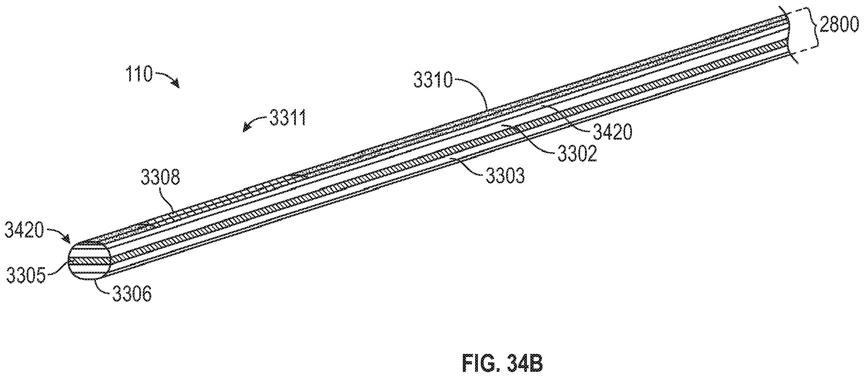

As shown in Fig. 34A, electrode layer 3308 may form a portion of a working electrode and may extend along the length of sensor 110 to conductively couple the working electrode to sensor electronics 112 (not shown in Fig. 34). However, this is merely illustrative. As shown in Fig. 34B, the plurality of planar layers 2800 of sensor 110 may include an additional trace layer 3420 (e.g., a silver or silver alloy trace layer) disposed between insulating layer 3302 and electrode layer 3308. As shown, trace layer 3420 may extend along the length of sensor 110 to provide a conductive coupling between a localized electrode layer 3308 within window 3311 and sensor electronics 112 (not shown in Fig. 34B).

The plurality of planar layers 2800 in the various implementations described in connection with Figs. 28, 32, 34A and/or 34B may, in aggregate, form an elongated conductive body for a flexible analyte sensor 110. The plurality of planar layers 2800 may, in aggregate, have a Young's modulus and/or a flexural modulus of, for example, less than 147 GPa, less than 1.5 GPa, less than 1GPa, less than 1 MPa, or less than 1kPa. The plurality of planar layers 2800 may, in aggregate, have a buckling force of, for example, less than 0.25 N, less than 0.02 N, less than 0.01 N, less than 0.001 N, or less or greater than the weight of the plurality of planar layers 2800 under gravity (as examples). The Young's modulus, the flexural modulus, and/or the buckling force of the plurality of planar layers may remain substantially unchanged in ex vivo and in vivo configurations or one or more of substantially planar insulating layers 2800 such as layer 2802 (Fig. 28), or 3303, 3302, and/or 3310 (Figs. 34A/34B) may be a state-change material that becomes more flexible in vivo than ex vivo due, for example, to a change in temperature, a change in hydration, a chemical and/or biological reaction with host tissue, or a change in an applied electric field. For example, upon insertion into a patient's skin, the Young's modulus and/or the flexural modulus of the aggregate of the plurality of substantially planar layers or the aggregate of a wire-type core and additional layers formed thereon may fall from, for example, above one GPa to below one GPa, below 1MPa, or below 1kPa in various implementations. Upon insertion into a patient's skin, the buckling force of the aggregate of the plurality of substantially planar layers or the aggregate of a wire-type core and additional layers formed thereon may fall from, for example, greater than the weight of the plurality of planar layers 2800 under gravity to less than the weight of the plurality of planar layers 2800 under gravity or from greater than 0.01N to less than 0.01 N. In this way, a freestanding sensor 1 10 can be provided ex vivo that transforms into a non-freestanding sensor in vivo.

Although the examples of Figs. 28 and 34 show a single working electrode, planar sheet embodiments may facilitate formation of multiple working electrodes at various locations on the sensor (e.g., for spatial signal averaging and/or for multi analyte sensing) and allow for the fabrication of multi-analyte sensor such as glucose and lactate dual analyte sensors.

Various benefits such as reduced background and reduced noise may be provided by flexible analyte sensors as described herein as indicated, respectively, by graphs 3500 and 3600 of Fig. 35. For example, a flexible core wire with a low elastic modulus as described herein may be, compared to, for example, a tantalum core wire, more amenable to long-term transcutaneous use with low levels of, for example, FBGC encapsulation. Furthermore, the use of a cleanroom sputter coated deposition of working electrode material such as platinum may provide improved electrochemical performance in terms of reduced background signal and absolute noise as indicated by the respective comparison graphs 3500 and 3600.

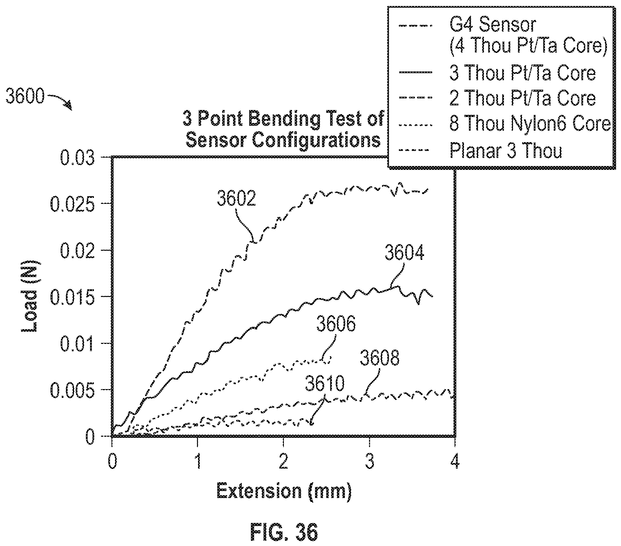

Fig. 36 shows a graph of exemplary 3-point bending loads for five exemplary sensor implementations, which may provide another measure of the flexibility of a flexible analyte sensor. In particular, in the example of Fig. 36, a first curve 3602 corresponds to a load in Newtons (N) as a function of extension in millimeters for a sensor having a tantalum core with a diameter of 4 thousandths of an inch. Curve 3604 corresponds to a load in Newtons (N) as a function of extension in millimeters for a sensor having a tantalum core with a diameter of 3 thousandths of an inch. Curve 3606 corresponds to a load in Newtons (N) as a function of extension in millimeters for a sensor having a tantalum core with a diameter of 2 thousandths of an inch. Curve 3608 corresponds to a load in Newtons (N) as a function of extension in millimeters for a sensor having a nylon (e.g., Nylon6) core with a diameter of 8 thousandths of an inch. Curve 3610 corresponds to a load in Newtons (N) as a function of extension in millimeters for a sensor having a plurality of substantially planar layers (with a polymeric substrate) with an aggregate thickness of 3 thousandths of an inch. Curves 3602, 3604, 3606, 3608, and 3610 may represent bend test results over a span of 0.5 inches at a speed of one inch/min.

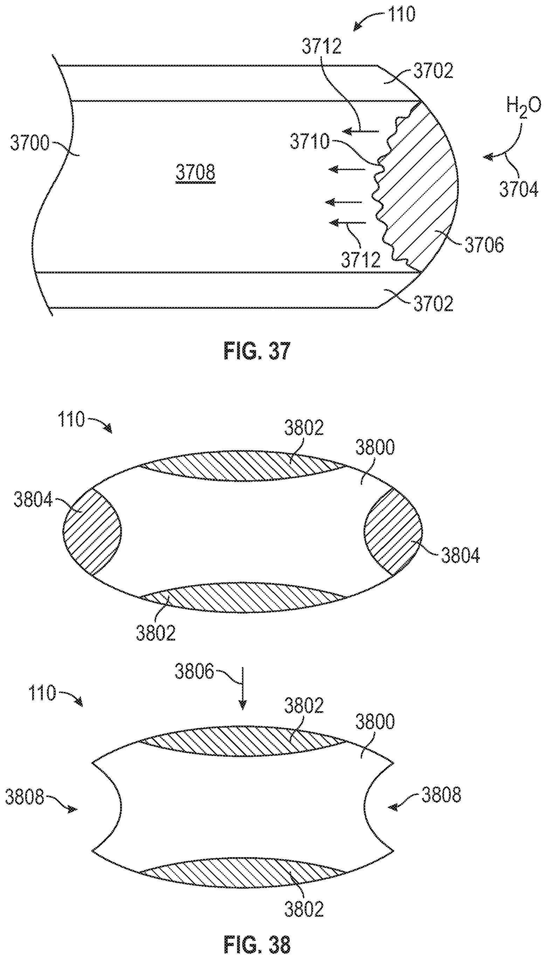

As described herein, in some embodiments, a flexible analyte sensor may be formed, in part, from a material that changes state from substantially freestanding to substantially non-freestanding upon implantation. Fig. 37 shows an example of a sensor 110 having a plurality of substantially planar layers such as layers 3700 and 3702 (e.g., layers corresponding to one or more of layers 2800 of Figs. 28 or 36). In the example of Fig. 37, sensor 110 includes a hydrophilic layer 3700 (e.g., a hydrophilic polymer as discussed herein) configured to absorb fluid such as water (H2O) as indicated by arrow 3704. In the configuration illustrated in Fig. 37, a portion 3706 of layer 3700 has absorbed fluid from the host's tissue. A hydration interface 3710 between wetted portion 3706 and un-wetted portion 3708 may extend into an interior portion of layer 3700, as indicated by arrows 3712, as layer 3700 absorbs the host fluid. Upon hydration, the Young's modulus and/or the flexural modulus of layer 3700 may be reduced from, for example above one GPa to below 1GPa, below 1MPa, or below 1kPa in various implementations and/or the buckling force of layer 3700 may be reduced from, for example, greater than 0.01N to less than 0.01 N or from greater than the weight of the plurality of planar layers 2800 under gravity to less than the weight of the plurality of planar layers 2800 under gravity. Layer 3700 may be configured become hydrated within hours, minutes or seconds of in vivo placement (as examples). The length of time for a state change from freestanding to non-freestanding may be determined such that coating and/or other manufacturing operations that involve fluids do not cause an undesirable state change.

Fig. 38 shows an example of a transition of a sensor 110 from a freestanding sensor to a non-freestanding due to a chemical and/or biological reaction. In the example of Fig. 38, a non-conductive (e.g., polymer) core 3800 with conductive traces 3802 (e.g., an implementation of non-conductive core 702 and conductive traces 1000, 1600, 1800, or 2000 as described herein) may include one or more portions 3804 that chemically and/or biologically reacts with tissue and/or fluid of the host in vivo. For example, portions 3804 may be formed from a dissolvable material such as processed collagen, silk, hair, and/or other natural or synthetic materials that the host body can break down. As shown in Fig. 38, and as indicated by arrow 3806, portions 3804 may be dissolved in vivo such that sensor 110 loses material from recesses 3808, thereby reducing the rigidity of the sensor.

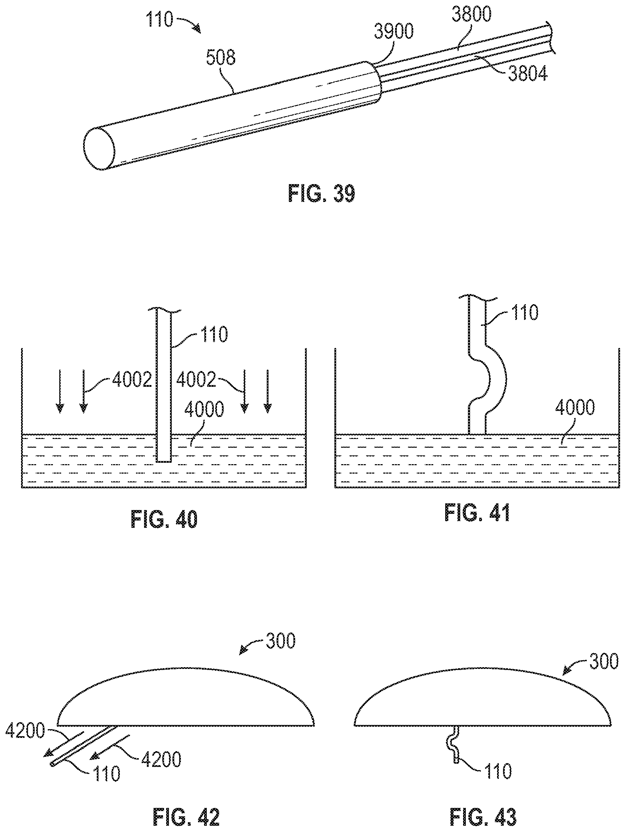

As shown in Fig. 39, portion(s) 3804 may extend beyond an edge 3900 of the region of sensor 110 on which membrane 508 is formed so that portion(s) 3804 can interact and interface directly with the host tissue. Portion(s) 3804 may also extend underneath membrane 508 and may be dissolved from underneath membrane 508, thereby reducing the rigidity of portions of sensor 110 on which membrane 508 is disposed.

Fig. 40 shows an example of a portion of a sensor 110 having a field-dependent stiffness that can be stiffened by application of an electromagnetic field. As shown in Fig. 40, during dip coating operations in which a sensor 110 is dipped into a membrane fluid 4000, an electric or magnetic field 4002 may be applied in the dipping direction to enhance the stiffness of sensor 110 in that direction.

As shown in Fig. 41, without the application of field 4002, the stiffness of sensor 110 may be low enough that sensor 110 may be deformed due to the surface tension of fluid 4000. Accordingly, in some embodiments, sensor 110 may be provided with electro- or magneto-alignable materials that stiffen in a desired direction upon application of a predetermined field.

In the example of Figs. 40 and 41, field 4002 is an externally applied field generated, for example, by dip coating equipment during dip coating. In other embodiments, the stiffness of sensor 110 may be modified and/or controlled by sensor electronics 112 when the sensor integrated into a sensor system such sensor system 101 (Fig. 1). For example, as shown in Fig. 42, sensor electronics within housing 300 may generate a field 4200 that stiffens sensor 110 prior to insertion into the patient's skin. Upon removal of field 4200, sensor 110 may transition from the freestanding sensor of Fig. 42 to the non-freestanding sensor of Fig. 43, or vice versa.

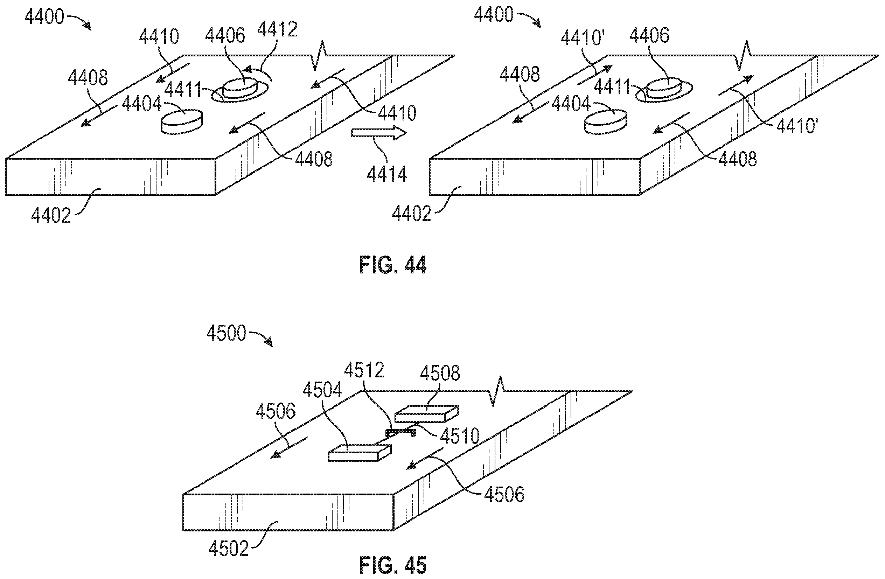

Figs. 44 and 45 show exemplary configurations of electromagnetic field generating components that may be used to control the stiffness of sensor 110 from, for example, within housing 300. In some embodiments, these electromagnetic field generating components may be part of sensor electronics 112 that be part of an on-skin assembly (e.g., on the sensor electronics housing) adhered to the patient's skin.

In the example of Fig. 44, magnetic field generating components 4400 that may be included in housing 300 include a pair of magnetic field generating components (e.g., magnets) 4404 and 4406 on a substrate 4402. As shown, coaligned magnetic fields 4408 and 4410, generated respectively by magnets 4404 and 4406, may generate an overall magnetic field that extends from housing 300 to stiffen sensor 110. Magnets 4404 and 4406 may be provided in an aligned arrangement as shown in Fig. 44 prior to insertion of sensor 110 into the patient's skin. As indicated by arrow 4414, upon rotation of, for example, magnet 4406 in a direction 4412 (e.g., by 180 degrees on a rotating platform 4411), the direction of magnetic field 4410 of magnet 4406 may be reversed to form a counter field 4410' that cancels field 4408 of magnet 4404. In this way, an applied magnetic field that stiffens sensor 110 (e.g., for insertion or other pre-insertion processes) can be removed to allow the sensor to become non-freestanding in vivo. Rotation of magnet 4406 may be performed automatically or manually by a user (e.g., by actuating a button coupled to platform 4411 and accessible on an outer surface of housing 300).

In the example of Fig. 45, electric field generating components 4500 that may be included in housing 300 include an electric field generator 4504 on a substrate 4502. As shown, an electric field 4506 may be generated by field generator 4504 using power provided by power source 4508 (e.g., a battery) to stiffen sensor 110. Field generator 4504 and power source 4508 may be provided in a powered arrangement prior to insertion of sensor 110 into the patient's skin in which power is provided to field generator 4505 along a conductive trace 4510 on the substrate. A mechanism 4520 (e.g., an actuatable circuit breaker) operable to break trace 4510 and thereby permanently depower field generator 4505 may be provided. In this way, an applied electric field that stiffens sensor 110 (e.g., for insertion or other pre-insertion processes) can be removed to allow the sensor to become non-freestanding in vivo. Actuation of mechanism 4512 may be performed automatically or manually by a user (e.g., by actuating a button coupled to platform 4411 and accessible on an outer surface of housing 300).

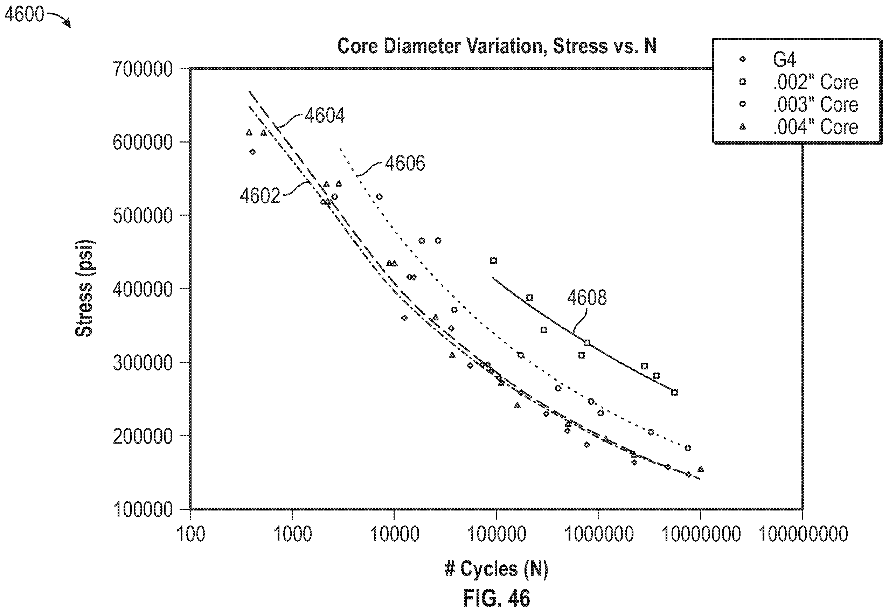

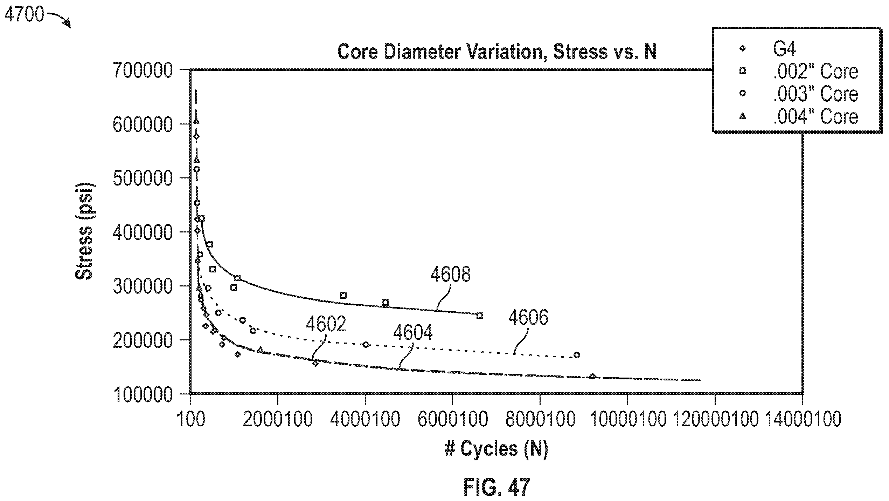

FIGS. 46 and 47 respectively show linear-scale and log-scale graphs 4600 and 4700 of fatigue characteristics for various exemplary flexible analyte sensors. In particular, fatigue characteristic curves 4602, 4604, 4606, and 4608 respectively are shown for a sensor having a tantalum wire core with a 0.007" diameter, a 0.004" diameter, a 0.003" inch diameter, and a 0.002" diameter. As shown, of the illustrated wire core examples, the 0.002" diameter core wire has the longest fatigue life (i.e. highest fatigue endurance limit), followed by the 0.003". The 0.004" core wire fatigue characteristics can be seen to be relatively similar to the 0.007" diameter core.

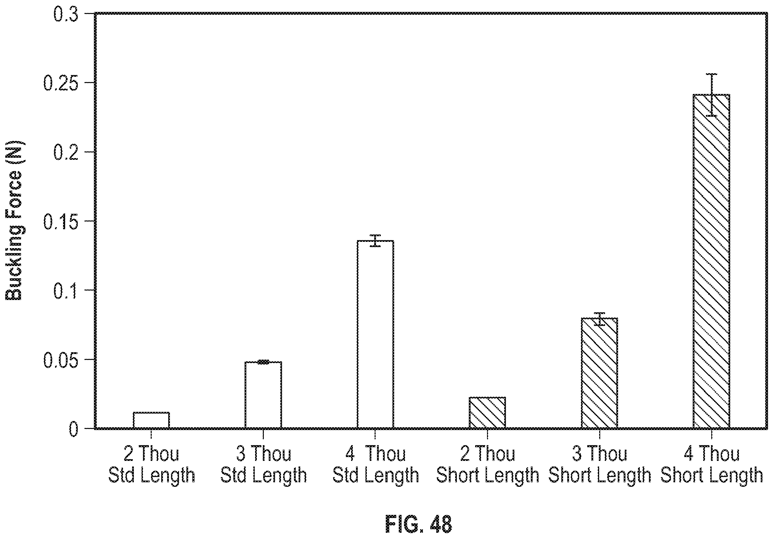

FIG. 48 shows a graph illustrating buckling forces for various exemplary flexible analyte sensors. The values of the buckling forces in the graph of FIG. 48 are reflected in the table below:

| Standard length (0,402" Exposed length) | Buckling Force (N) |
|---|---|
| 2 Thou | 0.0110 |
| 3 Thou | 0.0481 |
| 4 Thou | 0.136 |

| Short Length (0.307" Exposed Length) | Buckling Force (N) |
|---|---|
| 2 Thou | 0.0222 |
| 3 Thou | 0.0791 |
| 4 Thou | 0.241 |

In particular, for a wire-core sensor (e.g., a metal core sensor as described herein in accordance with some aspects) having a length of 0.402" (sometimes referred to herein for convenience as a "Standard Length" or "Std Length" in FIG. 48), the buckling force may be 0.0110 N, 0.0481 N, or 0.136 N respectively for a core diameter (or semi-major axis) of 2, 3, or 4 thousandths of an inch. For a wire-core sensor having a length of 0.307" (sometimes referred to herein as a "Short Length" in FIG. 48), the buckling force may be 0.0222 N, 0.0791 N, or 0.241 N respectively for a core diameter (or semi-major axis) of 2, 3, or 4 thousandths of an inch. The lengths in FIG. 48 and the table above may correspond to lengths of a portion of a continuous analyte sensor that extends from a sensor electronics housing in various implementations. The buckling force of a polymer core sensor or a sensor formed from a plurality of planar layers as described herein (e.g., for a portion of a sensor that extends from a sensor electronics housing and/or having a length of between 0.2" and 0.5") may be substantially less than the buckling forces of the wire-core sensors of FIG. 48 and the table above. For example, buckling force of a polymer core sensor or a sensor formed from a plurality of planar layers may be less than 0.25 N, less than 0.02 N, less than 0.01 N or less than 0.001N in various implementations. Moreover, the buckling force may decrease in vivo relative to the buckling force ex vivo as described herein in various implementations.

Various examples have been described herein of state-change continuous analyte sensors having an elongated conductive body that transforms from a freestanding elongated conductive body ex vivo and a non-free standing elongated conductive body due to temperature, hydration, biological/chemical, or field conditions in the environment of the sensor. Although temperature, hydration, biological/chemical, or field dependent state-change sensors are sometimes discussed herein independently, it should be appreciated that the materials and arrangements of the various layers of the sensors described herein can provide a combination of any or all of the temperature, hydration, biological/chemical, and/or field dependent state-change properties described herein in any suitable combination.

It should also be appreciated that, particularly in sensor implementations with a plurality of planar layers formed from a singulated sheet, a sensor having a relatively low modulus ex vivo and in vivo (e.g., a sensor formed from a plurality of substantially planar layers that, in aggregate, have a Young's modulus of less than 1GPa, less than 1MPa, or less than 1kPa, a flexural modules of less than 1GPa, less than 1MPa, or less than 1kPa, and/or a buckling force that is less than 0.25 N, less than 0.02 N, less than 0.01 N, less than 0.001 N, or less than the weight of the sensor) such as a permanently non-freestanding sensor may be provided.

For many plastics, the compressive stiffness is higher, than the tensile stiffness, and so the neutral surface (to be right in the middle of the beam) shifts upward (e.g., a smaller net cross-sectional area in compression is stiff enough to resist the larger cross-sectional area under tension). For many polymeric materials the compressive stiffness and the tensile stiffness are not the same, and therefore Young's modulus and flexural modulus will not be same. In some scenarios (e.g., for a plastic part that is loaded mostly in tension) the mechanical properties may be characterized by the Young's modulus. However, for a sensor that may be loaded mostly in bending, of that may be subjected to a wide mixture of stress directions (e.g., in the most-highly loaded regions), the flexural modulus may be a more accurate indicator of the mechanical properties thereof than the Young's modulus.

In embodiments in which the sensor is a non-freestanding sensor ex vivo, sensor deployment mechanisms may include specialized components for deployment of a non-freestanding sensor. For example, with a freestanding sensor, the sensor may be disposed within a lumen of an inserter needle that is inserted into the skin of the host along with the sensor and withdrawn, leaving the sensor embedded in the skin. For a non-freestanding sensor, in some embodiments, a folding-unfolding mechanism or a coiling-uncoiling mechanism may be provided within the needle to control the position of the sensor and, if desired, to leverage internal void space inside the lumen of the cylindrical needle. In embodiments, in which the sensor is too soft for needle/cannula delivery, a preconnected c-needle deployment system may be used.

### EXEMPLARY SENSORS AND SENSOR SYSTEMS

Sensor Embodiment 1: A continuous analyte sensor configured for in vivo use, the continuous analyte sensor comprising: an elongated core; a working electrode disposed on the elongated core; and a membrane covering at least a portion of the working electrode, wherein the membrane comprises an enzyme layer, and wherein a portion of the continuous analyte sensor is configured to extend from a sensor electronics housing and to have a buckling force of less than 0.25 Newtons (N).

Sensor Embodiment 2: The continuous analyte sensor of Sensor Embodiment 1, wherein the elongated core comprises an elongated conductive core having a Young's modulus of less than one hundred forty-seven GPa.

Sensor Embodiment 3: The continuous analyte sensor of Sensor Embodiment 2, further comprising a layer of conductive material covering at least a portion of the elongated conductive core.

Sensor Embodiment 4: The continuous analyte sensor of Sensor Embodiment 3, wherein the elongated conductive core comprises at least one material selected from the group consisting of copper, gold, Magnesium, Silver, Tin, Titanium, a Titanium alloy, and Zinc.

Sensor Embodiment 5: The continuous analyte sensor of Sensor Embodiment 4, wherein the conductive material comprises a conductive material selected from the group consisting of platinum, platinum-iridium, gold, palladium, iridium, alloys thereof, graphite, carbon, and a conductive polymer.

Sensor Embodiment 6: The continuous analyte sensor of Sensor Embodiment 3, further comprising a layer of insulating material coving at least a portion of the layer of conductive material, wherein the working electrode is formed, in part, by a window in the layer of insulating material that exposes an electrode portion of the layer of conductive material.

Sensor Embodiment 7: The continuous analyte sensor of Sensor Embodiment 6, further comprising an additional conductive layer covering at least a portion of the layer of insulating material, the additional conductive layer comprising a reference electrode.

Sensor Embodiment 8: The continuous analyte sensor of any one of Sensor Embodiments 1-7, wherein the elongated core comprises an elongated polymer core and wherein the portion of the elongated core has a flexural modulus of less than one and a half GPa.

Sensor Embodiment 9: The continuous analyte sensor of Sensor Embodiment 8, further comprising at least one conductive trace that runs along a length of the elongated polymer core, wherein a portion of the conductive trace comprises the working electrode.

Sensor Embodiment 10: The continuous analyte sensor of Sensor Embodiment 8, wherein the elongated polymer core comprises an elongated elliptical polymer core and wherein at least one conductive trace runs along the elongated elliptical polymer core.

Sensor Embodiment 11: The continuous analyte sensor of Sensor Embodiment 10, wherein the working electrode comprises a portion of the at least one conductive trace.

Sensor Embodiment 12: The continuous analyte sensor of The continuous analyte sensor of any one of Sensor Embodiments 1-11, wherein the elongated core comprises an elongated fiber core.

Sensor Embodiment 13: The continuous analyte sensor of Sensor Embodiment 12, wherein the elongated core further comprises: an elongated insulating body substantially surrounding the elongated fiber core; and at least one conductive trace that runs along the elongated insulating body.

Sensor Embodiment 14: The continuous analyte sensor of Sensor Embodiment 12, wherein the elongated fiber core comprises one or more Kevlar fibers.

Sensor Embodiment 15: The continuous analyte sensor of Sensor Embodiment 14, wherein the elongated core further comprises a conductive coating on the one or more Kevlar fibers, and wherein a portion of the one or more Kevlar fibers having the conductive coating forms the working electrode.

Sensor System Embodiment 16: A continuous analyte sensor system comprising the continuous analyte sensor of any one of Sensor Embodiments 1-15 and sensor electronics configured to process sensor signals from the continuous analyte sensor, wherein the sensor electronics are disposed in the sensor electronics housing, and wherein the sensor electronics housing is configured to attach to the exterior of a patient's skin.

Sensor Embodiment 17: The continuous analyte sensor of any one of Sensor Embodiments 1-15, wherein the portion of the elongated core has a buckling force of less than 0.02 N.

Sensor Embodiment 18: The continuous analyte sensor of Sensor Embodiment 17, wherein the portion of the elongated core has a flexural modulus between 0.1 kPa and 300 kPa.

Sensor Embodiment 19: A continuous analyte sensor configured for in vivo use, the continuous analyte sensor comprising: an elongated conductive body comprising a working electrode, wherein the elongated conductive body comprises a plurality of substantially planar layers, and wherein a portion of the plurality of substantially planar layers that is configured to extend from a housing of a continuous analyte sensor system has, in aggregate, a buckling force of less than 0.25 Newtons (N); and a membrane covering at least a portion of the working electrode, wherein the membrane comprises an enzyme layer.

Sensor Embodiment 20: The continuous analyte sensor of Sensor Embodiment 19, wherein the plurality of substantially planar layers comprise: an insulating polymer layer; a first electrode layer disposed on a first side of the insulating polymer layer; and a second electrode layer disposed on an opposing second side of the insulating polymer layer, wherein the working electrode comprises a portion of the first electrode layer and wherein the second electrode layer comprises a reference electrode.

Sensor Embodiment 21: The continuous analyte sensor of Sensor Embodiment 20, further comprising an additional insulating polymer layer formed on the first electrode layer, wherein the additional insulating polymer layer comprises a window that defines the working electrode.

Sensor Embodiment 22: The continuous analyte sensor of Sensor Embodiment 21, wherein the membrane is disposed on the first electrode layer within the window in the additional insulating polymer layer, and wherein the additional insulating polymer layer is substantially free of the membrane outside of the windows

Sensor Embodiment 23: The continuous analyte sensor of Sensor Embodiment 21, wherein the plurality of substantially planar layers, in cross-section, comprises a substantially elliptical outer surface.

Sensor Embodiment 24: The continuous analyte sensor of Sensor Embodiment 21, wherein the insulating polymer layer comprises first and second insulating polymer layers and wherein the plurality of substantially planar layers further comprises a counter electrode layer disposed between the first and second insulating polymer layers.

Sensor Embodiment 25: The continuous analyte sensor of Sensor Embodiment 20, wherein the plurality of substantially planar layers further comprises a conductive trace layer disposed between the first electrode layer and the insulating polymer layer.

Sensor Embodiment 26: The continuous analyte sensor of Sensor Embodiment 25, wherein the plurality of substantially planar layers further comprises an additional insulating polymer layer formed on the conductive trace layer, and wherein the additional insulating polymer layer comprises a window that defines the working electrode.

Sensor Embodiment 27: The continuous analyte sensor of any one of Sensor Embodiments 19-26, wherein additional portions of the elongated conductive body are substantially free of the membrane.

Sensor Embodiment 28: A continuous analyte sensor configured for in vivo use, the continuous analyte sensor comprising: an elongated conductive body comprising a working electrode, wherein the elongated conductive body is configured to be a freestanding elongated conductive body ex vivo and a non-freestanding elongated conductive body in vivo; and a membrane covering at least a portion of the working electrode, wherein the membrane comprises an enzyme layer.

Sensor Embodiment 29: The continuous analyte sensor of Sensor Embodiment 28, wherein the elongated conductive body is configured to transform from the freestanding elongated conductive body to the non-freestanding elongated conductive body responsive to contact between at least a portion of the elongated conductive body and tissue of a patient.

Sensor Embodiment 30: The continuous analyte sensor of any one of Sensor Embodiments 28-29, wherein the elongated conductive body is configured to transform from the freestanding elongated conductive body to the non-freestanding elongated conductive body at a transition temperature of between seventy-eight and one-hundred degrees Fahrenheit.

Sensor Embodiment 31: The continuous analyte sensor of any one of Sensor Embodiments 28-30, wherein the elongated conductive body is configured to transform from the freestanding elongated conductive body to the non-freestanding elongated conductive body responsive to absorption of a fluid from patient tissue by at least a portion of the elongated conductive body.

Sensor Embodiment 32: The continuous analyte sensor of any one of Sensor Embodiments 28-31, wherein the elongated conductive body is configured to transform from the freestanding elongated conductive body to the non-freestanding elongated conductive body responsive to a chemical reaction between a fluid from patient tissue and at least a portion of the elongated conductive body.

Sensor Embodiment 33: The continuous analyte sensor of any one of Sensor Embodiments 28-32, wherein the elongated conductive body is configured to transform from the freestanding elongated conductive body to the non-freestanding elongated conductive body responsive to an electromagnetic field generated by sensor electronics for the continuous analyte sensor.

Sensor Embodiment 34: The continuous analyte sensor of any one of Sensor Embodiments 28-33, wherein the elongated conductive body comprises an elongated conductive core.

Sensor Embodiment 35: The continuous analyte sensor of any one of Sensor Embodiments 28-34, wherein the elongated conductive body comprises an elongated polymeric core.

Sensor Embodiment 36: The continuous analyte sensor of any one of Sensor Embodiments 28-35, wherein the elongated conductive body comprises a plurality of substantially planar layers.

Sensor System Embodiment 37: A continuous analyte sensor system comprising the continuous analyte sensor of any one of Sensor Embodiments 28-36 and sensor electronics configured to process sensor signals from the continuous analyte sensor, wherein the sensor electronics are disposed in a housing configured to attach to the exterior of a patient's skin.

Sensor System Embodiment 38: The continuous analyte sensor system of Sensor Embodiment 37, wherein, in vivo, the elongated conductive body has a buckling force of less than 0.01 N.

Sensor Embodiment 39: The continuous analyte sensor of any one of Sensor Embodiments 28-38, wherein the non-freestanding elongated conductive body has a weight under gravity and a buckling force that is less than the weight under gravity.

Any of the features of a particular sensor embodiment is generally applicable, i.e., independently combinable with any of the other aspects or embodiments identified herein. Moreover, any of the features of a sensor embodiment is independently combinable, partly or wholly, with other sensor embodiments described herein in any way, e.g., one, two, or three or more embodiments may be combinable in whole or in part. Further, any of the features of a sensor embodiment may be made optional to other aspects or embodiments. Any aspect or embodiment of a method as described herein can be performed by a sensor, system, or apparatus of another aspect or embodiment, and any aspect or embodiment of a sensor, system or apparatus can be configured to perform a method of another aspect or embodiment.

The methods disclosed herein comprise one or more steps or actions for achieving the described methods. The method steps and/or actions may be interchanged with one another without departing from the scope of the claims. In other words, unless a specific order of steps or actions is specified, the order and/or use of specific steps and/or actions may be modified without departing from the scope of the claims.

The connections between the elements shown in some figures illustrate exemplary communication paths. Additional communication paths, either direct or via an intermediary, may be included to further facilitate the exchange of information between the elements. The communication paths may be bi-directional communication paths allowing the elements to exchange information.

Various operations of methods described above may be performed by any suitable means capable of performing the operations, such as various hardware and/or software component(s), circuits, and/or module(s). Generally, any operations illustrated in the figures may be performed by corresponding functional means capable of performing the operations.

The various illustrative logical blocks, modules and circuits described in connection with the present disclosure (such as the blocks of Fig. 2) may be implemented or performed with a digital signal processor (DSP), an application specific integrated circuit (ASIC), a field programmable gate array signal (FPGA) or other programmable logic device (PLD), discrete gate or transistor logic, discrete hardware components or any combination thereof designed to perform the functions described herein. A processor may be a microprocessor, but in the alternative, the processor may be any commercially available processor, controller, microcontroller or state machine. A processor may also be implemented as a combination of computing devices, e.g., a combination of a DSP and a microprocessor, a plurality of microprocessors, one or more microprocessors in conjunction with a DSP core, or any other such configuration.

In one or more aspects, various functions described may be implemented in hardware, software, firmware, or any combination thereof. If implemented in software, the functions may be stored on or transmitted over as one or more instructions or code on a computer-readable medium. Computer-readable media includes both computer storage media and communication media including any medium that facilitates transfer of a computer program from one place to another. A storage media may be any available media that can be accessed by a computer. By way of example, and not limitation, such computer-readable media can comprise various types of RAM, ROM, CD-ROM or other optical disk storage, magnetic disk storage or other magnetic storage devices, or any other medium that can be used to carry or store desired program code in the form of instructions or data structures and that can be accessed by a computer. Also, any connection is properly termed a computer-readable medium. For example, if the software is transmitted from a website, server, or other remote source using a coaxial cable, fiber optic cable, twisted pair, digital subscriber line (DSL), or wireless technologies such as infrared, radio, and microwave, then the coaxial cable, fiber optic cable, twisted pair, DSL, or wireless technologies such as infrared, radio, WiFi, Bluetooth^{®}, RFID, NFC, and microwave are included in the definition of medium. Disk and disc, as used herein, includes compact disc (CD), laser disc, optical disc, digital versatile disc (DVD), floppy disk and Blu-ray^{®} disc where disks usually reproduce data magnetically, while discs reproduce data optically with lasers. Thus, in some aspects a computer readable medium may comprise non-transitory computer readable medium (e.g., tangible media). In addition, in some aspects a computer readable medium may comprise transitory computer readable medium (e.g., a signal). Combinations of the above should also be included within the scope of computer-readable media.

Certain aspects may comprise a computer program product for performing the operations presented herein. For example, such a computer program product may comprise a computer readable medium having instructions stored (and/or encoded) thereon, the instructions being executable by one or more processors to perform the operations described herein. For certain aspects, the computer program product may include packaging material.

Software or instructions may also be transmitted over a transmission medium. For example, if the software is transmitted from a website, server, or other remote source using a coaxial cable, fiber optic cable, twisted pair, digital subscriber line (DSL), or wireless technologies such as infrared, radio, and microwave, then the coaxial cable, fiber optic cable, twisted pair, DSL, or wireless technologies such as infrared, radio, and microwave are included in the definition of transmission medium.

Further, it should be appreciated that modules and/or other appropriate means for performing the methods and techniques described herein can be downloaded and/or otherwise obtained by a user terminal and/or base station as applicable. For example, such a device can be coupled to a server to facilitate the transfer of means for performing the methods described herein. Alternatively, various methods described herein can be provided via storage means (e.g., RAM, ROM, a physical storage medium such as a compact disc (CD) or floppy disk, etc.), such that a user terminal and/or base station can obtain the various methods upon coupling or providing the storage means to the device. Moreover, any other suitable technique for providing the methods and techniques described herein to a device can be utilized.

It is to be understood that the claims are not limited to the precise configuration and components illustrated above. Various modifications, changes and variations may be made in the arrangement, operation and details of the methods and apparatus described above without departing from the scope of the claims.

Unless otherwise defined, all terms (including technical and scientific terms) are to be given their ordinary and customary meaning to a person of ordinary skill in the art, and are not to be limited to a special or customized meaning unless expressly so defined herein. It should be noted that the use of particular terminology when describing certain features or aspects of the disclosure should not be taken to imply that the terminology is being re-defined herein to be restricted to include any specific characteristics of the features or aspects of the disclosure with which that terminology is associated. Terms and phrases used in this application, and variations thereof, especially in the appended claims, unless otherwise expressly stated, should be construed as open ended as opposed to limiting. As examples of the foregoing, the term 'including' should be read to mean 'including, without limitation,' 'including but not limited to,' or the like; the term 'comprising' as used herein is synonymous with 'including,' 'containing,' or 'characterized by,' and is inclusive or open-ended and does not exclude additional, unrecited elements or method steps; the term 'having' should be interpreted as 'having at least;' the term 'includes' should be interpreted as 'includes but is not limited to;' the term 'example' is used to provide exemplary instances of the item in discussion, not an exhaustive or limiting list thereof, adjectives such as 'known', 'normal', 'standard', and terms of similar meaning should not be construed as limiting the item described to a given time period or to an item available as of a given time, but instead should be read to encompass known, normal, or standard technologies that may be available or known now or at any time in the future; and use of terms like 'preferably,' 'preferred,' 'desired,' or 'desirable,' and words of similar meaning should not be understood as implying that certain features are critical, essential, or even important to the structure or function of the invention, but instead as merely intended to highlight alternative or additional features that may or may not be utilized in a particular embodiment of the invention. Likewise, a group of items linked with the conjunction 'and' should not be read as requiring that each and every one of those items be present in the grouping, but rather should be read as 'and/or' unless expressly stated otherwise. Similarly, a group of items linked with the conjunction 'or' should not be read as requiring mutual exclusivity among that group, but rather should be read as 'and/or' unless expressly stated otherwise.

Where a range of values is provided, it is understood that the upper and lower limit and each intervening value between the upper and lower limit of the range is encompassed within the embodiments.

With respect to the use of substantially any plural and/or singular terms herein, those having skill in the art can translate from the plural to the singular and/or from the singular to the plural as is appropriate to the context and/or application. The various singular/plural permutations may be expressly set forth herein for sake of clarity. The indefinite article "a" or "an" does not exclude a plurality. A single processor or other unit may fulfill the functions of several items recited in the claims. The mere fact that certain measures are recited in mutually different dependent claims does not indicate that a combination of these measures cannot be used to advantage. Any reference signs in the claims should not be construed as limiting the scope.

It will be further understood by those within the art that if a specific number of an introduced claim recitation is intended, such an intent will be explicitly recited in the claim, and in the absence of such recitation no such intent is present. For example, as an aid to understanding, the following appended claims may contain usage of the introductory phrases "at least one" and "one or more" to introduce claim recitations. However, the use of such phrases should not be construed to imply that the introduction of a claim recitation by the indefinite articles "a" or "an" limits any particular claim containing such introduced claim recitation to embodiments containing only one such recitation, even when the same claim includes the introductory phrases "one or more" or "at least one" and indefinite articles such as "a" or "an" (e.g., "a" and/or "an" should typically be interpreted to mean "at least one" or "one or more"); the same holds true for the use of definite articles used to introduce claim recitations. In addition, even if a specific number of an introduced claim recitation is explicitly recited, those skilled in the art will recognize that such recitation should typically be interpreted to mean at least the recited number (e.g., the bare recitation of "two recitations," without other modifiers, typically means at least two recitations, or two or more recitations). Furthermore, in those instances where a convention analogous to "at least one of A, B, and C, etc." is used, in general such a construction is intended in the sense one having skill in the art would understand the convention, e.g., as including any combination of the listed items, including single members (e.g., "a system having at least one of A, B, and C" would include but not be limited to systems that have A alone, B alone, C alone, A and B together, A and C together, B and C together, and/or A, B, and C together, etc.). In those instances where a convention analogous to "at least one of A, B, or C, etc." is used, in general such a construction is intended in the sense one having skill in the art would understand the convention (e.g., "a system having at least one of A, B, or C" would include but not be limited to systems that have A alone, B alone, C alone, A and B together, A and C together, B and C together, and/or A, B, and C together, etc.). It will be further understood by those within the art that virtually any disjunctive word and/or phrase presenting two or more alternative terms, whether in the description, claims, or drawings, should be understood to contemplate the possibilities of including one of the terms, either of the terms, or both terms. For example, the phrase "A or B" will be understood to include the possibilities of "A" or "B" or "A and B."

All numbers expressing quantities of ingredients, reaction conditions, and so forth used in the specification are to be understood as being modified in all instances by the term 'about.' Accordingly, unless indicated to the contrary, the numerical parameters set forth herein are approximations that may vary depending upon the desired properties sought to be obtained. At the very least, and not as an attempt to limit the application of the doctrine of equivalents to the scope of any claims in any application claiming priority to the present application, each numerical parameter should be construed in light of the number of significant digits and ordinary rounding approaches.

All references cited herein are incorporated herein by reference in their entirety. To the extent publications and patents or patent applications incorporated by reference contradict the disclosure contained in the specification, the specification is intended to supersede and/or take precedence over any such contradictory material.

Headings are included herein for reference and to aid in locating various sections. These headings are not intended to limit the scope of the concepts described with respect thereto. Such concepts may have applicability throughout the entire specification.

Furthermore, although the foregoing has been described in some detail by way of illustrations and examples for purposes of clarity and understanding, it is apparent to those skilled in the art that certain changes and modifications may be practiced. Therefore, the description and examples should not be construed as limiting the scope of the invention to the specific embodiments and examples described herein, but rather to also cover all modification and alternatives coming with the true scope and spirit of the invention.

Various system and methods described may be fully implemented and/or controlled in any number of computing devices. Typically, instructions are laid out on computer readable media, generally non-transitory, and these instructions are sufficient to allow a processor in the computing device to implement the method of the invention. The computer readable medium may be a hard drive or solid state storage having instructions that, when run, are loaded into random access memory. Inputs to the application, e.g., from the plurality of users or from any one user, may be by any number of appropriate computer input devices. For example, users may employ a keyboard, mouse, touchscreen, joystick, trackpad, other pointing device, or any other such computer input device to input data relevant to the calculations. Data may also be input by way of an inserted memory chip, hard drive, flash drives, flash memory, optical media, magnetic media, or any other type of file - storing medium. The outputs may be delivered to a user by way of a video graphics card or integrated graphics chipset coupled to a display that maybe seen by a user. Alternatively, a printer may be employed to output hard copies of the results. Given this teaching, any number of other tangible outputs will also be understood to be contemplated by the invention. For example, outputs may be stored on a memory chip, hard drive, flash drives, flash memory, optical media, magnetic media, or any other type of output. It should also be noted that the invention may be implemented on any number of different types of computing devices, e.g., personal computers, laptop computers, notebook computers, net book computers, handheld computers, personal digital assistants, mobile phones, smart phones, tablet computers, and also on devices specifically designed for these purpose. In one implementation, a user of a smart phone or wi-fi - connected device downloads a copy of the application to their device from a server using a wireless Internet connection. An appropriate authentication procedure and secure transaction process may provide for payment to be made to the seller. The application may download over the mobile connection, or over the WiFi or other wireless network connection. The application may then be run by the user. Such a networked system may provide a suitable computing environment for an implementation in which a plurality of users provide separate inputs to the system and method. In the below system where factory calibration schemes are contemplated, the plural inputs may allow plural users to input relevant data at the same time.

### Embodiments

1. A continuous analyte sensor configured for in vivo use, the continuous analyte sensor comprising:
   an elongated core;
   a working electrode disposed on the elongated core; and
   a membrane covering at least a portion of the working electrode, wherein the membrane comprises an enzyme layer, and wherein a portion of the continuous analyte sensor is configured to extend from a sensor electronics housing and to have a buckling force of less than 0.25 Newtons (N).
2. The continuous analyte sensor of Emb. 1, wherein the elongated core comprises an elongated conductive core having a Young's modulus of less than one hundred forty-seven GPa.
3. The continuous analyte sensor of Emb. 2, further comprising a layer of conductive material covering at least a portion of the elongated conductive core.
4. The continuous analyte sensor of Emb. 3, wherein the elongated conductive core comprises at least one material selected from the group consisting of copper, gold, Magnesium, Silver, Tin, Titanium, a Titanium alloy, and Zinc.
5. The continuous analyte sensor of Emb. 4, wherein the conductive material comprises a conductive material selected from the group consisting of platinum, platinum-iridium, gold, palladium, iridium, alloys thereof, graphite, carbon, and a conductive polymer.
6. The continuous analyte sensor of Emb. 3, further comprising a layer of insulating material coving at least a portion of the layer of conductive material, wherein the working electrode is formed, in part, by a window in the layer of insulating material that exposes an electrode portion of the layer of conductive material.
7. The continuous analyte sensor of Emb. 6, further comprising an additional conductive layer covering at least a portion of the layer of insulating material, the additional conductive layer comprising a reference electrode.
8. The continuous analyte sensor of any of Embs. 1-7, wherein the elongated core comprises an elongated polymer core and wherein the portion of the elongated core has a flexural modulus of less than one and a half GPa.
9. The continuous analyte sensor of Emb. 8, further comprising at least one conductive trace that runs along a length of the elongated polymer core, wherein a portion of the conductive trace comprises the working electrode.
10. The continuous analyte sensor of Emb. 8, wherein the elongated polymer core comprises an elongated elliptical polymer core and wherein at least one conductive trace runs along the elongated elliptical polymer core.
11. The continuous analyte sensor of Emb. 10, wherein the working electrode comprises a portion of the at least one conductive trace.
12. The continuous analyte sensor of any of Embs. 1-11, wherein the elongated core comprises an elongated fiber core.
13. The continuous analyte sensor of Emb. 12, wherein the elongated core further comprises:
   an elongated insulating body substantially surrounding the elongated fiber core; and
   at least one conductive trace that runs along the elongated insulating body.
14. The continuous analyte sensor of Emb. 12, wherein the elongated fiber core comprises one or more Kevlar fibers.
15. The continuous analyte sensor of Emb. 14, wherein the elongated core further comprises a conductive coating on the one or more Kevlar fibers, and wherein a portion of the one or more Kevlar fibers having the conductive coating forms the working electrode.
16. A continuous analyte sensor system comprising the continuous analyte sensor of any one of Embs. 1-15 and sensor electronics configured to process sensor signals from the continuous analyte sensor, wherein the sensor electronics are disposed in the sensor electronics housing, and wherein the sensor electronics housing is configured to attach to the exterior of a patient's skin.
17. The continuous analyte sensor of any one of Embs. 1-15, wherein the portion of the elongated core has a buckling force of less than 0.02 N.
18. The continuous analyte sensor of Emb. 17, wherein the portion of the elongated core has a flexural modulus between 0.1 kPa and 300 kPa.
19. A continuous analyte sensor configured for in vivo use, the continuous analyte sensor comprising:
   an elongated conductive body comprising a working electrode, wherein the elongated conductive body comprises a plurality of substantially planar layers, and wherein a portion of the plurality of substantially planar layers that is configured to extend from a housing of a continuous analyte sensor system has, in aggregate, a buckling force of less than 0.25 Newtons (N); and
   a membrane covering at least a portion of the working electrode, wherein the membrane comprises an enzyme layer.
20. The continuous analyte sensor of Emb. 19, wherein the plurality of substantially planar layers comprise:
   an insulating polymer layer;
   a first electrode layer disposed on a first side of the insulating polymer layer; and
   a second electrode layer disposed on an opposing second side of the insulating polymer layer, wherein the working electrode comprises a portion of the first electrode layer and wherein the second electrode layer comprises a reference electrode.
21. The continuous analyte sensor of Emb. 20, further comprising an additional insulating polymer layer formed on the first electrode layer, wherein the additional insulating polymer layer comprises a window that defines the working electrode.
22. The continuous analyte sensor of Emb. 21, wherein the membrane is disposed on the first electrode layer within the window in the additional insulating polymer layer, and wherein the additional insulating polymer layer is substantially free of the membrane outside of the window.
23. The continuous analyte sensor of Emb. 21, wherein the plurality of substantially planar layers, in cross-section, comprises a substantially elliptical outer surface.
24. The continuous analyte sensor of Emb. 21, wherein the insulating polymer layer comprises first and second insulating polymer layers and wherein the plurality of substantially planar layers further comprises a counter electrode layer disposed between the first and second insulating polymer layers.
25. The continuous analyte sensor of Emb. 20, wherein the plurality of substantially planar layers further comprises a conductive trace layer disposed between the first electrode layer and the insulating polymer layer.
26. The continuous analyte sensor of Emb. 25, wherein the plurality of substantially planar layers further comprises an additional insulating polymer layer formed on the conductive trace layer, and wherein the additional insulating polymer layer comprises a window that defines the working electrode.
27. The continuous analyte sensor of any one of Embs. 19-26, wherein additional portions of the elongated conductive body are substantially free of the membrane.
28. A continuous analyte sensor configured for in vivo use, the continuous analyte sensor comprising:
   an elongated conductive body comprising a working electrode, wherein the elongated conductive body is configured to be a freestanding elongated conductive body ex vivo and a non-freestanding elongated conductive body in vivo; and
   a membrane covering at least a portion of the working electrode, wherein the membrane comprises an enzyme layer.
29. The continuous analyte sensor of Emb. 28, wherein the elongated conductive body is configured to transform from the freestanding elongated conductive body to the non-freestanding elongated conductive body responsive to contact between at least a portion of the elongated conductive body and tissue of a patient.
30. The continuous analyte sensor of any one of Embs. 28-29, wherein the elongated conductive body is configured to transform from the freestanding elongated conductive body to the non-freestanding elongated conductive body at a transition temperature of between seventy-eight and one-hundred degrees Fahrenheit.
31. The continuous analyte sensor of any one of Embs. 28-30, wherein the elongated conductive body is configured to transform from the freestanding elongated conductive body to the non-freestanding elongated conductive body responsive to absorption of a fluid from patient tissue by at least a portion of the elongated conductive body.
32. The continuous analyte sensor of any one of Embs. 28-31, wherein the elongated conductive body is configured to transform from the freestanding elongated conductive body to the non-freestanding elongated conductive body responsive to a chemical reaction between a fluid from patient tissue and at least a portion of the elongated conductive body.
33. The continuous analyte sensor of any one of Embs. 28-32, wherein the elongated conductive body is configured to transform from the freestanding elongated conductive body to the non-freestanding elongated conductive body responsive to an electromagnetic field generated by sensor electronics for the continuous analyte sensor.
34. The continuous analyte sensor of any one of Embs. 28-33, wherein the elongated conductive body comprises an elongated conductive core.
35. The continuous analyte sensor of any one of Embs. 28-34, wherein the elongated conductive body comprises an elongated polymeric core.
36. The continuous analyte sensor of any one of Embs. 28-35, wherein the elongated conductive body comprises a plurality of substantially planar layers.
37. A continuous analyte sensor system comprising the continuous analyte sensor of any one of Claims 28-36 and sensor electronics configured to process sensor signals from the continuous analyte sensor, wherein the sensor electronics are disposed in a housing configured to attach to the exterior of a patient's skin.
38. The continuous analyte sensor of Emb. 37, wherein, in vivo, the elongated conductive body has a buckling force of less than 0.01 N.
39. The continuous analyte sensor of any one of Embs. 28-38, wherein the non-freestanding elongated conductive body has a weight under gravity and a buckling force that is less than the weight under gravity.

## Claims

1. A continuous analyte sensor system configured for in vivo use, the analyte sensor system comprising:
an elongated body comprising a working electrode, wherein the elongated body is configured to be a freestanding elongated body ex vivo and a non-freestanding elongated body in vivo; and
a membrane covering at least a portion of the working electrode, wherein the membrane comprises an enzyme layer.

2. The system of claim 1, wherein the elongated body is configured to transform from the freestanding elongated body to the non-freestanding elongated body responsive to contact between at least a portion of the elongated body and tissue of a host.

3. The system of claim 1 or 2, wherein the elongated body is configured to transform from the freestanding elongated body to the non-freestanding elongated body at a transition temperature of between seventy-eight and one-hundred degrees Fahrenheit.

4. The system of any of claims 1 to 3, wherein the elongated body is configured to transform from the freestanding elongated body to the non-freestanding elongated body responsive to absorption of a fluid from patient tissue by at least a portion of the elongated body.

5. The system of any of claims 1 to 3, wherein the elongated body is configured to transform from the freestanding elongated body to the non-freestanding elongated body responsive to a chemical reaction between a fluid from patient tissue and at least a portion of the elongated body.

6. The system of any of claims 1 to 3, wherein the elongated body is configured to transform from the freestanding elongated body to the non-freestanding elongated body responsive to an electromagnetic field generated by sensor electronics for the analyte sensor system.

7. The system of any of claims 1 to 6, wherein the elongated body comprises an elongated conductive core.

8. The system of claim 7, wherein the elongated conductive core comprises at least one material selected from the group consisting of copper, gold, iridium, magnesium, palladium, platinum, silver, tin, titanium, zinc, carbon, conductive polymer, graphite, and alloys or combinations thereof.

9. The system of any of claims 1 to 6, wherein the elongated body comprises an elongated polymeric core.

10. The system of any of claims 1 to 6, wherein the elongated body comprises an elongated fiber core.

11. The system of claim 10, wherein the elongated fiber core further comprises:
an elongated insulating body substantially surrounding the elongated fiber core; and
at least one conductive trace that runs along the elongated insulating body.

12. The system of any of claims 1 to 12, wherein the elongated body comprises a plurality of substantially planar layers.

13. The system of any of claims 1 to 13, wherein, in vivo, the elongated body has a buckling force of less than 0.25 N.

14. The system of any of claims 1 to 16, wherein the non-freestanding elongated body has a weight under gravity and a buckling force that is less than the weight under gravity.

15. The system of any of claims 1 to 17, wherein the elongated body has a flexural modulus between 0.1 kPa and 300 kPa and a Young's modulus of less than one hundred forty to seven GPa.
